# EUROPEAN PATENT APPLICATION

(11) **EP 4 620 949 A1**
(43) Date of publication of application: **24.09.2025**
(21) Application number: 24164226.3
(22) Date of filing: 18.03.2024
(51) Int. Cl.: C07D 209/42, C07D 307/85, C07D 333/38, C07D 333/70, C07D 409/04, A61K 31/381, A61P 7/02

(54) **ACKR3 MODULATORS FOR CARDIOVASCULAR OR ANTIPLATELET THERAPY**

(71) Applicant: Eberhard Karls Universität Tübingen (Medizinische Fakultät), 72074 Tübingen (DE); Luxembourg Institute of Health (LIH), 1445 Strassen (LU)
(72) Inventor: LAUFER, Stefan, 72070 Tübingen (DE); GAWAZ, Meinrad, 72076 Tübingen (DE); THANIGAIMALAI, Pillaiyar, 72076 Tübingen (DE); BAYRAK, Alp, 72076 Tübingen (DE); ROHLFING, Anne-Katrin, 72076 Tübingen (DE); DICENTA-BAUNACH, Valerie, 72076 Tübingen (DE); SZPAKOWSKA, Martyna, 1370 Luxembourg (LU); CHEVIGNÉ, Andy, 6747 Saint Léger (BE)
(74) Representative: Witte, Weller & Partner Patentanwälte mbB

(57) **Abstract**

The present invention discloses a compound of any of the general formulas (la) to (Ig): 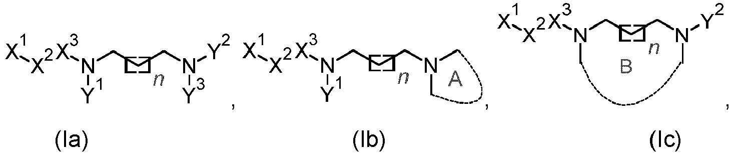 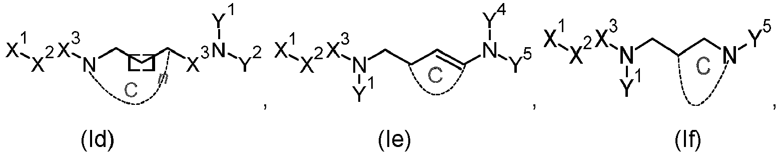 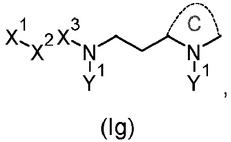 pharmaceutical compositions comprising said compounds and their use in the treatment of diseases, especially a disease is mediated by ACKR3, preferably a cardiovascular disease or a platelet disorder. In addition, method for treating a disease in which mediation, such as inhibition, regulation and/or modulation, of ACKR3 is beneficial in a human or a warm-blooded or mammal animal in need of such treatment is disclosed.

## Description

The invention relates to a novel compound for the treatment and prophylaxis of a disease, especially a disease that is mediated by ACKR3, including but not limited to a cardiovascular disease or a platelet disorder, a pharmaceutical composition comprising said compound, and to a method for the treatment and/or prophylaxis of a disease, preferably a cardiovascular disease or a platelet disorder.

### FIELD OF THE INVENTION

The invention relates to the field of pharmaceutically active compounds, more particularly to the field of compounds useful in the treatment and prevention of conditions affecting the heart or blood vessels or in the treatment and prevention of abnormal clotting of platelets.

### BACKGROUND OF THE INVENTION

Chemokines, also known as chemotactic cytokines, are a family of signaling proteins, which primarily induce cell migration. Their involvement is prominent in several types of cell movement processes, for example, chemotaxis, haptotaxis, chemo-kinesis, haptokinesis, and transcellular migration. In addition to their function as chemoat-tractants, they take part in a variety of important processes like the homeostasis and development of the immune system or inflammatory signaling. Over 50 human chemokines have been identified, which are classified into four subfamilies (C, CC, CXC, CX3C). Their respective chemokine receptors can be divided into two families, the conventional chemokine receptors (cCKRs) and the atypical chemokine receptors (ACKRs). While all chemokine receptors are structurally homologous to GPCRs and therefore belong to the large family of G protein-coupled receptors, only the members of the cCKRs transduce signals through G-proteins. ACKRs engage in different transducers and functions, for example in the case of the atypical chemokine receptor 3 (ACKR3) by recruitment of beta-arrestin.

Due to their key roles in important physiological processes, efforts have been made to develop ligands for various chemokine receptors. With Maraviroc as the first C-C chemokine receptor type 5 antagonist in HIV treatment therapy and Plerixaflor, a C-X-C chemokine receptor type 4 (CXCR4) antagonist initially developed for HIV treatment, being used in some cancer patients before radiation or chemotherapy to mobilize stem cells into the bloodstream, the chemokine receptors were proven as viable drug targets. ACKR3, formerly known as C-X-C chemokine receptor type 7 (CXCR7), is associated with multiple diseases. It is expressed in a range of regions, among other things in the central nervous system, the adrenal gland, endothelial cells, immune cells, and platelets. Particularly, ACKR3's importance in cardiovascular diseases (CVD) was discussed frequently in the recent scientific literature. As of today, cardiovascular diseases are the leading cause of death worldwide with approximately 17.9 million deaths per year. Therefore, new therapeutic strategies are in high demand. Several studies showed chemokines and ACKR3 play key roles in the process of CVD development, including upregulated expression of ACKR3 in mice or humans suffering from atherosclerosis, strokes, myocardial infarction, and platelet function (Gawaz, M. et al. Current concepts and novel targets for antiplatelet therapy. Nature Reviews Cardiology 2023, 20, 583-599).

The enhanced surface expression of ACKR3 and its endogenous ligand stromal cell-derived factor-1 (SDF-1), also known as CXCL12, on platelets of patients with acute coronary syndrome was described. The role of ACKR3 in prothrombotic events was shown and substantiated later when all-cause mortality in patients with coronary artery disease (CAD) was associated with base-line levels of ACKR3 and C-X-C chemokine receptor type 4 (CXCR4). While CXCR4 ligation by CXCL12 leads to Gᵢ-mediated platelet activation, the opposite occurs when activating ACKR3. ACKR3 ligation promotes platelet survival, and inhibits platelet activation/aggregation and thrombus formation.

In Bayrak, A. et al., the first-in-class ACKR3 agonists for the modulation of platelet aggregation and degranulation are developed. Hereby, it was possible to prove, that ACKR3 ligation leads to the inhibition of platelet aggregation (Bayrak, A. et al. Discovery and development of first-in-class ACKR3/CXCR7 superagonists for platelet degranulation modulation. J. Med. Chem. 2022, 65, 13365-13384).

An objective underlying the present invention is to provide compounds that are effective in inhibiting, regulating, and/or modulating ACKR3. Another objective is to provide compounds with one or more greatly improved potency, functional activity, and selectivity toward ACKR3. Still, another objective is targeting ACKR3 with small molecules. A further objective resides in the provision of ACKR3 inhibitors, regulators, and/or modulators as a medicament. Still, a further objective resides in the provision of ACKR3 inhibitors, regulators, and/or modulators that are efficient for use in the treatment of a cardiovascular disease or a platelet disorder as well as other diseases. Another objective resides in the provision of such compounds or compositions comprising such compounds with low toxicity to humans and/or warm-blooded or mammal animals.

### SUMMARY OF THE INVENTION

The abovementioned objectives have been achieved by providing a compound of any of the general formulas (Ia) to (Ig): 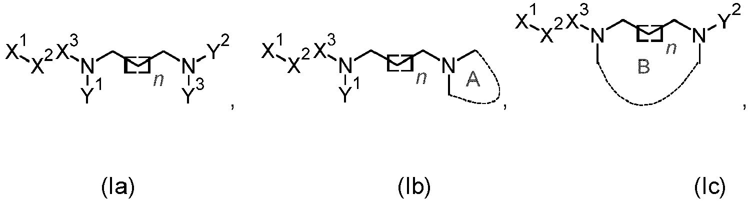 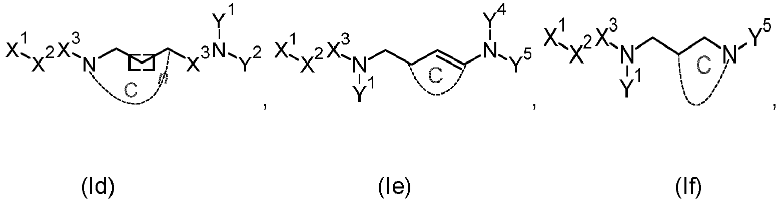 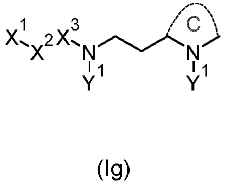 or a stereoisomer, enantiomer, tautomer, pro-drug, and/or a pharmaceutically acceptable salt thereof,
wherein
X¹ is selected from substituted or unsubstituted cyclyl, substituted or unsubstituted heterocyclyl, substituted or unsubstituted fused cyclyl, and substituted or unsubstituted fused heterocyclyl,
X² is selected from -SCH₂-, -CH₂CH₂-, and a bond,
X³ is selected from carbonyl, thiocarbonyl, and methylene,
Y¹ is selected from H and methyl,
Y² is selected from substituted or unsubstituted phenyl, substituted or unsubstituted benzyl, tert-butyl, and tert-butyloxycarbonyl,
Y³ is selected from H, substituted or unsubstituted C₁-C₅ alkyl, and C₃-C₅ cycloalkyl,
Y⁴ and Y⁵ are O, or Y⁴ is selected from H, and methyl, Y⁵ is selected from substituted or unsubstituted benzyl, substituted or unsubstituted benzoyl, and linear or branched C₁-C₅ alkylcarboxylate,
A and B are independently selected from substituted or unsubstituted heterocyclyl, and substituted or unsubstituted fused heterocyclyl,
C is selected from substituted or unsubstituted cyclyl, and
n is 0-2.

The term "cyclyl" as used herein encompasses saturated or at least partially unsaturated cyclic compound having, e.g., 3 to 8 C-atoms, such as 3 C-atoms, 4 C-atoms, 5 C-atoms, and 6 C-atoms, and 7 C-atoms, and 8 C-atoms. Cyclyl residues also encompass aryl residues, such as phenyl. The definition of cyclyl is likewise applicable to any group that includes a cyclyl group, such as phenoxy. The cyclyl group may be bound to the neighboring group, e.g., another cyclyl residue, via a carbon atom (C-bound). Cyclyl residues may be also present as fused cyclyl residues, in which two or more C-atoms, such as 2 C-atoms, of a first cyclyl residue can form part of a second cyclyl residue.

The term "heterocyclyl" as used herein designates cyclyl residues containing one to three heteroatoms that may be independently selected from S, O, and N. The heterocyclyl group may be bound to the neighboring group, e.g., another cyclyl residue, e.g., via a carbon atom (C-bound) or a nitrogen heteroatom (N-bound). The definition of heterocyclyl is likewise applicable to any group which includes a heterocyclyl group. Heterocyclyl residues may be also present as fused heterocyclyl residues, in which two or more atoms of a first heterocyclyl residue can form part of a second heterocyclyl or cyclyl residue. Heterocyclyl groups can include thiophenyl, pyrrolyl, pyrazolyl, imidazolyl, pyridinyl, pyrimidinyl, azetidinyl, pyrrolidinyl, piperidinyl, piperazinyl, tetrahydrochinoline, and in-doline.

A preferred heterocyclyl residue is thiophen-2-yl. The thiophen-2-yl may be unsubstituted or substituted with one or two substituents, preferably, one substituent, more preferably at position 5 of the thiophen-2-yl residue.

A more preferred heterocyclyl residue is (substituted or unsubstituted phenyl)thiophen-2-yl. The substituted or unsubstituted phenyl residue may be located at position 5 of the thiophen-2-yl-residue forming (unsubstituted or 5-substituted phenyl)-thio-phen-2-yl-residue.

Preferred examples of substituted phenyl residues include 4-hydroxyphenyl, 2-halo-phenyl, 3-halo-phenyl, 4-halo-phenyl, 4-methyl-phenyl, 4-tert-butyl-phenyl, 4-methoxy-phenyl, and one of the before mentioned substituted phenyl residues having one additional preferred substituent, such as 3,4-dihalo-phenyl, such as 3,4-difluoro-phenyl.

Another more preferred heterocyclyl residue, which may form A of the compound Ib, preferably R² of the compound IIb, is substituted or unsubstituted 3,4-dihydroisoquinolin-2(1*H*)-yl, or substituted or unsubstituted piperidine-1-yl (each connected via the N-atom to the compound lb). Examples of substituted 3,4-dihydroisoquinolin-2(1*H*)-yl include 3,4-dihydroisoquinolin-2(1*H*)-yl. Examples of substituted piperidine-1-yl include a 4-(alkylcarbamoyl)piperidin-1-yl, wherein alkyl is linear or branched C1-C5 alkyl, such as *tert*-butyl.

The term "bond" as used herein describes a chemical bond, in particular, a covalent single bond, which is formed between two residues.

The term "alkyl" as used herein encompasses linear or branched carbon chains. Preferred alkyl groups include methyl, ethyl, n-propyl, iso-propyl, n-butyl, 2-butyl, iso-butyl, tert-butyl, pentyl, 1-methylbutyl, 2-methylbutyl, 3-methylbutyl, 2,2-dimethylpropyl, and 1-ethylpropyl. The definition of alkyl is likewise applicable to any group which includes an alkyl group, such as alkoxy, or thioalkoxy.

The term "substituted", or "substituent" as used herein may designate residues including H, alkyl, heteroalkyl, cyclyl, heterocyclyl, fused cyclyl, fused heterocyclyl, and combinations thereof. In addition, the term "substituted" designates residues including halogen or halo, such as F, Cl, Br, I, modifications to carbons by introducing one or more heteroatoms, such as N, S or O, or a carboxylate function, a primary, secondary, or tertiary amine function, which is optionally further substituted, an O carrying a substituent, a carboxylic group, which is optionally further substituted.

Preferably "substituted" designates alkyl, such as linear or branched C1-C5 alkyl, hydroxyl, alkoxy, such as linear or branched C1-C5 alkoxy, thiol, thioalkyl, such as linear or branched C1-C5 thioalkyl, halogen or halo, such as F, Cl, Br, I, nitro, amine, mono- or dialkylamine, wherein each alkyl residue is independently selected from linear or branched C1-C5 alkyl, alkyl-carboxamide, and cyclyl-carboxamide.

Preferred examples of substituted benzyl residues include 2-halo-benzyl, 3-halo-benzyl, 4-halo-benzyl, 3-nitro-benzyl, 4-methoxy-benzyl.

The molecular sizes of the present residues are selected such that the present compounds do not exhibit a steric hindrance that negatively affects or even impedes biological activity. Therefore, the overall molecular weight of a substituent may be about ≤ 200 g/mol, such as about ≤ 150 g/mol, or about ≤ 100 g/mol.

The term "small molecule" as used herein may refer to compounds of the invention having a low molecular weight of about 1.000 g/mol or less, such as 900 g/mol to 300 g/mol, 800 g/mol to 350 g/mol, 700 g/mol to 400 g/mol, 600 g/mol to 450 g/mol, or 650 g/mol to 500 g/mol. Small molecules have the advantage of a simpler structure, which facilitates their synthesis. In addition, their low molecular weight lends more predictability to their pharmacokinetics and/or pharmacodynamics, which in turn may facilitate dosing. Furthermore, the small molecules can be easily administered, e.g. orally, and/or they can pass through cell membranes to reach intracellular targets.

The term "the present compounds", "the compounds of the invention" or "the compounds of formula (I)" or any other formula and/or structure include the pharmaceutically acceptable salts, pro-drugs, biologically active metabolites, solvates, and stereoisomers thereof.

The pharmaceutically acceptable salts may be acid addition salts with pharmaceutically acceptable acids. Examples of suitable pharmaceutically acceptable organic and inorganic acids are hydrochloric acid, hydrobromic acid, phosphoric acid, sulfuric acid, sulfamic acid, C1-C4-alkylsulfonic acids, such as methanesulfonic acid, cycloaliphatic sulfonic acids, such as S-(+)-10-camphor sulfonic acid, aromatic sulfonic acids, such as benzenesulfonic acid and toluenesulfonic acid, di- and tricarboxylic acids and hydroxycarboxylic acids having 2 to 10 carbon atoms, such as oxalic acid, malonic acid, maleic acid, fumaric acid, lactic acid, tartaric acid, citric acid, glycolic acid, adipic acid, and benzoic acid. Further suitable pharmaceutically acceptable acids may be derived from, e.g., Remington: The Science and Practice of Pharmacy, 23rd edition, October 30, 2020, Adeboye Adejare.

The invention also includes any tautomeric, crystal, and polymorphic forms of the compounds, salts, and mixtures thereof.

The invention also includes solvates such as hydrates.

The compounds of the invention may contain one or more chiral centers and exist in different optically active forms such as enantiomers and diastereomers.

As used herein, the term "pro-drug" refers to an agent that is converted into the parent drug in vivo by some physiological chemical process. An example, without limitation, of a pro-drug would be a compound of the present invention in the form of an ester.

Pro-drugs have many useful properties. For example, a pro-drug may be more water soluble than the ultimate drug, thereby facilitating intravenous administration of the drug. A pro-drug may also have a higher level of oral bioavailability than the ultimate drug. After administration, the pro-drug is enzymatically or chemically cleaved to deliver the ultimate drug in the blood or tissue. Suitable pro-drugs are known to the skilled person.

The compounds of the invention may mediate (i.e., inhibit, regulate, and/or modulate) ACKR3 and are therefore useful in therapy, particularly, in the treatment of diseases or conditions mediated at least in part by ACKR3. They may be particularly useful in treating a cardiovascular disease or a platelet disorder.

Exemplary, cardiovascular diseases include coronary artery diseases, such as angina pectoris and heart attack, stroke, heart failure, hypertensive heart disease, rheumatic heart disease, cardiomyopathy, abnormal heart rhythms, congenital heart disease, valvular heart disease, carditis, aortic aneurysms, peripheral artery disease, thromboembolic disease, venous thrombosis, and atherosclerosis. Exemplary platelet disorders comprise thrombocytosis, thrombocythemia, and thrombocytopenia, such as immune thrombocytopenia and thrombotic thrombocytopenic purpura, Bernard Soulier disease, Glanzmann's thrombasthenia, Hermansky Pudlak syndrome, Jacobsen syndrome, Lowe syndrome, platelet release, and storage pool defects, thrombocytopenia with absent radius (TAR) syndrome and thrombotic thrombocytopenic purpura (TTP).

The compounds of the invention can also be used for the treatment of other diseases and disorders modulated through ACKR3, such as treatment of distress dysfunction diseases or conditions, chronic or acute pain, cancers (breast, brain, lymphoma), fibrosis (e.g. cardiac fibrosis), inflammatory or autoimmune diseases and conditions, conditions of excessive or abnormal vascularization (e.g. wound healing), stem cell differentiation and mobilization disorders, brain and neuronal dysfunctions (e.g. Alzheimer's disease, multiple sclerosis and demyelinating diseases), kidney dysfunction, renal dysfunction, preeclampsia and obesity.

According to a preferred embodiment, the compound has the formula (Ia) or (lb), wherein
X¹ is substituted or unsubstituted heterocyclyl, preferably substituted or unsubstituted thio-phen-2-yl, more preferably, (substituted or unsubstituted phenyl)thiophen-2-yl,
X² is a bond,
X³ is carbonyl,
Y¹ is H,
Y² is unsubstituted benzyl,
Y³ is selected from linear or branched C₁-C₄ alkyl, C₂-C₃ alkyl phenyl and C₃-C₄ cycloalkyl, preferably methyl, and
A is selected from substituted or unsubstituted 1-piperidinyl, substituted or unsubstituted 3,4-dihydroisoquinolin-2(1*H*)-yl, and
n is 1.

According to another preferred embodiment, the compound has any of the general formulas (IIa) to (IIc): 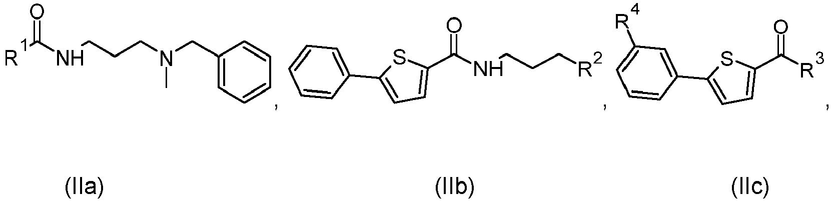 wherein
R1 is 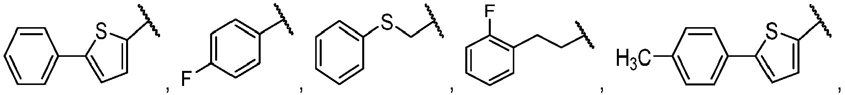 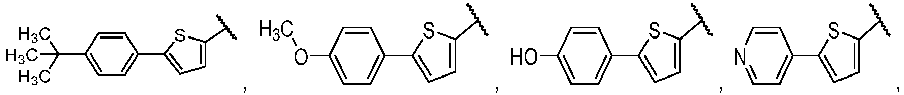 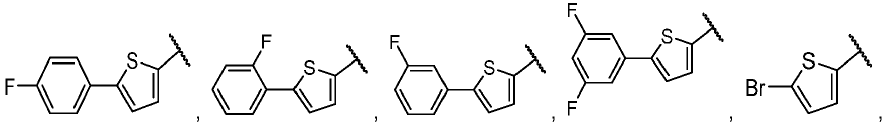 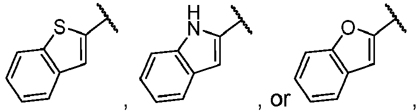
R² is 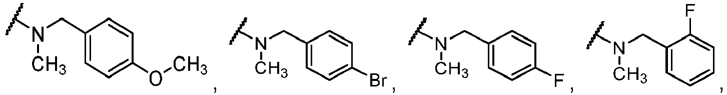 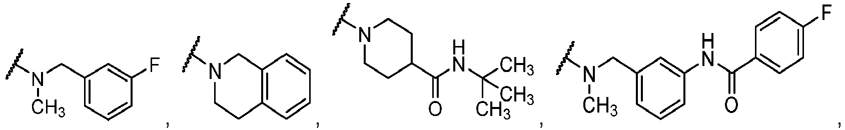 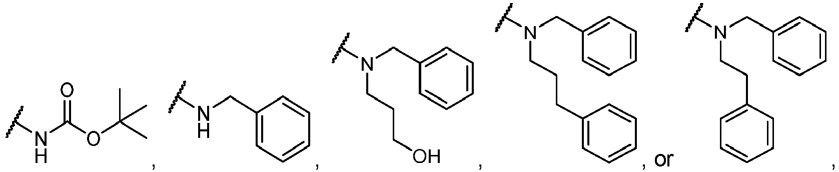
R³ is 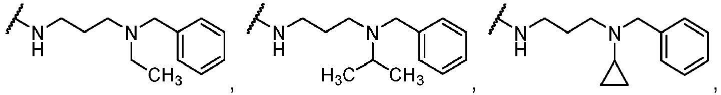 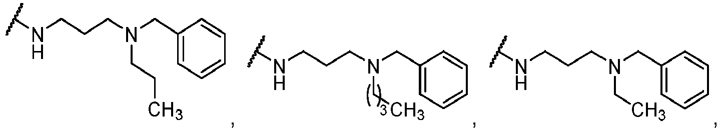 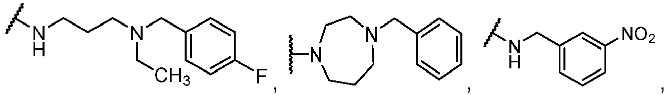 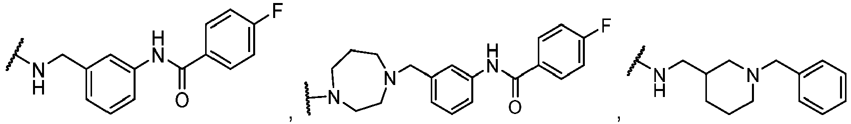 or 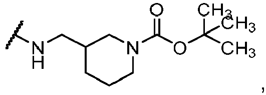 and
R⁴ is halogen, preferably F.

Alternatively, the compound has the general formula (Ila), wherein R¹ is 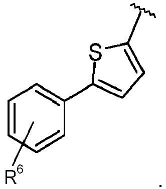 R⁶ is one or two residues that are independently selected from a substituent, wherein the substituent is optionally further substituted. Preferably, R⁶ is one or two residues that are independently selected from the group consisting of H, substituted or unsubstituted C1-C5 alkyl, halo, such as F, Cl, Br, I, an O carrying a substituent, such as OH, and a S carrying a substituent, such as SH, a carboxylic group, which is optionally further substituted. More preferably R⁶ is one or two residues that are independently selected from the group consisting of H, unsubstituted alkyl, linear or branched unsubstituted alkyl, OH, O(alkyl), halo or dihalo, such as F, Cl, Br, I.

Alternatively, the compound has the general formula (IIb), wherein R² is 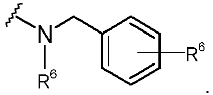 R⁶ is independently selected from a substituent, wherein the substituent is optionally further substituted. Preferably, R⁶ is independently selected from the group consisting of H, substituted or unsubstituted C1-C5 alkyl, substituted or unsubstituted C3-C5 cyclyl, halo, such as F, Cl, Br, I, a carboxylate function, which is optionally further substituted, an O carrying a substituent. More preferably R⁶ is independently selected from the group consisting of H, halo, such as F, Cl, Br, I, unsubstituted alkyl, unsubstituted cyclyl, O(C1-C5 unsubstituted alkyl. Still more preferably R⁶, is independently selected from the group consisting of H, F, C!, Br, unsubstituted alkyl, unsubstituted cyclyl, substituted or unsubstituted C1-C4 alkyl, linear or branched C1-C4 alkyl, unsubstituted cyclyl, C2-C3 alkyl phenyl; R⁶, at benzyl, is independently selected from the group consisting of H, Br, F, O(alkyl), NCO(substituted cyclyl).

According to still another preferred embodiment, the compound has the general formula (Ild) or (Ile): 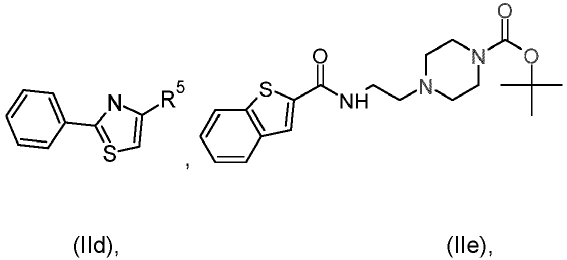 wherein
R⁵ is

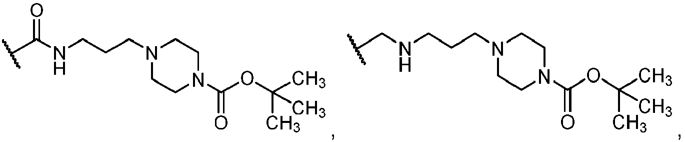

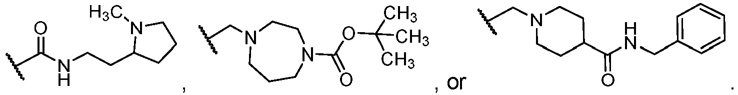

According to a preferred embodiment, the compound has a β-Arrestin recruitment EC₅₀ of 0.5 µM or less, preferably 0.1 µM or less, and/or a P-Selectin expression reduction of 71% or more, preferably 90% or more. The compound has a β-Arrestin recruitment EC₅₀ of 0.5 µM or less, such as about 0.45 µM or less, about 0.40 µM or less, about 0.35 µM or less, about 0.30 µM or less, about 0.25 µM or less, about 0.20 µM or less, or about 0.15 µM or less. Preferably, the compound has a β-Arrestin recruitment EC₅₀ of 0.1 µM or less. The compound has a P-Selectin expression reduction of 71% or more, such as 72% or more, 73% or more, 74% or more, 75% or more, 80% or more, or 85% or more. Preferably, the compound has a P-Selectin expression reduction of 90% or more, such as 95% or more, 96% or more, or 97% to 100%. The β-Arrestin recruitment EC₅₀ and/or the P-Selectin expression reduction can be measured according to the description.

According to another preferred embodiment, a pharmaceutical composition is provided that comprises the present compound or a pharmaceutically acceptable salt, pro-drug, biologically active metabolite, solvate, or stereoisomer thereof.

According to still another preferred embodiment, the pharmaceutical composition further comprises an inert carrier and/or one or more other therapeutic agents.

According to a preferred embodiment, a compound of the present invention or a pharmaceutically acceptable salt, pro-drug, biologically active metabolite, solvate, or stereoisomer thereof, or the present composition, is provided for use in the treatment of a disease.

According to another preferred embodiment, the disease is mediated by ACKR3.

According to still another preferred embodiment, the disease is a cardiovascular disease or a platelet disorder.

According to a preferred embodiment, the cardiovascular disease is selected from coronary artery diseases, such as angina pectoris and heart attack, stroke, heart failure, hypertensive heart disease, rheumatic heart disease, cardiomyopathy, abnormal heart rhythms, congenital heart disease, valvular heart disease, carditis, aortic aneurysms, peripheral artery disease, thromboembolic disease, venous thrombosis, and atherosclerosis and wherein the platelet disorder is selected from thrombocytosis, thrombocythemia, and thrombocytopenia, such as immune thrombocytopenia and thrombotic thrombocytopenic purpura, Bernard Soulier disease, Glanzmann's thrombasthenia, Hermansky Pudlak syndrome, Jacobsen syndrome, Lowe syndrome, platelet release and storage pool defects, thrombocytopenia with absent radius (TAR) syndrome and thrombotic thrombocytopenic purpura (TTP).

According to another preferred embodiment, a method for treating a disease is provided, in which mediation, such as activation or inhibition, regulation, and modulation, of ACKR3 is beneficial in a human or a warm-blooded or mammal animal in need of such treatment, which comprises administering to said human or warm-blooded or mammal animal a therapeutically effective amount of the present compound, or a pharmaceutically acceptable salt, pro-drug, biologically active metabolite, solvate or stereoisomer thereof, or the present composition.

According to still another preferred embodiment, the disease is a cardiovascular disease or a platelet disorder.

According to a preferred embodiment, the cardiovascular disease is selected from coronary artery diseases, such as angina pectoris and heart attack, stroke, heart failure, hypertensive heart disease, rheumatic heart disease, cardiomyopathy, abnormal heart rhythms, congenital heart disease, valvular heart disease, carditis, aortic aneurysms, peripheral artery disease, thromboembolic disease, venous thrombosis, and atherosclerosis and wherein the platelet disorder is selected from thrombocytosis, thrombocythemia, and thrombocytopenia, such as immune thrombocytopenia and thrombotic thrombocytopenic purpura, Bernard Soulier disease, Glanzmann's thrombasthenia, Hermansky Pudlak syndrome, Jacobsen syndrome, Lowe syndrome, platelet release and storage pool defects, thrombocytopenia with absent radius (TAR) syndrome and thrombotic thrombocytopenic purpura (TTP).

The present compounds may be customarily administered in the form of pharmaceutical compositions which comprise at least one compound according to the invention, optionally together with a carrier, such as a pharmaceutically acceptable excipient and, optionally, one or more other drugs, such as a drug that is suitable for treating or preventing a cardiovascular disease or a platelet disorder.

These compositions can, for example, be administered orally, rectally, transdermally, subcutaneously, intraperitoneally, intravenously, intramuscularly, or intranasally. Examples of suitable pharmaceutical compositions are solid medicinal forms, such as powders, granules, tablets, in particular film tablets, lozenges, sachets, cachets, sugar- coated tablets, capsules, such as hard gelatin capsules and soft gelatin capsules, or suppositories, semisolid medicinal forms, such as ointments, creams, hydrogels, pastes or plasters, and also liquid medicinal forms, such as solutions, emulsions, in particular oil-in-water emulsions, suspensions, for example, lotions, injection preparations, and infusion preparations. In addition, liposomes and/or microspheres may be also used.

When producing the compositions, the compounds according to the invention are optionally mixed or diluted with one or more carriers, such as pharmaceutically acceptable excipients. Carriers, such as pharmaceutically acceptable excipients, can be solid, semisolid, or liquid materials that serve as vehicles, carriers, or medium for the compound of the present invention.

Suitable carriers, such as pharmaceutically acceptable excipients, are known to the skilled person and may be readily derived from textbooks. Formulations of suitable carriers, such as pharmaceutically acceptable excipients, can comprise pharmaceutically acceptable auxiliary substances, such as wetting agents; emulsifying and suspending agents; preservatives; antioxidants; anti-irritants; chelating agents; coating auxiliaries; emulsion stabilizers; film formers; gel formers; odor masking agents; taste corrigents; resins; hydrocolloids; solvents; solubilizers; neutralizing agents; diffusion accelerators; pigments; quaternary ammonium compounds; refatting and overfatting agents; raw materials for ointments, creams or oils; silicone derivatives; spreading auxiliaries; stabilizers; sterilants; suppository bases; tablet auxiliaries, such as binders, fillers, glidants, disintegrants or coatings; propellants; drying agents; opacifiers; thickeners; waxes; plasticizers and white mineral oils. Formulations of suitable carriers may be prepared according to, e.g., Fiedler, H.P., Lexikon der Hilfsstoffe fur Pharmazie, Kosmetik und angrenzende Gebiete [Encyclopedia of auxiliary substances for pharmacy, cosmetics, and related fields], 5th edition, ECV-Editio-Cantor-Verlag, 2001.

The compounds of the invention can be administered at a unit dose within the range of 2.5 to 5000 mg/m² body area or 0.05 to 100 mg/kg of the human or animal, particularly a warm-blooded or mammal animal. A unit dose form can contain 0.1 to 250 mg of compound of the invention. The daily dose will be necessarily varied depending upon the host treated, the route of administration, any co-therapies, and the severity of the disorder.

The present compounds may also be suitable for combination with other therapeutic agents or drugs. The invention therefore further relates to a combination comprising a compound of the invention with one or more further therapeutic agents, particularly, for use in treating a cardiovascular disease, a platelet disorder, a disease associated with a cardiovascular disease, or a disease associated with a platelet disorder. The combination therapies of the invention may be administered adjunctively. By adjunctive administration is meant the coterminous or overlapping administration of each of the components in the form of separate pharmaceutical compositions or devices. This regime of therapeutic administration of two or more therapeutic agents is referred to generally by those skilled in the art and herein as adjunctive therapeutic administration; it is also known as add-on therapeutic administration. Any and all treatment regimes in which a patient receives separate but coterminous or overlapping therapeutic administration of the compounds of the invention and at least one further therapeutic agent are within the scope of the current invention. In one embodiment of adjunctive therapeutic administration as described herein, a patient is typically stabilized on a therapeutic administration of one or more of the components for a period of time and then receives administration of another component.

The combination therapies of the invention may also be administered simultaneously or sequentially. By simultaneous administration is meant a treatment regime wherein the individual components are administered together, either in the form of a single pharmaceutical composition or device comprising or containing both components, or as separate compositions or devices, each comprising one of the components, administered simultaneously. Such combinations of the separate individual components for a simultaneous combination may be provided in the form of a kit-of-parts.

Suitable agents for use in combination with the compounds of the inventions include for example: anticoagulants, such as apixaban, dabigatran, edoxaban, heparin, rivaroxaban, warfarin, antiplatelet agents and agents for dual antiplatelet therapy (DAPT), such as acetylsalicylic acid, clopidogrel, dipyridamole, prasugrel, ticagrelor, angiotensin-converting enzyme (ACE) inhibitors, such as benazepril, captopril, enalapril, fosinopril, lisinopril, moexipril, perindopril, quinapril, ramipril, trandolapril, angiotensin II receptor blockers (or inhibitors), such as azilsartan, candesartan, eprosartan, irbesartan, losartan, olmesartan, telmisartan, valsartan, angiotensin receptor-neprilysin inhibitors (AR-Nls), such as, sacubitril/valsartan, beta blockers, such as acebutolol, atenolol, betaxolol, bisoprolol/hydrochlorothiazide, bisoprolol, metoprolol, nadolol, propranolol, sotalol, combined alpha and beta-blockers, such as carvedilol, labetalol hydrochloride, calcium channel blockers, such as amlodipine, diltiazem, felodipine, nifedipine, nimodipine, nisoldipine, verapamil, cholesterol-lowering medications, such as statins including atorvastatin, fluvastatin, lovastatin, pitavastatin, pravastatin, rosuvastatin, simvastatin, nicotinic acids including niacine, cholesterol absorption inhibitor including ezetimibe, combination statin and cholesterol absorption inhibitors such as ezetimibe/simvastatin, digitalis preparations, such as digoxin, diuretics, such as acetazolamide, amiloride, bumetanide, chlorothiazide chlorthalidone, furosemide, hydro-chlorothiazide, indapamide, metalozone, spironolactone, torsemide, and vasodilators, such as isosorbide dinitrate, isosorbide mononitrate, hydralazine, nitroglycerin, minoxidil.

### DETAILED DESCRIPTION

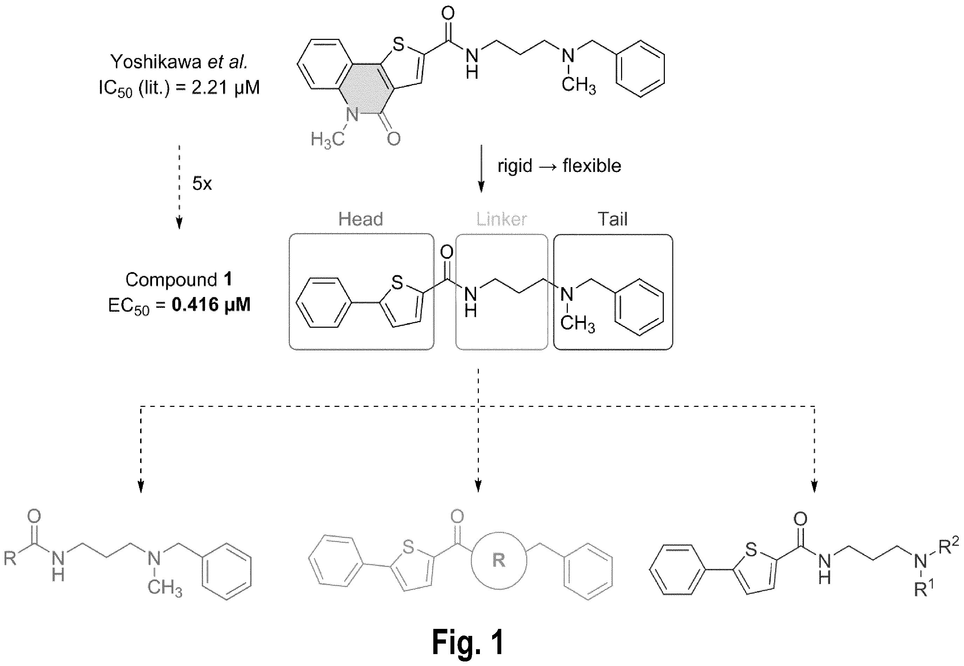

Design of New Compounds. The gist of the invention resides in finding potent ACKR3 ligands and screening them for their functional activity on platelet modulation. Starting from a publication from Yoshikawa *et al.,* wherein a set of compounds were derived from molecular docking studies and evaluated for their binding affinity towards ACKR3 (Yoshikawa, Y. et al. Optimized method of g-protein-coupled receptor homology modeling: Its application to the discovery of novel CXCR7 ligands. J. Med. Chem. 2013, 56, 4236-4251), small molecules with moderate to good biological activity in ACKR3 models were shown. It was chosen to optimize one of the reported ligands to achieve lower EC₅₀ values and functional activity. For the optimization process, a systematic approach was chosen. At first, the rigid ring moiety of the reported ligand was removed to open up to a more flexible core structure (Fig. 1). The initial compound 1 showed already 5-fold improved potency on our beta-arrestin recruitment assays. The new hit molecule was separated into 3 core components: the head, the tail, and the connecting linker (Fig. 1).

Starting with the head component, each moiety was then derivatized and tested individually for its affinity towards ACKR3. Profound structure-activity relationship studies led to lead compound 27 (EC₅₀ = 0.069 *µ*M; P-Selectin exp. red. = 77%), with excellent *β-Arrestin* recruitment potency, full-agonistic properties, and great platelet functional activity. Therefore, compound 27 was further evaluated for its metabolic stability, cell toxicity, and selectivity towards ACKR3.

Hereinafter, the rational design, development, and evaluation of novel ACKR3 agonists, with high potency, efficacy, and selectivity towards ACKR3 is described. In addition, they inherit excellent properties, like functional activity and low cell toxicity, to allow this new class of compounds to be employed in future *in vivo* studies.

Chemistry. The synthesis of the final compounds was achieved in a range of different routes, but a significant number of finals (compounds 1-32) could be synthesized using a modular 3 to 5-step synthesis (Scheme 1). Beginning with the *in situ* Finkelstein reaction and nucleophile substitution reaction of *tert*-butyl (3-bromopropyl)carbamate and the respective alkyl benzylamine provided the boc-protected linker as a precursor. While most alkyl benzylamines were commercially available, compound 20's, 29's, and 30's precursor (compound 20c, 29c, and 30c) were synthesized by employing a nucleophile substitution reaction or a reductive amination first. After successful purification, the boc-protected linker was deprotected using 4 M HCl in 1,4-dioxane. If possible, the solid HCl-salt of the free amine was collected using suction filtration and washed to obtain the pure product, otherwise, the solvent was evaporated, and the intermediate was used without further purification. The amine could then be coupled with the appropriate carboxylic acid using HATU and DIPEA as a base to obtain the final compounds. Compound 35 was synthesized likewise (Scheme 2), starting with commercially available tetrahydroisoquinoline. In the case of left-hand site substituted compounds ("head optimization", Structure A, table 1), multiple compounds were synthesized by applying an extra step. By synthesizing the bromothiophene derivative (compound 2) first, it was possible to employ Suzuki-coupling reactions with the respective boronic acid (pinacol ester) to obtain a range of different "left-hand site" substituted derivatives (compounds 9-17, 31, 32) in a short amount of time.

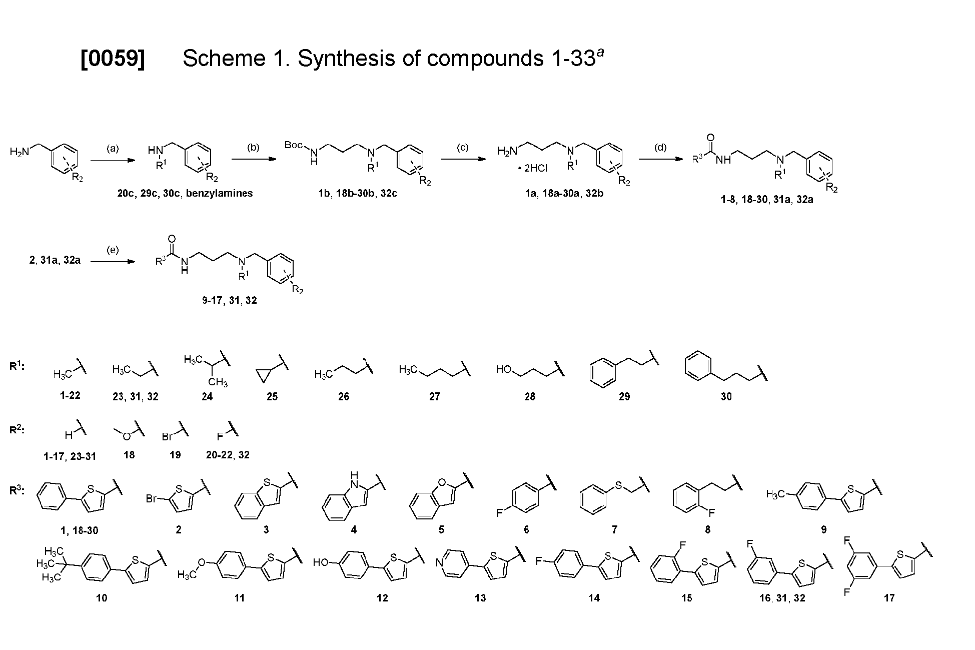

*^{a}*Reagents and conditions: (a) methanamine, 1-(bromomethyl)-4-fluorobenzene, Cs₂CO₃, KI, acetone, rt, 72 h or benzaldehyde, respective amine, NaBH₃CN, EtOH, rt, 16 h, 33%-38%; (b) *tert*-butyl (3-bromopropyl)carbamate, respective benzylamine, Cs₂CO₃, KI, acetone, rt, 72 h, 39%-69%; (c) 4M HCl in 1,4-dioxane, rt, 1 h, 52%-98%; (d) respective carboxylic acid, DIPEA, HATU, DMF, rt, 16 h, 7%-86%; (d) respective boronic acid (pinacol ester), K₂CO₃, XPhos Pd G4, water/1,4-dioxane (1:4), 100°C, 16 h, 18%-72%.

Similar to the synthesis described above (Scheme 1), compounds 33, 38, 40, 44, 45, and 49 could be obtained in a short one-step amide coupling reaction. Compound 33 was then further derivatized in two steps, boc-deprotection, and reductive amination, to compound 34.

While most compounds could be obtained as described in a straightforward manner, the remaining compounds needed to be synthesized in multiple different steps due to their heterogenic molecular structures. Compound 37 was synthesized beginning with saponification of the *N*-boc-protected piperidine carboxylic acid ethyl ester. Subsequently, an amide coupling reaction with the resulting carboxylic acid (37e) and *tert-*butyl amine gave the intermediate 37d. After the acquisition of the deprotected amine with the use of 4 M HCl in 1,4-dioxane, the compound was treated as described above: *in situ* Finkelstein reaction with nucleophile substitution, boc-deprotection, and final amide coupling to give compound 37.

Compound 39 was synthesized in 3 steps. At first, reductive amination with benzaldehyde and the boc-protected 3-methylpiperidine, afterwards boc-deprotection using HCl in 1,4-dioxane, and finally the amide coupling with the carboxylic acid.

While compounds 36, 41, and 43 share similar structural elements, their synthesis was achieved in different routes. 36 and 41 were initially synthesized in an amide coupling reaction between acetal-protected amino benzaldehyde and 4-fluorobenzoic acid. After subsequent deprotection of the acetal to the aldehyde and reductive amination with either *tert*-butyl (3-(methylamino)propyl)carbamate or *tert*-butyl 1,4-diazepane-1-carboxylate the intermediates 36b and 41b could be generated. Deprotection of the *N*-terminal boc-group and amide coupling yielded final compound 36 or 41. Conversely, 43 was synthesized in multiple steps, starting with 1-(bromomethyl)-3-nitrobenzene and employing a Gabriel synthesis using the Ing-Manske procedure to create the desired amine (precursor 42a). For easier handling, 4 M HCl in 1,4-dioxane was added to the product to precipitate the HCl salt of the amine. The precursor was then coupled with 5-phenylthiophene-2-carboxylic acid to give the compound 42. From there on, a reduction of the nitro group and a subsequent amide coupling led to compound 43.

The precursor 46a for compounds 46 and 47 was synthesized in a 2-step route. Nucleophile substitution of mono boc-protected piperazine and *N*-(3-Bromopropyl)phthalimide gave intermediate 46b. Hydrazinolysis in ethanol yielded the desired intermediate, which was either used in an amide coupling reaction to give compound 46 or in a reductive amination reaction to give compound 47. Similarly, compound 48 was generated with a reductive amination reaction of precursor 48a and phenylthiazole carbaldehyde using sodium triacetoxyborohydride and triethylamine in dichloroethane. The precursor was synthesized from the above-mentioned compound 37e, which was, in this case, coupled with benzylamine instead of *tert*-butyl amine as in the synthesis of compound 37. Subsequent deprotection gave the desired intermediate 48a.

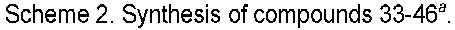

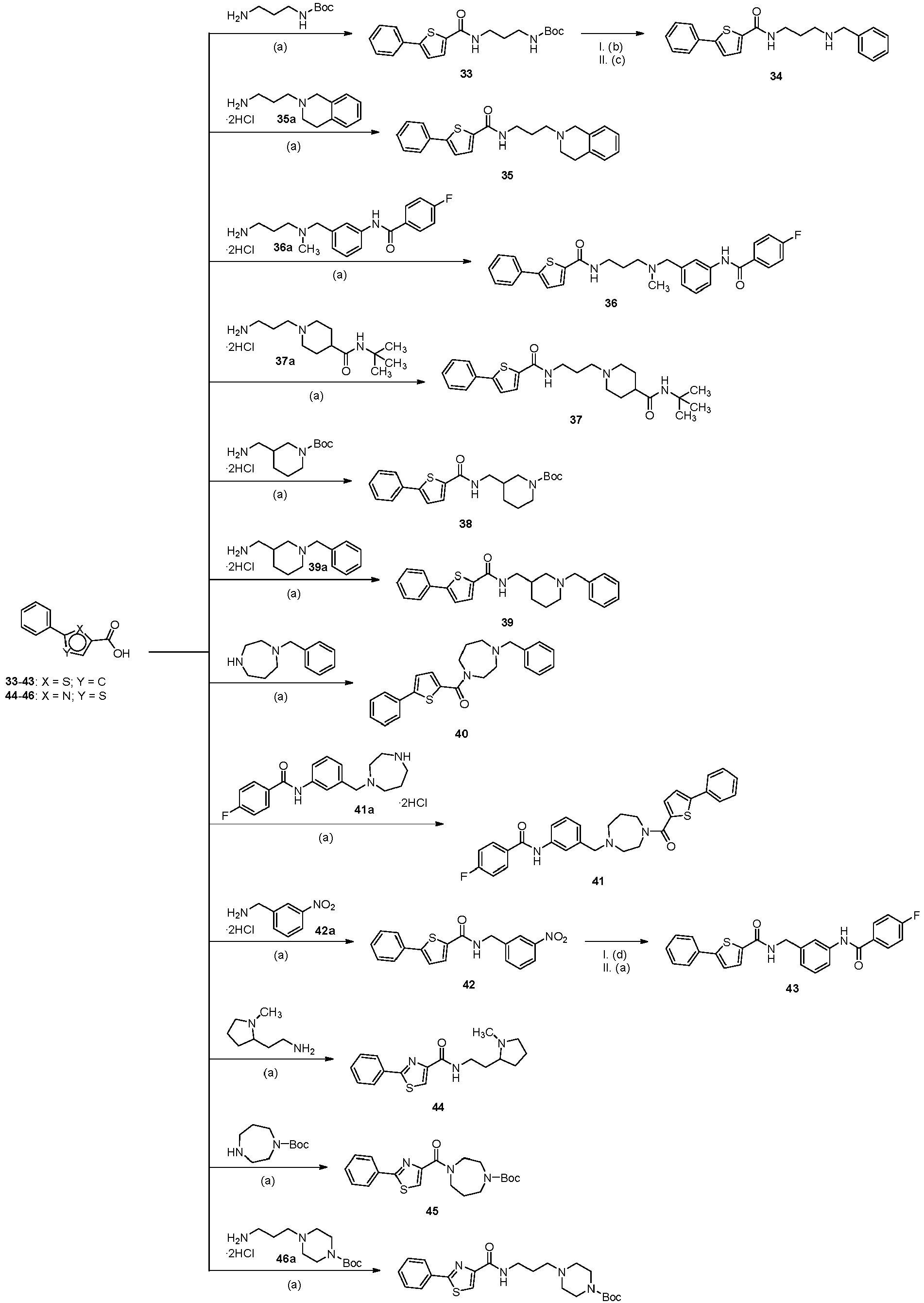

*^{a}*Reagents and conditions: (a) respective amine, DIPEA, HATU, DMF, rt, 16 h, 9%-quant.; (b) 4M HCl in 1,4-dioxane, rt, 1 h, quant.; (c) benzaldehyde, NaBH(OAc)₃, Et₃N, DCE, rt, 16 h, 30%; (d) Pd/C 10%, H₂, EtOH, rt, 16 h.

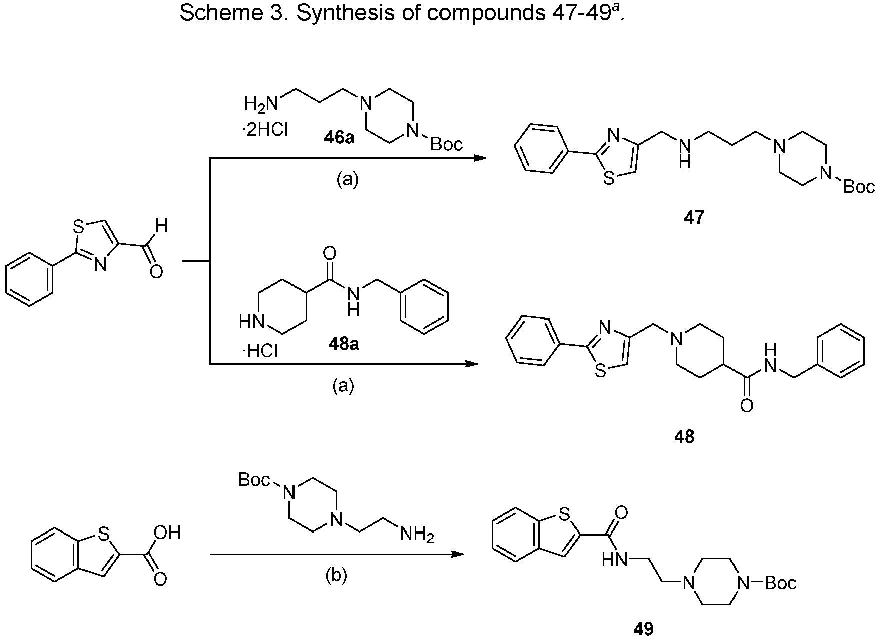

*^{a}*Reagents and conditions: (a) respective amine, NaBH(OAc)₃, AcOH or Et₃N, THF or DCE, rt, 16 h, 7%-71%; (b) amine, DIPEA, HATU, DMF, rt, 16 h, 46%.

Structure-Activity Relationship Studies of Initial Hit. Starting from the compound from Yoshikawa *et al.* (Fig. 1) (EC₅₀ = 2.21 *µ*M), it was chosen to create a more flexible core structure by deleting the *N*-methylamide moiety from the thieno[3,2-c]quinolin "head" part. This now new liberated phenylthiophene compound, 1 (EC₅₀ = 0.416 *µ*M), improved the potency in *β-Arrestin* recruitment assays by ~5 fold. However, efforts to change this moiety to different aromatic structures, 6-7, depleted the compound's *β-Arrestin* recruitment activity. Attempts to fuse the phenyl moiety with the five-membered heteroaromat (compounds 3-5) reduced the activity as well, with 3 (EC₅₀ = 0.775 *µ*M) being the best of this set of molecules. Therefore, our efforts were focused on the derivatization of the phenylthiophene moiety. Sterically demanding substituents like (*tert*-butyl)phenyl (10) and methoxyphenyl (11) diminished activity, while smaller substituents were often tolerated. Interestingly, the difluoro compound 17 (EC₅₀ = 0.758 *µ*M) lowered activity, but some single fluorinated compounds, for example, compound 16, improved it. The molecules showed regiospecific preferences. Most prominently, meta substitution (16, EC₅₀ = 0.267 *µ*M), elevated the activity by a great amount. Summarizing our findings towards "head" optimization, as described above, suggests that the phenylthiophene moiety is highly conserved in the binding of ACKR3. Only small substituents, like fluor, were tolerated. The elevated binding affinity suggests either an interaction with the fluorine in the binding pocket or that modulation of lipophilicity through fluorination may play a key role.

The right-hand side of the molecule, the "tail", was approached in a similar manner. In the beginning, the benzyl moiety was exchanged for more space-demanding motifs. This let to loss of agonistic activity, for example in compounds 36 (EC₅₀ = 2.1 *µ*M) and 37 (EC₅₀ = 3.2 *µ*M). Focusing on smaller changes similar to the "head" optimization, with substituents like p-methoxy (18), *p*-bromo (19), or *p*-fluoro (20). Again, the fluorination proved to be the best choice with the *m*-fluorobenzyl derivate (22) showing the same potency as its *m*-fluoro "head" counterpart. With lipophilicity being an important factor, a sterically more demanding, but more lipophile dihydroisoquinolin derivate (35) was synthesized. Interestingly, this compound elevated the agonistic activity to be our most potent candidate so far (EC₅₀ = 0.150 *µ*M).

Next, the optimization process was focused on the methyl substitution of the tertiary amine. At first, the binding pockets' spatial breadth was challenged with the synthesis of sterically demanding compounds 24 (EC₅₀ = 0.709 *µ*M) and 25 (EC₅₀ = 0.630 *µ*M). While both compounds were still in the nanomolar range, they reduced initial hit activity moderately. The next rational approach was to investigate the binding pocket's space by elongating the alkyl chain of the tertiary amine. Ethylamine derivative 23 (EC₅₀ = 0.111 *µ*M) greatly improved potency and encouraged further elongation of the alky chain. While propylamine derivative 26 (EC₅₀ = 0.111 *µ*M) did not enhance activity, butylamine derivative 27 (EC₅₀ = 0.069 *µ*M) showed excellent potency in the lower nanomolar range. Hereby, the long nonpolar unsaturated alkyl chains further supported our hypothesis, that lipophilicity may play a key role in the structure-activity relationship.

On the contrary, rigidification of the "linker" section abolished the *β-Arrestin* recruitment potency of the compounds. For example, 43 (EC₅₀ = 3.35 *µ*M), with an aromatic linker part and a similar structure to 36 (EC₅₀ = 2.1 *µ*M), showed moderate to low agonistic activity. Non-aromatic cyclization approaches, like diazepane derivatives 40 (EC₅₀ = 1.2 *µ*M) and 41 (EC₅₀ = 76 *µ*M), likewise lowered the potency by at least 3-fold.

Other experiments, like changing the thiophene ring to the more polar thiazole (derivatives "Structure E", table 1) led to the loss of agonistic activity. Derivatization of this scaffold of compounds resulted in a maximum EC₅₀ of 1.5 *µ*M with compound 48.

Shorter molecules were also synthesized, but not sufficient enough to be further characterized, for example 49 at 4.55 *µ*M with a short linker chain and a fused benzothiophene core.

Efficacy of Derivatives. The endogenous ligand of ACKR3, CXCL12, was set at 100% efficacy in *β-Arrestin* recruitment assays to serve as the standard for the determination of each compound's efficacy. As shown in Table 1, most of the tested compounds were found to be full agonists of ACKR3. Hit compound 1 already showed a good efficacy of 71% and was improved further alongside the optimization efforts for *β-Arrestin* recruitment and platelet degranulation activity. Most changes were tolerated well and did not influence the efficacy of compounds. Notably, highly potent compounds like 16 (*E*ₘₐₓ = 103%), 23 (*E*ₘₐₓ = 95%), or 26 (*E*ₘₐₓ = 96%) showed full-agonistic properties for ACKR3. With 82% efficacy, the lead compound 27 proved to be an ACKR3 full-agonist while displaying excellent *β-Arrestin* recruitment potency in the lower nanomolar range.

Platelet Degranulation Studies of Derivatives. Concurrent with the *β-Arrestin* recruitment potency optimization, the compounds were also tested and developed according to their functional activity. To determine the ability of a compound to modulate platelets, P-Selectin surface expression was measured. P-Selectin is a surface protein involved in cell adhesion mechanisms and can be found on activated platelets. After induction of platelet aggregation in platelet-rich plasma (PRP), treatment with the respective compound should therefore lead to a significant reduction in P-Selectin expression. Fluorochrome-conjugated antibodies against P-Selectin were used to determine P-Selectin concentration compared to untreated PRP. The initial hit compound 1 (P-Selectin expression reduction = 71%) proved to be a potent platelet degranulation inhibitor. Although structural changes of the "head" moiety (Structure A, Table 1) of 1 improved the compound's *β-Arrestin* recruitment activity, functional activity was preserved, and in some cases even upgraded to nearly 100% P-Selectin expression reduction. Especially, compounds 16 and 31 showed high platelet activity while being up to 1.5-fold more potent than 1. Only compound 5 of the tested compounds from the "head"-scaffold were inactive in terms of functional activity. Otherwise, the compounds showed resistance against changes in the left-hand side of the molecule. On the contrary, changes in the "tail" compartment, showed a major influence in terms of platelet modulation activity. Further rigidization, like compound 35, led to decreased functional activity, while substitution of the benzyl moiety in the para-position seemed to enhance P-Selectin expression reduction. Compound 20 (EC₅₀ = 0.370 µM; P-Selectin expression reduction = 86%), is the best molecule of this group considering both *β-Arrestin* recruitment and flow cytometry assays. Interestingly, in contrast to 20's para-fluorination, meta, and ortho fluorination decreased function activity in compounds 22 with 66% reduction and 21 with 40% reduction. Similarly, platelet degranulation potency was highly sensitive toward "linker"-changes (Structure C, Table 1). A distinct trend could not be registered. With compounds 23, 26, and 27 as the most potent molecules in terms of *β-Arrestin* recruitment, they proved to be potent platelet modulators as well. Compared to the commercially available reference compound VUF11207 (P-Selectin expression reduction = 65%), most compounds showed superior functional activity.

Indeed, 27 showed excellent *β-Arrestin* recruitment activity (EC₅₀ = 69 nM) and high P-selection expression reduction (77%) and was found to be the most interesting substance for further evaluations.

**Table 1. Structure-activity relationship studies.**

| 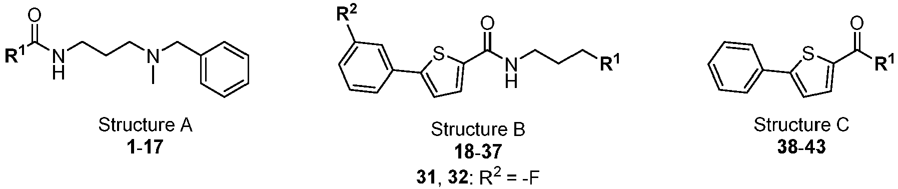 | | | | |
|---|---|---|---|---|
| 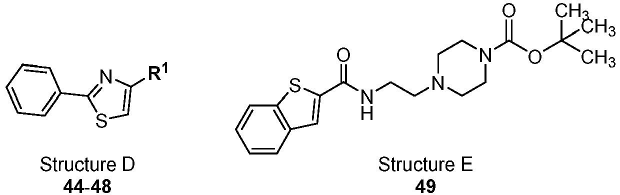 | | | | |
| Compound | R¹ | ACKR3 | | Flow cytometry |
| | | *β*-Arrestin recruitment EC₅₀ (µM)*^{a}* | Efficacy (Eₘₐₓ) *vs* CXCL12 (%)*^{b}* | P-Selectin expression reduction (%)*^{c}* |
| **Structure A: Head optimization** | | | | |
| **VUF11207** | 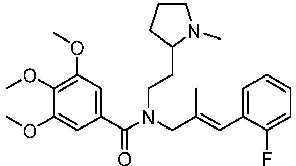 | 0.0436 | 95 | 65±6 |
| 1 | 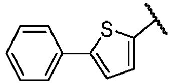 | 0.416 | 73 | 74±9 |
| **2** | 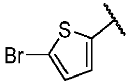 | 1.62 | 80 | - |
| **3** | 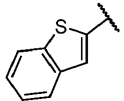 | 0.775 | 89 | 90 |
| **4** | 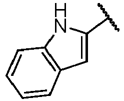 | 2.67 | 81 | 80 |
| **5** | 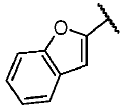 | 1.48 | 83 | 35 |
| **6** | 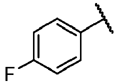 | 16.97 | 80 | - |
| **7** | 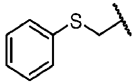 | 809 | 99 | - |
| **8** | 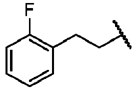 | 6.42 | 90 | - |
| **9** | 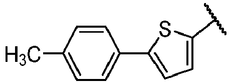 | 0.576 | 93 | 91±4 |
| **10** | 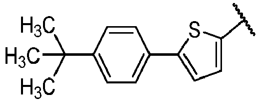 | 10.23 | 46 | - |
| **11** | 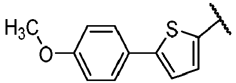 | 2.13 | 70 | - |
| **12** | 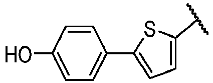 | 1.75 | 64 | - |
| **13** | 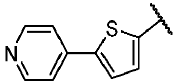 | 1.72 | 80 | - |
| **14** | 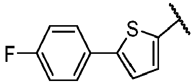 | 0.382 | 33 | 87±7 |
| **15** | 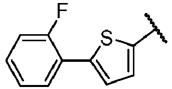 | 0.507 | 62 | 71±11 |
| **16** | 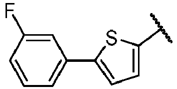 | 0.267 | 103 | 88±4 |
| **17** | 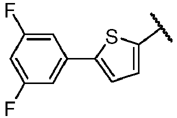 | 0.758 | 86 | 81±9 |

| **Structure B: Tail optimization** | | | | |
|---|---|---|---|---|
| **18** | 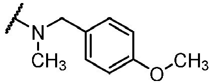 | 0.930 | 93 | 82±6 |
| **19** | 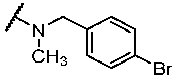 | 0.668 | 76 | 89±3 |
| **20** | 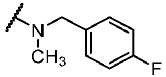 | 0.370 | 96 | 86±6 |
| **21** | 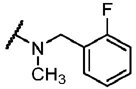 | 0.302 | 85 | 40±10 |
| **22** | 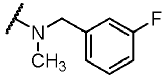 | 0.249 | 81 | 66±14 |
| **23** | 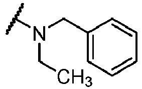 | 0.111 | 95 | 87±6 |
| **24** | 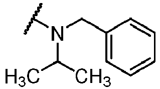 | 0.709 | 84 | 90±3 |
| **25** | 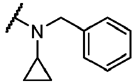 | 0.630 | 64 | 16±7 |
| **26** | 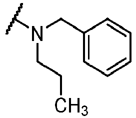 | 0.111 | 96 | 76±7 |
| **27** | 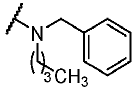 | 0.069 | 82 | 77±6 |
| **28** | 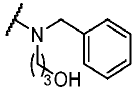 | 0.413 | - | 85±4 |
| **29** | 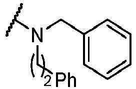 | 0.459 | - | 2±8 |
| **30** | 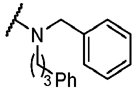 | 1.060 | - | 1±5 |
| **31** | 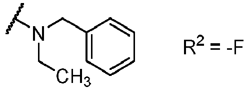 | 0.334 | 85 | 95±1 |
| **32** | 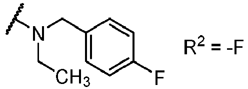 | 0.846 | 78 | 96±1 |
| **33** | 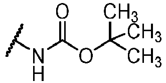 | no activity | - | 0±4 |
| **34** | 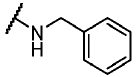 | 1.964 | - | 95±1 |
| **35** | 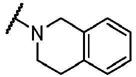 | 0.150 | 95 | 27±9 |
| **36** | 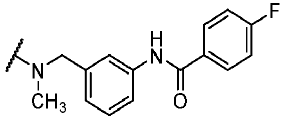 | 1.1 | 86 | - |
| **37** | 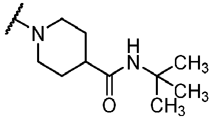 | 2.5 | 102 | - |

| **Structure C: Linker optimization** | | | | |
|---|---|---|---|---|
| **38** | 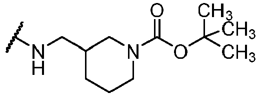 | 4.677 | 18 | - |
| **39** | 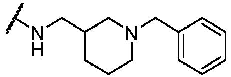 | 1.220 | - | 42±9 |
| **40** | 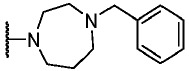 | 1.2 | 32 | 76 |
| **41** | 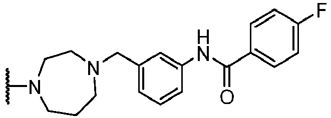 | 89 | 50 | - |
| **42** | 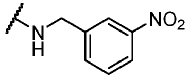 | 0.790 | 67 | 17±3 |
| **43** | 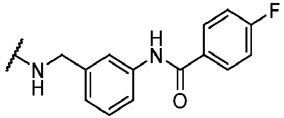 | 3.35 | 53 | - |

| **Structure D: Thiazole derivates** | | | | |
|---|---|---|---|---|
| **44** | 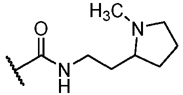 | 3 | 121 | - |
| **45** | 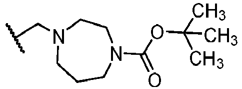 | 5 | 29 | 93 |
| **46** | 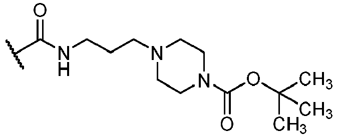 | 2.7 | 65 | 86 |
| **47** | 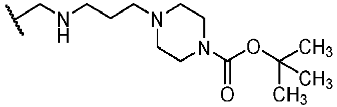 | 2.4 | 89 | 100 |
| **48** | 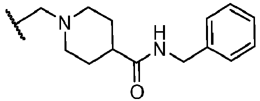 | 1.1 | 110 | - |

| **Structure E: Shortened Linker** | | | | |
|---|---|---|---|---|
| **49** | for structure see above | 4.55 | 25 | 70 |

| | | | | |
|---|---|---|---|---|
| *^{a}*Average value of three independent experiments. *^{b}*The efficacy of CXCL12 at 300 nM was set as 100%; the average value of three independent experiments. *^{c}*PRP was pre-incubated with compound (100 µM) and then treated with CRP-XL (1 µg/mL). Data are expressed as a percentage of P-Selectin-MFI (% ±SEM) normalized to CRP-XL-treated vehicle PRP (DMSO 0.1%) from at least three individual experiments in duplicate. Dash (-) = Not tested. | | | | |

Selectivity studies. To evaluate the selectivity of chosen most promising compounds, the compounds were tested against a variety of receptors, ACKR2, CXCR3, and CXCR4. Especially, CXCR4 selectivity was desired, since its activation often led to opposite physiological effects compared to ACKR3.

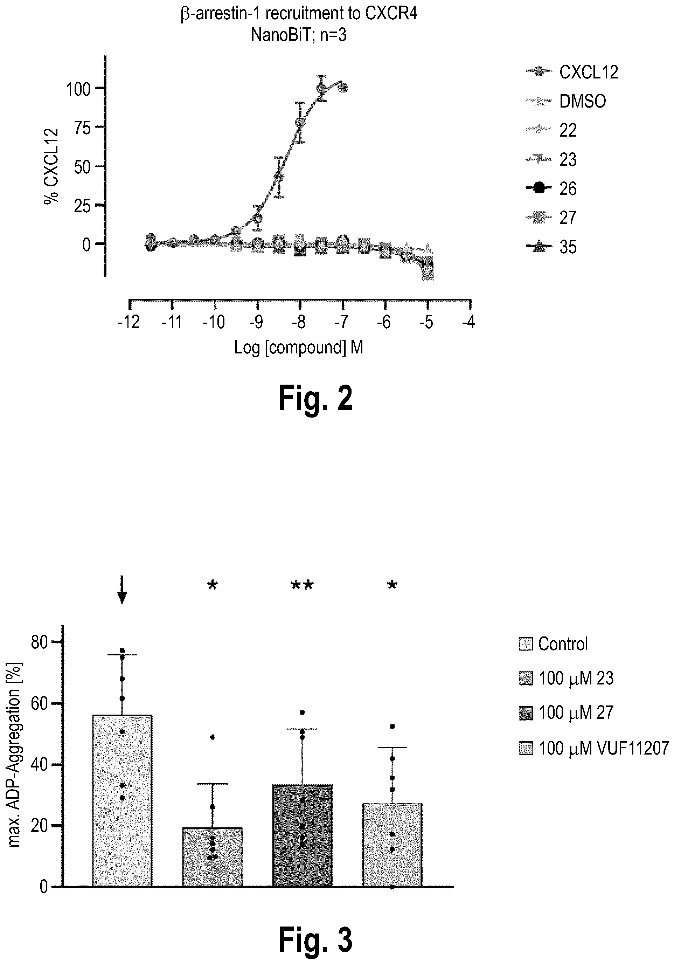

All compounds showed high selectivity against stated receptors. Their potency for these receptors was over the measuring limit of our assay and determined higher than 10 µM for all tested compounds as shown in Table 2 and Fig. 2.

**Table 2: selectivity studies**

| | | *β-Arrestin* recruitment EC₅₀ (µM) | | | |
|---|---|---|---|---|---|
| Cmpd | Structure | ACKR3 | ACKR2 | CXCR3 | CXCR4 |
| 22 | 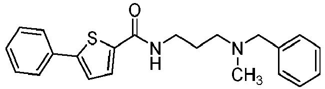 | 0.249 | > 10 | > 10 | > 10 |
| 23 | 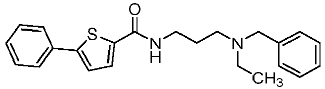 | 0.111 | > 10 | > 10 | > 10 |
| 26 | 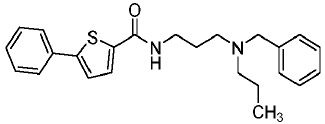 | 0.111 | > 10 | > 10 | > 10 |
| 27 | 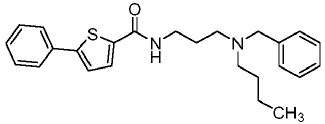 | 0.069 | > 10 | > 10 | > 10 |
| 35 | 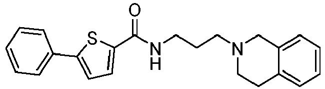 | 0.150 | > 10 | > 10 | > 10 |

| | | | | | |
|---|---|---|---|---|---|
| *^{a}*Average value of three independent experiments (µM). | | | | | |

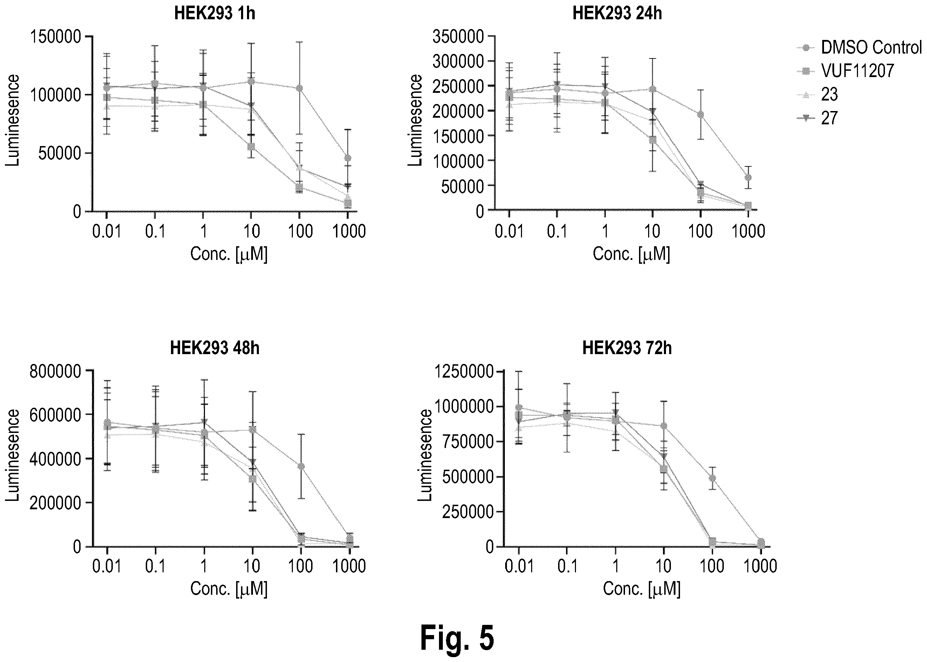

Cell viability studies. For further evaluations, the compounds 23 and 27, were tested in cell viability assays to determine their toxicity. In HEK293 cells, both compounds showed superior qualities compared to VUF11207 in terms of cell toxicity. Both tested compounds were tolerated well by the HEK293 cells in concentrations of 1 µM and below. Even longer testing periods of up to 72 h showed no influence on the cells during compound treatment (Fig. 5).

Platelet aggregation studies. To further evaluate the compounds inhibitory potential of platelet aggregation, aggregation experiments were conducted. All tested compounds, including reference compound VUF11207, showed a reduction of platelet aggregation compared to the control. Notably, compound 23 showed the highest potency for platelet aggregation inhibition. Combined with its excellent potency in FACS experiments, compound 23 shows great potential as a tool compound as shown in Fig. 3.

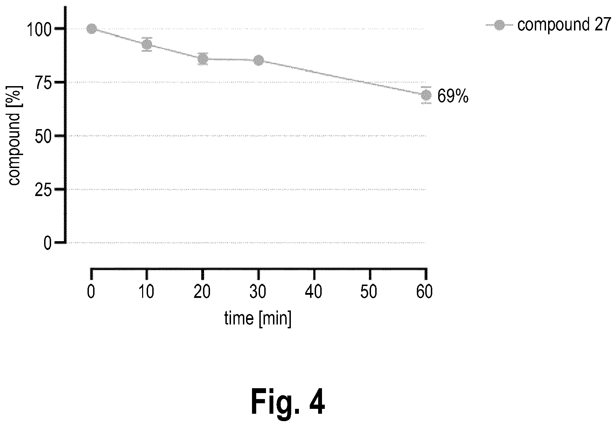

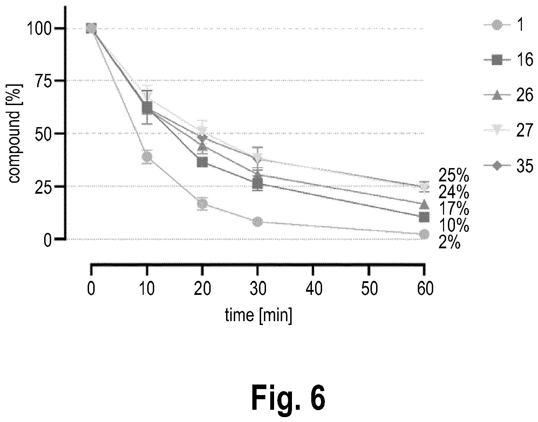

Metabolic stability studies. As shown in Fig. 4, compound 27 was stable in human liver microsomes over the course of 60 min. Around 69% of compound 27 remained, while the rest was determined to be a mixture of different metabolites. The most prominent metabolite was identified to be the de-benzylated compounds, while the dealkylation of the *N*-butyl chain took second place in terms of metabolite quantity. Subsequently, the metabolism of the compound further using mouse liver microsomes was studied. The substance showed weaker stability properties and remained in quantities around 24% after 60 min in this assay. Concluding, while the compound has already great characteristics in terms of human metabolic activity, it requires further optimization efforts toward mouse liver microsome stability. In addition, *in vitro* metabolic stability studies of compounds 1, 12, 23, 29, and 30 in mouse liver microsomes are shown (Fig. 6).

Solubility studies: The solubility of compounds 26, 30 has been tested (Table 3). Both compounds show good solubility in a mixture 1 vol.% DMSO and 99 vol.% PBS buffer.

**Table 3. Kinetic solubility studies of selected compounds.**

| Compound | Structure | Solubility [µM]*^{a}* | Solubility [mg/ml]*^{a}* |
|---|---|---|---|
| 23 | 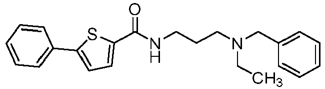 | 75±1 | 0.03 |
| 27 | 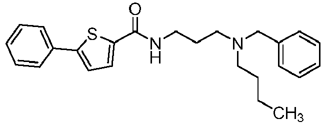 | 28±4 | 0.01 |

| | | | |
|---|---|---|---|
| *^{a}*Average value of three independent experiments (µM) ± SEM). The compound was solved in 1% DMSO, mixed with 99% PBS buffer (pH 7.4), then centrifuged at 16,000 g for 20 min. Solubility was determined in comparison to a standard curve of the respective compound. | | | |

It is to be understood that the before-mentioned features and those to be mentioned in the following cannot only be used in the combination indicated in the respective case, but also in other combinations or in an isolated manner without departing from the scope of the invention.

The invention is now further explained by means of embodiments resulting in additional features, characteristics, and advantages of the invention. The embodiments are of pure illustrative nature and do not limit the scope or range of the invention. The features mentioned in the specific embodiments are general features of the invention which are not only applicable in the specific embodiment but also in an isolated manner and in the context of any embodiment of the invention.

The invention is now described and explained in further detail by referring to the following non-limiting examples and figures.

### BRIEF DESCRIPTION OF THE FIGURES

Fig. 1 shows the optimization process starting with the compound developed by Yoshikawa Y. *et al.*
Fig. 2 shows β-Arrestin recruitment activity of selected most potent compounds against CXCR4.
Fig. 3 shows the aggregation of platelets after preincubation with 100 µM ACKR3 agonist and subsequent activation with 2.5 µM ADP.
Fig. 4 shows *in vitro* metabolic stability studies of lead compound 27 in human liver microsomes.
Fig. 5 shows cell viability assays of compounds 23 and 27 in comparison to reference compound VUF11207 over 1 h, 24 h, 48 h, and 72h.
Fig. 6 shows *in vitro* metabolic stability studies of compounds 1, 16, 26, 27, and 35 in mouse liver microsomes.

### EXAM PLES

### Chemistry and Methods.

Starting materials, reagents, and (anhydrous) solvents were commercially available and purchased from a range of manufacturers and used without further purification. Thin-layer chromatography (TLC) with Macherey-Nagel precoated 60 F254 silica plates was used for reaction controls. TLC-Spots were visualized either by ultraviolet (UV) light (254 nm/365 nm) or staining solutions. For purification purposes, flash column chromatography was carried out using Grace Davison Davisil LC60A (20-45 *µ*m) or Merck Geduran Si60 (mesh 63-200 *µ*m) using a LaFlash (VWR International GmbH, Darmstadt, Germany) automated flash chromatography system. NMR spectra were recorded on a Bruker Avance 400 MHz spectrometer at ambient temperature. Chemical shifts (*δ*) are reported in parts per million (ppm) relative to the internal control tetramethylsilane (TMS), and the spectra were calibrated against the residual solvent peak of the used deuterated solvent (either DMSO-d6 or CDCl₃). Coupling constants (*J*) are expressed in hertz (Hz). The purity of all compounds was determined by RP-HPLC using an Agilent 1100 Series LC with a Phenomenex Luna C8 analytical column (150 x 4.6 mm, 5 *µ*m) and detected by a UV DAD detector at 254 nm and 230 nm wavelength. Elution was carried out with the following gradient: [A = 0.01 M KH₂PO₄, pH 2.30, B = MeOH] 40% B to 85% B in 8 min, 85% B for 5 min, 85% to 40% B in 1 min, 40% B for 2 min, stop time 16 min, flow 1.5 mL/min. Mass spectra analysis were obtained from an Advion expression compact mass spectrometer (electrospray ionization, ESI) with a TLC plate reader system (using the following settings: ESI voltage 3.50 kV, capillary voltage 187 V, source voltage 44 V, capillary temperature 250 °C, desolvation gas temperature 250 °C, gas flow 5 L/min). High-resolution mass spectra (HRMS) were determined for final compounds by the mass spectrometry department, Institute of Organic Chemistry, Eberhard Karls University Tübingen on a Bruker maXis 4G ESI-TOF (Bruker Daltonik GmbH, Bremen, Germany). The instrument was operated in ESI positive mode, and settings were as follows: nebulizer gas 1.2 bar, gas flow 6.0 L/min, source temperature 200 °C, capillary voltage +4500 V, end plate offset -500 V. The m/z range was from 80 to 1050 m/z. All final compounds are >95% pure determined by above mentioned HPLC procedure. Compound NMR spectra are accessible in the SI Pages S26-S68. Synthesis of precursors are available in SI Pages S4-S25.

Synthesis. *General Procedure I: Amide Coupling.* The carboxylic acid was dissolved in dry DMF (2.5 mL) and HATU (1.2 eq.) was slowly added to the solution. After 15 min of continuous stirring, amine (1 eq.) and DIPEA (3 eq.) were added to the mixture. The mixture was allowed to stir at room temperature overnight. After complete reaction, the mixture was diluted with DCM (10 mL) and washed with water (3x 10 mL) and brine (3x 10 mL). The combined organic phases were dried over anhydrous Na₂SO₄, filtered, and evaporated to dryness. The crude product was purified via flash chromatography or suction filtration.

*General Procedure II: Suzuki Coupling.* To a vial containing the organo-halide, the boronic acid or boronic acid pinacol ester (1.1 eq.), XPhos Pd G4 (0.05 eq.), and K₂CO₃ (3 eq.) were added 1,4-dioxane (2 mL) and water (0.5 mL). The mixture was degassed with argon and heated to 100 °C for 16 h. After complete reaction, the mixture was diluted with EtOAc (10 mL) and washed with water (3x 10 mL) and brine (3x 10 mL). The combined organic phases were dried over anhydrous Na₂SO₄, filtered, and evaporated to dryness. The crude product was purified via flash chromatography (SiO₂; DCM/MeOH).

*General Procedure III: Reductive Amination.* The carbaldehyde (1.1 eq.) was dissolved in THF or DCE (5 mL) and one drop of acetic acid or triethylamine was added to the solution. After 15 min of continuous stirring, the amine was added, and the mixture was allowed to stir for 1 h. The mixture was cooled to 0 °C and sodium triacetoxyborohydride (1.5 eq.) was added. After complete reaction, the mixture was diluted with EtOAc (10 mL) and washed with water (3x 10 mL) and brine (3x 10 mL). The combined organic phases were dried over anhydrous Na₂SO₄, filtered, and evaporated to dryness. The crude product was purified via flash chromatography (SiO₂; DCM/MeOH).

*N-(3-(benzyl(methyl)amino)propyl)-5-phenylthiophene-2-carboxamide (1)*. Prepared from 5-phenylthiophene-2-carboxylic acid (0.84 mmol) and *N*¹-benzyl-*N*¹-methylpropane-1,3-diamine dihydrochloride (1a, 0.84 mmol, 1 eq.) according to general procedure 1. Purification by flash chromatography with DCM/MeOH (2-7%). Yield: 150 mg (49%), yellow semi-solid. ¹H NMR (400 MHz, CDCl₃) δ 8.02 (s, 1H), 7.59 (d, *J* = *7.2* Hz, 2H), 7.46 - 7.28 (m, 9H), 7.21 (d, *J* = 3.9 Hz, 1H), 3.74 (s, 2H), 3.56 (q, *J* = 11.4, 5.5 Hz, 2H), 2.80 (t, 2H), 2.42 (s, 3H), 1.92 (quint, 2H). ¹³C NMR (101 MHz, CDCl₃) δ 162.84, 149.07, 137.46, 135.50, 133.70, 130.00, 129.48, 129.17, 128.95, 128.61, 128.43, 126.22, 123.72, 62.68, 56.20, 40.74, 39.30, 25.16. TLC-MS (ESI+): calcd. *m*/*z* 364.16 for C₂₀H₂₄N₂OS. Found 365.2 [M+H]⁺. HPLC *t*_{R} = 5.778 min.

*N-(3-(benzyl(methyl)amino)propyl)-5-bromothiophene-2-carboxamide (2)*. Prepared from 5-bromothiophene-2-carboxylic acid (1.04 mmol) and *N*¹-benzyl-*N*¹-methylpropane-1,3-diamine dihydrochloride (1a, 1.04 mmol, 1 eq.) according to general procedure 1. Purification by flash chromatography with DCM/MeOH (3-7%). Yield: 235 mg (53%), pale brown solid. ¹H NMR (400 MHz, CDCl₃) δ 8.24 (s, 1H), 7.36-7.26 (m, 5H), 6.93 (dd, *J* = 12.9, 3.9 Hz, 2H), 3.54 (s, 2H), 3.50 (q, *J* = 10.4, 4.1 Hz, 2H), 2.61 (t, 2H), 2.27 (s, 3H), 1.78 (quint, *J* = 11.6, 5.8 Hz, 2H). ¹³C NMR (101 MHz, CDCl₃) δ 160.85, 141.21, 138.02, 130.58, 129.62, 128.62, 127.84, 127.68, 117.28, 63.37, 57.37, 50.97, 41.62, 40.66, 25.05. TLC-MS (ESI+): calcd. *m*/*z* 366.04/369.04 for C₁₆H₁₉BrN₂OS. Found 367.1/369.2 [M+H]⁺. HPLC *t*_{R} = 4.709 min.

*N-(3-(benzyl(methyl)amino)propyl)benzo[b]thiophene-2-carboxamide (3).* Prepared from benzo[*b*]thiophene-2-carboxylic acid (0.6 mmol) and *N*¹-benzyl-*N*¹-methylpropane-1,3-diamine dihydrochloride (1a, 0.6 mmol, 1 eq.) according to general procedure 1. Purification by flash chromatography with DCM/MeOH (2-5%). Yield: 73 mg (36%), yellow solid. ¹H NMR (400 MHz, CDCl₃) δ 8.41 (t, 1H), 7.82 (d, *J* = 7.6 Hz, 1H), 7.71 (d, *J* = 7.3 Hz, 1H), 7.60 (s, 1H), 7.44 - 7.33 (m, 4H), 7.34 - 7.27 (m, 3H), 3.70 (s, 2H), 3.58 (q, *J* = 10.9, 5.3 Hz, 2H), 2.77 (t, *J* = 5.7 Hz, 2H), 2.39 (s, 3H), 1.91 (quint, 2H). ¹³C NMR (101 MHz, CDCl₃) δ 162.7, 141.0, 139.4, 139.1, 130.0, 128.8, 128.2, 126.2, 125.2, 125.1, 124.8, 122.7, 62.8, 56.5, 41.0, 39.9, 25.0. TLC-MS (ESI+): calcd. m/z 338.15 for C₂₀H₂₂N₂OS. Found 339.2 [M+H]⁺. HPLC *t*_{R} = 4.067 min.

*N-(3-(benzyl(methyl)amino)propyl)-1H-indole-2-carboxamide (4)*. Prepared from 1*H*-indole-2-carboxylic acid (0.84 mmol) and *N*¹-benzyl-*N*¹-methylpropane-1,3-diamine dihydrochloride (1a, 0.84 mmol, 1 eq.) according to general procedure 1. Purification by flash chromatography with DCM/MeOH (2-7%). Yield: 151 mg (56%), yellow solid. ¹H NMR (400 MHz, CDCl₃) δ 9.73 (s, 1H), 8.20 (t, 1H), 7.47 (d, *J* = 8.0 Hz, 1H), 7.37 (d, *J* = 8.2 Hz, 1H), 7.32 - 7.22 (m, *J* = 14.0, 6.8, 3.5 Hz, 5H), 7.18 (td, *J* = 15.3, 0.9 Hz, 1H), 7.04 (t, *J* = 7.5 Hz, 1H), 6.55 (s, 1H), 3.56 (s, 2H), 3.52 (q, *J* = 11.3, 5.5 Hz, 2H), 2.73 - 2.55 (m, 2H), 2.26 (s, 3H), 1.78 (quint, 2H). ¹³C NMR (101 MHz, CDCl₃) δ 162.14, 136.90, 136.49, 131.06, 129.86, 128.82, 128.03, 127.80, 124.34, 121.99, 120.54, 112.18, 102.62, 63.03, 56.85, 41.10, 39.65, 25.25. TLC-MS (ESI+): calcd. *m*/*z* 318.18 for C₂₀H₂₃N₃O. Found 322.2 [M+H]⁺. HPLC *t*_{R} = 1.089 min.

*N-(3-(benzyl(methyl)amino)propyl)benzofuran-2-carboxamide* (5). Prepared from benzofuran-2-carboxylic acid (0.16 mmol) and *N*¹-benzyl-*N*¹-methylpropane-1,3-diamine dihydrochloride (1a, 0.16 mmol, 1 eq.) according to general procedure 1. Purification by flash chromatography with DCM/MeOH (0-4%). Yield: 15 mg (30%), yellow semi-solid. ¹H NMR (400 MHz, CDCl₃) δ 8.23 (s, 1H), 7.62 (d, *J* = 7.8 Hz, 1H), 7.44 - 7.15 (m, 9H), 3.61 (s, 2H), 3.56 (q, *J* = 11.2, 5.5 Hz, 2H), 2.64 (t, *J* = 5.9 Hz, 2H), 2.30 (s, 3H), 1.85 (quint, 2H). ¹³C NMR (101 MHz, CDCl₃) δ 159.2, 154.9, 149.3, 129.6, 128.7, 127.8, 127.7, 126.7, 123.7, 122.7, 111.9, 110.0, 63.0, 56.4, 41.6, 39.2, 29.8, 25.7. TLC-MS (ESI+): calcd. *m*/*z* 322.17 for C₂₀H₂₂N₂O₂. Found 323.2 [M+H]⁺. HPLC *t*_{R} = 4.123 min.

*N-(3-(benzyl(methyl)amino)propyl)-4-fluorobenzamide (6).* Prepared from 4-fluorobenzoic acid (0.33 mmol) and *N*¹-benzyl-*N*¹-methylpropane-1,3-diamine dihydrochloride (1a, 0.33 mmol, 1 eq.) according to general procedure 1. Purification by flash chromatography with DCM/MeOH (3-7%). Yield: 41 mg (41%), brown semi-solid. ¹H NMR (400 MHz, CDCl₃) δ 8.17 (s, 1H), 7.72 - 7.60 (m, *J=* 8.4, 5.5 Hz, 2H), 7.31 - 7.18 (m, *J* = 1.8 Hz, 5H), 6.99 (t, *J* = 8.6 Hz, 2H), 3.63 - 3.46 (m, 4H), 2.64 (t, 2H), 2.26 (s, 3H), 1.82 (quint, *J* = 11.4, 5.8 Hz, 2H). ¹³C NMR (101 MHz, CDCl₃) δ 166.29, 165.86, 163.37, 137.98, 130.90, 130.87, 129.53, 129.38, 129.29, 128.56, 127.60, 115.53, 115.32, 63.22, 57.21, 41.53, 40.45, 25.25. TLC-MS (ESI+): calcd. *m*/*z* 300.16 for C₁₈H₂₁FN₂O. Found 301.2 [M+H]⁺. HPLC *t*_{R} = 3.618 min.

*N-(3-(benzyl(methyl)amino)propyl)-2-(phenylthio)acetamide* (7). Prepared from 2-(phenylthio)acetic acid (0.36 mmol) and *N*¹-benzyl-*N*¹-methylpropane-1,3-diamine dihydrochloride (1a, 0.36 mmol, 1 eq.) according to general procedure 1. Purification by flash chromatography with DCM/MeOH (1-4%). Yield: 28 mg (24%), yellow oil. ¹H NMR (400 MHz, CDCl₃) δ 7.71 (s, 1H), 7.32 - 7.13 (m, 10H), 3.59 (s, 2H), 3.41 (s, 2H), 3.30 (q, *J* = 12.1, 5.9 Hz, 2H), 2.32 (t, *J=* 6.3 Hz, 2H), 2.09 (s, 3H), 1.60 (quint, 2H). ¹³C NMR (101 MHz, CDCl₃) δ 167.87, 138.30, 135.31, 129.32, 129.31, 128.43, 127.57, 127.34, 126.36, 62.85, 55.35, 41.93, 39.11, 37.12, 26.03. TLC-MS (ESI+): calcd. *m*/*z* 328.16 for C₁₉H₂₄N₂OS. Found 329.2 [M+H]⁺. HPLC *t*_{R} = 4.079 min.

*N-(3-(benzyl(methyl)amino)propyl)-3-(2-fluorophenyl)propanamide (8).* Prepared from 3-(2-fluorophenyl)propanoic acid (1.67 mmol) and *N*¹-benzyl-*N*¹-methylpropane-1,3-diamine dihydrochloride (1a, 1.67 mmol, 1 eq.) according to general procedure 1. Purification by flash chromatography with DCM/MeOH (2-6%). Yield: 470 mg (86%), brown semi-solid. ¹H NMR (400 MHz, CDCl₃) δ 7.32 - 7.19 (m, 5H), 7.19 - 7.09 (m, 2H), 7.04 - 6.89 (m, 2H), 6.72 (t, 1H), 3.47 (s, 2H), 3.25 (q, 2H), 2.91 (t, *J* = 7.7 Hz, 2H), 2.42 (t, *J* = 6.1 Hz, 2H), 2.36 (t, 2H), 2.17 (s, 3H), 1.61 (quint, 2H). ¹³C NMR (101 MHz, CDCl₃) δ 172.28, 162.39, 159.96, 137.72, 130.87, 130.82, 129.31, 128.61, 128.10, 128.02, 127.87, 127.71, 127.66, 124.19, 124.15, 115.40, 115.18, 62.61, 55.41, 41.51, 38.57, 36.80, 25.68, 25.33, 25.31. TLC-MS (ESI+): calcd. *m*/*z* 328.20 for C₂₀H₂₅FN₂O. Found 329. [M+H]⁺. HPLC *t*_{R} = 4.507 min.

*N-(3-(benzyl(methyl)amino)propyl)-5-(p-tolyl)thiophene-2-carboxamide (9).* Prepared from *N*-(3-(benzyl(methyl)amino)propyl)-5-bromothiophene-2-carboxamide (2, 0.22 mmol) and *p*-tolylboronic acid (0.24 mmol, 1.1 eq.) according to general procedure 2. Purification by flash chromatography with DCM/MeOH (4-8%). Yield: 30 mg (33%), brown semi-solid. ¹H NMR (400 MHz, CDCl₃) δ 8.06 (s, 1H), 7.35 (d, *J* = 7.2 Hz, 1H), 7.29 - 7.20 (m, 9H), 6.92 (d, *J* = 3.8 Hz, 1H), 3.56 - 3.48 (m, 4H), 2.58 (t, 2H), 2.39 (s, 3H), 2.27 (s, 3H), 1.78 (quint, 2H). ¹³C NMR (101 MHz, CDCl₃) δ 161.93, 147.40, 138.91, 138.25, 136.17, 133.59, 130.98, 130.42, 129.56, 128.53, 128.51, 128.02, 127.49, 126.91, 126.14, 63.30, 57.04, 41.82, 40.43, 25.43, 21.19. TLC-MS (ESI+): calcd. *m*/*z* 378.18 for C₂₃H₂₆N₂OS. Found 379.2 [M+H]⁺. HPLC *t*_{R} = 6.242 min.

*N-(3-(benzyl(methyl)amino)propyl)-5-(4-(tert-butyl)phenyl)thiophene-2-carboxamide (10).* Prepared from *N*-(3-(benzyl(methyl)amino)propyl)-5-bromothiophene-2-carboxamide (2, 0.22 mmol) and (4-(*tert*-butyl)phenyl)boronic acid (0.25 mmol, 1.1 eq.) according to general procedure 2. Purification by flash chromatography with DCM/MeOH (6-10%). Yield: 27 mg (26%), brown solid. ¹H NMR (400 MHz, CDCl₃) δ 8.06 (t, 1H), 7.43 (dd, *J* = 45.9, 8.4 Hz, 4H), 7.28 - 7.20 (m, 6H), 7.11 (d, *J* = 3.8 Hz, 1H), 3.54 - 3.46 (m, 4H), 2.57 (t, *J* = 5.8 Hz, 2H), 2.25 (s, 3H), 1.76 (quint, 2H), 1.30 (s, 9H). ¹³C NMR (101 MHz, CDCl₃) δ 161.94, 151.72, 148.49, 138.17, 137.85, 131.05, 129.62, 128.86, 128.57, 127.53, 126.04, 125.91, 122.99, 63.30, 57.10, 41.74, 40.45, 34.80, 31.35, 25.38. TLC-MS (ESI+): calcd. *m*/*z* 420.22 for C₂₆H₃₂N₂OS. Found 421.2 [M+H]⁺. HPLC *t*_{R} = 7.967 min.

*N-(3-(benzyl(methyl)amino)propyl)-5-(4-methoxyphenyl)thiophene-2-carboxamide (11).* Prepared from *N*-(3-(benzyl(methyl)amino)propyl)-5-bromothiophene-2-carboxamide (2, 0.19 mmol) and (4-methoxyphenyl)boronic acid (0.21 mmol, 1.1 eq.) according to general procedure 2. Purification by flash chromatography with DCM/MeOH (4-8%). Yield: 57 mg (71%), yellow-brown solid. ¹H NMR (400 MHz, CDCl₃) δ 7.95 (t, 1H), 7.50 - 7.42 (m, 2H), 7.30 - 7.16 (m, *J* = 12.8, 7.9, 4.7 Hz, 6H), 7.01 (d, *J* = 3.8 Hz, 1H), 6.90 - 6.82 (m, 2H), 3.78 (s, 3H), 3.53 - 3.45 (m, 4H), 2.55 (t, 2H), 2.23 (s, 3H), 1.74 (quint, 2H). ¹³C NMR (101 MHz, CDCl₃) δ 161.92, 159.98, 148.39, 138.33, 137.33, 129.57, 128.90, 128.53, 127.46, 126.65, 122.33, 114.51, 63.32, 57.14, 55.48, 41.76, 40.40, 25.50. TLC-MS (ESI+): calcd. *m*/*z* 394.17 for C₂₃H₂₆N₂O₂S. Found 395. [M+H]⁺. HPLC *t*_{R} = 5.547 min.

*N-(3-(benzyl(methyl)amino)propyl)-5-(4-hydroxyphenyl)thiophene-2-carboxamide (12).* Prepared from *N*-(3-(benzyl(methyl)amino)propyl)-5-bromothiophene-2-carboxamide (2, 0.21 mmol) and (4-hydroxyphenyl)boronic acid (0.23 mmol, 1.1 eq.) according to general procedure 2. Purification by flash chromatography with DCM/MeOH (5-8%). Yield: 21 mg (24%), brown solid. ¹H NMR (400 MHz, CDCl₃) δ 8.14 (s, 1H), 7.37 (d, *J* = 10.8 Hz, 2H), 7.28 - 7.17 (m, *J* = 7.6, 3.5 Hz, 6H), 6.99 (d, *J* = 3.8 Hz, 1H), 6.87 (d, *J* = 8.4 Hz, 2H), 6.36 (s, 1H), 3.56 (s, 2H), 3.51 (q, *J* = 4.5 Hz, 2H), 2.61 (t, *J* = 5.8 Hz, 2H), 2.27 (s, 3H), 1.80 (quint, 2H). ¹³C NMR (101 MHz, CDCl₃) δ 162.56, 157.58, 149.40, 137.31, 136.10, 129.83, 129.49, 128.65, 127.78, 127.63, 125.63, 122.15, 116.30, 63.03, 56.80, 41.50, 40.39, 25.22. TLC-MS (ESI+): calcd. *m*/*z* 380.16 for C₂₂H₂₄N₂O₂S. Found 381.2 [M+H]⁺. HPLC *t*_{R} = 4.041 min.

*N-(3-(benzyl(methyl)amino)propyl)-5-(pyridin-4-yl)thiophene-2-carboxamide (13).* Prepared from *N*-(3-(benzyl(methyl)amino)propyl)-5-bromothiophene-2-carboxamide (2, 0.27 mmol) and pyridin-4-ylboronic acid hydrate (0.3 mmol, 1.1 eq.) according to general procedure 2. Purification by flash chromatography with DCM/MeOH (5-8%). Yield: 36 mg (33%), yellow oil. ¹H NMR (400 MHz, CDCl₃) δ 8.58 (d, *J* = 5.6 Hz, 2H), 8.35 (s, 1H), 7.40 (d, *J* = 4.7, 1.5 Hz, 2H), 7.30 (d, *J* = 4.5 Hz, 1H), 7.28 - 7.23 (m, 5H), 7.21 (d, *J* = 3.9 Hz, 1H), 3.57 - 3.49 (m, 4H), 2.61 (t, 2H), 2.25 (s, 3H), 1.78 (quint, 2H). ¹³C NMR (101 MHz, CDCl₃) δ 161.25, 150.62, 144.61, 140.95, 140.83, 138.18, 129.67, 128.71, 128.58, 127.59, 125.52, 120.06, 63.41, 57.56, 41.66, 40.82, 25.11. TLC-MS (ESI+): calcd. *m*/*z* 365.16 for C₂₁H₂₃N₃OS. Found 366.2 [M+H]⁺. HPLC *t*_{R} = 1.643 min.

*N-(3-(benzyl(methyl)amino)propyl)-5-(4-fluorophenyl)thiophene-2-carboxamide (14).* Prepared from *N*-(3-(benzyl(methyl)amino)propyl)-5-bromothiophene-2-carboxamide (2, 0.26 mmol) and 2-(4-fluorophenyl)-4,4,5,5-tetramethyl-1,3,2-dioxaboro-lane (0.29 mmol, 1.1 eq.) according to general procedure 2. Purification by flash chromatography with DCM/MeOH (2-6%). Yield: 20 mg (18%), brown semi-solid. ¹H NMR (400 MHz, CDCl₃) δ 8.16 (s, 1H), 7.59 - 7.49 (m, *J* = 8.1, 5.1, 2.5 Hz, 2H), 7.33 - 7.27 (m, *J* = 8.1, 3.9 Hz, 5H), 7.25 - 7.22 (m, *J* = 3.7 Hz, 1H), 7.14 - 7.02 (m, 3H), 3.59 - 3.51 (m, 4H), 2.67 - 2.58 (m, 2H), 2.29 (s, 3H), 1.86 - 1.76 (m, 2H). ¹³C NMR (101 MHz, CDCl₃) δ 164.14, 161.75, 147.19, 138.49, 138.08, 130.15, 130.12, 129.69, 128.83, 128.59, 127.96, 127.88, 127.59, 123.38, 116.23, 116.02, 63.33, 57.27, 41.65, 40.54, 25.29. TLC-MS (ESI+): calcd. *m*/*z* 382.15 for C₂₂H₂₃FN₂OS. Found 383.2 [M+H]⁺. HPLC *t*_{R} = 5.957 min.

*N-(3-(benzyl(methyl)amino)propyl)-5-(2-fluorophenyl)thiophene-2-carboxamide (15).* Prepared from *N*-(3-(benzyl(methyl)amino)propyl)-5-bromothiophene-2-carboxamide (2, 0.22 mmol) and (2-fluorophenyl)boronic acid (0.24 mmol, 1.1 eq.) according to general procedure 2. Purification by flash chromatography with DCM/MeOH (2-6%). Yield: 20 mg (22%), brown semi-solid. ¹H NMR (400 MHz, CDCl₃) δ 8.14 (s, 1H), 7.54 (t, 1H), 7.33 - 7.29 (m, *J* = 3.0 Hz, 1H), 7.29 - 7.20 (m, 7H), 7.17 - 7.06 (m, *J* = 16.5, 8.6 Hz, 2H), 3.59 - 3.44 (m, 4H), 2.58 (t, 2H), 2.26 (s, 3H), 1.77 (quint, 2H). ¹³C NMR (101 MHz, CDCl₃) δ 161.79, 160.58, 158.09, 141.14, 141.11, 139.17, 139.14, 137.96, 129.70, 129.66, 129.61, 128.98, 128.95, 128.59, 128.47, 127.60, 126.78, 126.71, 124.71, 124.67, 121.82, 121.69, 116.68, 116.46, 63.24, 56.98, 41.69, 40.45, 25.28. TLC-MS (ESI+): calcd. *m*/*z* 382.15 for C₂₂H₂₃FN₂OS. Found 383.2 [M+H]⁺. HPLC *t*_{R} = 6.044 min.

*N-(3-(benzyl(methyl)amino)propyl)-5-(3-fluorophenyl)thiophene-2-carboxamide (16).* Prepared from *N*-(3-(benzyl(methyl)amino)propyl)-5-bromothiophene-2-carboxamide (2, 0.22 mmol) and (3-fluorophenyl)boronic acid (0.24 mmol, 1.1 eq.) according to general procedure 2. Purification by flash chromatography with DCM/MeOH (4-8%). Yield: 63 mg (72%), brown semi-solid. ¹H NMR (400 MHz, CDCl₃) δ 8.13 (s, 1H), 7.35 - 7.22 (m, 8H), 7.21 (d, *J* = 3.9 Hz, 1H), 7.13 (d, *J* = 3.9 Hz, 1H), 7.02 - 6.93 (m, 1H), 3.56 - 3.46 (m, 4H), 2.58 (t, 2H), 2.24 (s, 3H), 1.76 (quint, 2H). ¹³C NMR (101 MHz, CDCl₃) δ 163.14, 161.57, 146.66, 139.17, 138.34, 135.95, 130.97, 130.71, 129.60, 129.60, 128.77, 128.55, 128.55, 127.54, 124.06, 121.85, 115.07, 113.09, 63.40, 57.39, 41.73, 40.63, 25.36. TLC-MS (ESI+): calcd. *m*/*z* 382.15 for C₂₂H₂₃FN₂OS. Found 383.2 [M+H]⁺. HPLC *t*_{R} = 5.940 min.

*N-(3-(benzyl(methyl)amino)propyl)-5-(3,5-difluorophenyl)thiophene-2-carboxamide (17)*. Prepared from *N*-(3-(benzyl(methyl)amino)propyl)-5-bromothiophene-2-carboxamide (2, 0.19 mmol) and (3,5-difluorophenyl)boronic acid (0.21 mmol, 1.1 eq.) according to general procedure 2. Purification by flash chromatography with DCM/MeOH (2-7%). Yield: 26 mg (31%), brown semi-solid. ¹H NMR (400 MHz, CDCl₃) δ 8.31 (s, 1H), 7.33 - 7.26 (m, 5H), 7.18 (dd, *J* = 18.3, 3.9 Hz, 2H), 7.10 - 7.01 (m, 2H), 6.82 - 6.72 (m, 1H), 3.59 - 3.50 (m, 4H), 2.66 - 2.61 (m, 2H), 2.28 (s, 3H), 1.80 (quint, *J* = 11.5, 5.8 Hz, 2H). ¹³C NMR (101 MHz, CDCl₃) δ 163.36, 161.39, 145.39, 139.79, 138.28, 136.81, 129.71, 128.82, 128.60, 127.65, 124.74, 109.05, 103.57, 63.49, 57.71, 41.66, 40.92, 25.12. TLC-MS (ESI+): calcd. *m*/*z* 400.14 for C₂₂H₂₂F₂N₂OS. Found 401.2 [M+H]⁺. HPLC *t*_{R} = 6.630 min.

*N-(3-((4-methoxybenzyl)(methyl)amino)propyl)-5-phenylthiophene-2-carboxamide (18).* Prepared from 5-phenylthiophene-2-carboxylic acid (0.29 mmol) and *N*¹-(4-methoxybenzyl)-*N*¹-methylpropane-1,3-diamine dihydrochloride (18a, 0.29 mmol, 1 eq.) according to general procedure 1. Purification by flash chromatography with DCM/MeOH (1-4%). Yield: 33 mg (28%), brown semi-solid. ¹H NMR (400 MHz, CDCl₃) δ 8.20 (s, 1H), 7.61 - 7.55 (m, *J* = 7.1, 4.8, 2.6 Hz, 2H), 7.42 - 7.36 (m, 2H), 7.35 - 7.28 (m, *J* = 11.8, 6.0, 2.6 Hz, 2H), 7.25 - 7.21 (m, *J* = 8.6 Hz, 2H), 7.19 (d, *J* = 3.9 Hz, 1H), 6.87 - 6.79 (m, 2H), 3.74 (s, 3H), 3.59 - 3.49 (m, 4H), 2.64 (t, 2H), 2.31 (s, 3H), 1.83 (quint, *J* = 11.4, 5.8 Hz, 2H). ¹³C NMR (101 MHz, CDCl₃) δ 161.93, 159.23, 148.35, 133.83, 131.25, 131.00, 129.23, 129.15, 129.05, 128.47, 126.16, 123.45, 113.97, 62.51, 55.34, 29.85, 25.11, 22.84, 14.27. TLC-MS (ESI+): calcd. *m*/*z* 394.17 for C₂₃H₂₆N₂O₂S. Found 395.2 [M+H]⁺. HPLC *t*_{R} = 6.072 min.

*N-(3-((4-bromobenzyl)(methyl)amino)propyl)-5-phenylthiophene-2-carboxamide (19).* Prepared from 5-phenylthiophene-2-carboxylic acid (0.29 mmol) and *N*¹-(4-bromobenzyl)-*N*¹-methylpropane-1,3-diamine dihydrochloride (19a, 0.29 mmol, 1 eq.) according to general procedure 1. Purification by flash chromatography with DCM/MeOH (1-4%). Yield: 63 mg (48%), yellow semi-solid. ¹H NMR (400 MHz, CDCl₃) δ 7.92 (t, *J* = 4.6 Hz, 1H), 7.59 - 7.51 (m, *J* = 5.2, 3.3 Hz, 2H), 7.43 - 7.33 (m, 4H), 7.32 - 7.25 (m, 2H), 7.20 - 7.13 (m, *J* = 5.9, 5.1 Hz, 3H), 3.57 - 3.42 (m, *J* = 11.7, 5.7 Hz, 4H), 2.58 (t, *J* = 5.9 Hz, 2H), 2.25 (s, 3H), 1.78 (quint, 2H). ¹³C NMR (101 MHz, CDCl₃) δ 161.99, 148.51, 137.87, 136.94, 133.60, 131.65, 131.23, 129.15, 129.08, 128.49, 126.09, 123.48, 121.48, 62.39, 56.84, 41.48, 40.05, 25.42. TLC-MS (ESI+): calcd. *m*/*z* 442.07 for C₂₂H₂₃BrN₂OS. Found 465.1 [M+Na]⁺. HPLC *t*_{R} = 6.856 min.

*N-(3-((4-fluorobenzyl)(methyl)amino)propyl)-5-phenylthiophene-2-carboxamide (20).* Prepared from 5-phenylthiophene-2-carboxylic acid (0.44 mmol) and *N*¹-(4-fluorobenzyl)-*N*¹-methylpropane-1,3-diamine dihydrochloride (20a, 0.44 mmol, 1 eq.) according to general procedure 1. Purification by flash chromatography with DCM/MeOH (1-6%). Yield: 90 mg (53%), brown semi-solid. ¹H NMR (400 MHz, CDCl₃) δ 8.05 (s, 1H), 7.54 (d, *J* = 9.6 Hz, 2H), 7.40 - 7.33 (m, 2H), 7.33 - 7.29 (m, 2H), 7.28 - 7.23 (m, *J* = 8.1, 5.3 Hz, 2H), 7.17 (d, *J* = 3.8 Hz, 1H), 6.96 (t, *J* = 8.6 Hz, 2H), 3.54 - 3.48 (m, 4H), 2.62 (t, *J* = 18.0, 12.1 Hz, 2H), 2.26 (s, 3H), 1.79 (quint, 2H). ¹³C NMR (101 MHz, CDCl₃) δ 162.22, 162.05, 148.54, 137.89, 133.59, 133.45, 131.19, 129.10, 129.07, 128.50, 126.07, 123.42, 115.38, 62.21, 56.77, 41.32, 40.10, 25.35. TLC-MS (ESI+): calcd. *m*/*z* 382.15 for C₂₀H₂₃FN₂OS. Found 383.2 [M+H]⁺. HPLC *t*_{R} = 6.094 min.

*N-(3-((2-fluorobenzyl)(methyl)amino)propyl)-5-phenylthiophene-2-carboxamide (21).* Prepared from 5-phenylthiophene-2-carboxylic acid (0.29 mmol) and *N*¹-(2-fluorobenzyl)-*N*¹-methylpropane-1,3-diamine dihydrochloride (21a, 0.29 mmol, 1 eq.) according to general procedure 1. Purification by flash chromatography with DCM/MeOH (1-4%). Yield: 100 mg (89%), yellow semi-solid. ¹H NMR (400 MHz, CDCl₃) δ 8.16 (t, *J* = 4.7 Hz, 1H), 7.57 - 7.51 (m, 2H), 7.37 - 7.22 (m, 6H), 7.15 (d, *J* = 3.9 Hz, 1H), 7.10 - 7.04 (m, 1H), 7.04 - 6.97 (m, *J* = 9.5, 8.2, 1.0 Hz, 1H), 3.66 (s, 2H), 3.52 (q, 2H), 2.68 (t, *J* = 13.8, 7.9 Hz, 2H), 2.31 (s, 3H), 1.81 (quint, 2H). ¹³C NMR (101 MHz, CDCl₃) δ 162.88, 162.11, 160.42, 148.41, 138.14, 133.73, 132.24, 132.20, 129.82, 129.73, 129.07, 128.93, 128.42, 126.09, 124.24, 124.20, 123.43, 115.80, 115.58, 57.02, 55.69, 53.55, 41.24, 40.17, 25.23. TLC-MS (ESI+): calcd. *m*/*z* 382.15 for C₂₂H₂₃FN₂OS. Found 383.2 [M+H]⁺. HPLC *t*_{R} = 5.901 min.

*N-(3-((3-fluorobenzyl)(methyl)amino)propyl)-5-phenylthiophene-2-carboxamide (22).* Prepared from 5-phenylthiophene-2-carboxylic acid (0.29 mmol) and *N*¹-(3-fluorobenzyl)-*N*¹-methylpropane-1,3-diamine dihydrochloride (22a, 0.29 mmol, 1 eq.) according to general procedure 1. Purification by flash chromatography with DCM/MeOH (1-4%). Yield: 33 mg (29%), yellow semi-solid. ¹H NMR (400 MHz, CDCl₃) δ 7.88 (t, *J* = 4.4 Hz, 1H), 7.59 - 7.52 (m, 2H), 7.41 - 7.34 (m, 2H), 7.33 - 7.28 (m, 2H), 7.25 - 7.16 (m, 2H), 7.11 - 7.00 (m, 2H), 6.98 - 6.90 (m, 1H), 3.60 - 3.49 (m, 4H), 2.60 (t, 2H), 2.27 (s, 3H), 1.80 (quint, 2H). ¹³C NMR (101 MHz, CDCl₃) δ 164.19, 161.97, 161.74, 148.49, 140.71, 138.04, 133.72, 130.13, 130.05, 129.11, 128.96, 128.49, 126.15, 125.11, 125.08, 123.48, 116.40, 116.19, 114.59, 114.38, 62.69, 57.05, 41.64, 40.17, 25.52. TLC-MS (ESI+): calcd. *m*/*z* 382.15 for C₂₂H₂₃FN₂OS. Found 383.2 [M+H]⁺. HPLC *t*_{R} = 6.155 min.

*N-(3-(benzyl(ethyl)amino)propyl)-5-phenylthiophene-2-carboxamide (23).* Prepared from 5-phenylthiophene-2-carboxylic acid (0.44 mmol) and *N*¹-benzyl-*N*¹-ethylpropane-1,3-diamine dihydrochloride (23a, 0.44 mmol, 1 eq.) according to general procedure 1. Purification by flash chromatography with DCM/MeOH (1-6%). Yield: 70 mg (42%), brown semi-solid. ¹H NMR (400 MHz, CDCl₃) δ 7.94 (s, 1H), 7.61 - 7.52 (m, 2H), 7.39 - 7.33 (m, *J* = 10.1, 4.7 Hz, 2H), 7.32 - 7.26 (m, *J* = 8.3, 5.6, 3.3 Hz, 4H), 7.26 - 7.21 (m, 3H), 7.16 (d, *J* = 3.9 Hz, 1H), 3.58 (s, 2H), 3.47 (q, *J* = 7.8, 3.9 Hz, 2H), 2.66 - 2.52 (m, 4H), 1.75 (quint, 2H), 1.08 (t, *J* = 7.1 Hz, 3H). ¹³C NMR (101 MHz, CDCl₃) δ 161.90, 148.32, 138.52, 138.28, 133.76, 129.53, 129.08, 128.80, 128.51, 128.41, 127.38, 126.09, 123.33, 58.43, 52.45, 47.05, 40.34, 25.23, 11.11. TLC-MS (ESI+): calcd. *m*/*z* 378.18 for C₂₃H₂₆N₂OS. Found 379.2 [M+H]⁺. HPLC *t*_{R} = 6.043 min.

*N-(3-(benzyl(isopropyl)amino)propyl)-5-phenylthiophene-2-carboxamide (24)*. Prepared from 5-phenylthiophene-2-carboxylic acid (0.42 mmol) and *N*¹-benzyl-*N*¹-isopropylpropane-1,3-diamine dihydrochloride (24a, 0.42 mmol, 1 eq.) according to general procedure 1. Purification by flash chromatography with DCM/MeOH (0-3%). Yield: 30 mg (18%), yellow semi-solid. ¹H NMR (400 MHz, CDCl₃) δ 7.65 - 7.56 (m, 2H), 7.45 - 7.32 (m, 6H), 7.31 - 7.22 (m, *J* = 10.2, 6.9, 2.7 Hz, 3H), 7.19 - 7.12 (m, *J* = 15.7, 3.8 Hz, 2H), 3.56 (s, 2H), 3.51 - 3.44 (m, *J* = 11.6, 5.4 Hz, 2H), 3.15-3.01 (m, 1H), 2.60 (t, 2H), 1.72 (quint, *J* = 11.6, 5.9 Hz, 2H), 1.07 (d, *J* = 6.6 Hz, 6H). ¹³C NMR (101 MHz, CDCl₃) δ 161.85, 148.36, 140.19, 138.31, 133.83, 129.39, 129.12, 128.74, 128.54, 128.44, 127.22, 126.15, 123.28, 54.26, 49.00, 47.99, 39.94, 26.08, 17.52. TLC-MS (ESI+): calcd. *m*/*z* 392.19 for C₂₄H₂₈N₂OS. Found 393.2 [M+H]⁺. HPLC *t*_{R} = 5.511 min.

*N-(3-(benzyl(cyclopropyl)amino)propyl)-5-phenylthiophene-2-carboxamide (25).* Prepared from 5-phenylthiophene-2-carboxylic acid (0.54 mmol) and *N*¹-benzyl-*N*¹-cyclopropylpropane-1,3-diamine (25a, 0.54 mmol, 1 eq.) according to general procedure 1. Purification by flash chromatography with PE/EtOAc (10-40%). Yield: 55 mg (26%), yellow semi-solid. ¹H NMR (400 MHz, CDCl₃) δ 7.65 - 7.55 (m, *J* = 5.2, 3.4 Hz, 2H), 7.42 - 7.35 (m, 2H), 7.34 - 7.29 (m, 1H), 7.28 - 7.19 (m, 8H), 3.76 (s, 2H), 3.44 (q, *J* = 11.6, 5.7 Hz, 2H), 2.71 (t, 2H), 1.86 - 1.72 (m, 3H), 0.57 - 0.51 (m, 2H), 0.50 - 0.44 (m, 2H). ¹³C NMR (101 MHz, CDCl₃) δ 161.76, 148.35, 138.11, 137.85, 133.79, 129.88, 129.16, 128.85, 128.50, 128.33, 127.34, 126.17, 123.40, 60.04, 54.37, 40.31, 37.65, 25.37, 7.37. TLC-MS (ESI+): calcd. *m*/*z* 390.18 for C₂₄H₂₆N₂OS. Found 391.2 [M+H]⁺. HPLC *t*_{R} = 6.192 min.

*N-(3-(benzyl(propyl)amino)propyl)-5-phenylthiophene-2-carboxamide* (26). Prepared from 5-phenylthiophene-2-carboxylic acid (0.36 mmol) and *N*¹-benzyl-*N*¹-propylpropane-1,3-diamine dihydrochloride (26a, 0.36 mmol, 1 eq.) according to general procedure 1. Purification by flash chromatography with DCM/MeOH (1-4%). Yield: 36 mg (26%), colorless oil. ¹H NMR (400 MHz, CDCl₃) δ 7.66 - 7.56 (m, 3H), 7.46 - 7.30 (m, 8H), 7.22 (d, *J* = 3.9 Hz, 1H), 3.88 (s, 2H), 3.51 (q, 2H), 2.87 (s, 2H), 2.80 (s, 1H), 2.70 (t, 2H), 1.95 (quint, 2H), 1.71 - 1.56 (m, 2H), 0.90 (t, *J* = 7.3 Hz, 3H). ¹³C NMR (101 MHz, CDCl₃) δ 163.47, 149.50, 136.79, 133.57, 129.99, 129.73, 129.18, 129.07, 128.68, 126.18, 123.86, 58.37, 54.95, 51.91, 38.72, 25.20, 18.62, 11.56. TLC-MS (ESI+): calcd. *m*/*z* 392.19 for C₂₄H₂₈N₂OS. Found 393.2 [M+H]⁺. HPLC *t*_{R} = 6.351 min.

*N-(3-(benzyl(butyl)amino)propyl)-5-phenylthiophene-2-carboxamide (27).* Prepared from 5-phenylthiophene-2-carboxylic acid (0.46 mmol) and *N*¹-benzyl-*N*¹-butylpropane-1,3-diamine dihydrochloride (27a, 0.46 mmol, 1 eq.) according to general procedure 1. Purification by flash chromatography with DCM/MeOH (0-3%). Yield: 13 mg (7%), yellow solid. ¹H NMR (400 MHz, CDCl₃) δ 7.66 - 7.53 (m, J= 5.3, 3.3 Hz, 3H), 7.43 - 7.37 (m, 2H), 7.36 - 7.22 (m, 7H), 7.20 (d, *J* = 3.8 Hz, 1H), 3.60 (s, 2H), 3.50 (q, *J* = 11.5, 5.5 Hz, 2H), 2.60 (t, 2H), 2.51 (t, 2H), 1.77 (quint, *J* = 11.4, 5.8 Hz, 2H), 1.60 - 1.48 (m, 2H), 1.35 - 1.26 (m, *J* = 15.0, 7.4 Hz, 2H), 0.88 (t, *J* = 7.3 Hz, 3H). ¹³C NMR (101 MHz, CDCl₃) δ 161.91, 148.39, 138.33, 133.86, 129.57, 129.15, 128.81, 128.57, 128.47, 127.43, 126.18, 123.35, 59.08, 53.64, 52.94, 40.09, 28.73, 25.55, 20.87, 14.18. TLC-MS (ESI+): calcd. *m*/*z* 406.21 for C₂₅H₃₀N₂OS. Found 407.2 [M+H]⁺. HPLC *t*_{R} = 6.820 min.

*N-(3-(benzyl(3-hydroxypropyl)amino)propyl)-5-phenylthiophene-2-carboxamide (28).* Prepared from 5-phenylthiophene-2-carboxylic acid (1.2 mmol) and 3-((3-aminopropyl)(benzyl)amino)propan-1-ol dihydrochloride (28a, 1.2 mmol, 1 eq.) according to general procedure 1. Purification by flash chromatography with DCM/MeOH (0-4%). Yield: 25 mg (5%), colorless oil. ¹H NMR (400 MHz, CDCl₃) δ 7.62 - 7.55 (m, 2H), 7.43 (d, *J* = 3.9 Hz, 1H), 7.41 - 7.25 (m, 8H), 7.22 (d, *J* = 3.9 Hz, 1H), 7.10 (t, *J* = 6.0 Hz, 1H), 4.24 (s, 1H), 3.79 - 3.71 (m, 4H), 3.44 (q, *J* = 6.3 Hz, 2H), 2.82 (t, *J* = 6.1 Hz, 2H), 2.71 (t, *J* = 6.6 Hz, 2H), 1.91 (quint, *J* = 6.5 Hz, 2H), 1.80 (quint, 2H). ¹³C NMR (101 MHz, CDCl₃) δ 162.68, 149.13, 137.49, 135.78, 133.66, 129.74, 129.29, 129.18, 128.95, 128.62, 128.29, 126.18, 123.72, 63.18, 58.69, 53.57, 50.97, 37.63, 27.34, 25.88. TLC-MS (ESI+): calcd. *m*/*z* 408.19 for C₂₄H₂₈N₂O₂S. Found 431.3 [M+Na]⁺. HPLC *t*_{R} = 5.528 min.

*N-(3-(benzyl(phenethyl)amino)propyl)-5-phenylthiophene-2-carboxamide (29).* Prepared from 5-phenylthiophene-2-carboxylic acid (0.45 mmol) and *N*¹-benzyl-*N*¹-phenethylpropane-1,3-diamine dihydrochloride (29a, 0.45 mmol, 1 eq.) according to general procedure 1. Purification by flash chromatography with PE/EtOAc (0-60%). Yield: 24 mg (12%), white solid. ¹H NMR (400 MHz, CDCl₃) δ 7.63 - 7.50 (m, 2H), 7.38 - 7.31 (m, 2H), 7.30 - 7.16 (m, 9H), 7.16 - 7.04 (m, 5H), 3.63 (s, 2H), 3.39 (q, *J* = 5.7 Hz, 2H), 2.86 - 2.70 (m, 4H), 2.61 (t, *J* = 5.9 Hz, 2H), 1.71 (quint, *J* = 6.0 Hz, 2H). ¹³C NMR (101 MHz, CDCl₃) δ 161.86, 148.42, 140.30, 138.92, 138.20, 133.81, 129.44, 129.13, 128.82, 128.79, 128.56, 128.47, 127.42, 126.23, 126.17, 123.35, 59.01, 55.52, 52.59, 39.80, 33.04, 25.87. TLC-MS (ESI+): calcd. m/z 454.21 for C₂₉H₃₀N₂OS. Found 477.1 [M+Na]⁺. HPLC *t*_{R} = 7.138 min.

*N-(3-(benzyl(3-phenylpropyl)amino)propyl)-5-phenylthiophene-2-carboxamide (30).* Prepared from 5-phenylthiophene-2-carboxylic acid (0.42 mmol) and *N*¹-benzyl-*N*¹ -(3-phenylpropyl)propane-1,3-diamine dihydrochloride (30a, 0.42 mmol, 1 eq.) according to general procedure 1. Purification by flash chromatography with DCM/MeOH (0-6%). Yield: 19 mg (10%), colorless oil. ¹H NMR (400 MHz, CDCl₃) δ 7.62 (d, 2H), 7.45 - 7.13 (m, 15H), 3.64 (s, 2H), 3.58 - 3.40 (m, 3H), 2.79 - 2.48 (m, 6H), 1.91 (quint, *J* = 9.9, 7.5 Hz, 2H), 1.79 (quint, 2H). ¹³C NMR (101 MHz, CDCl₃) δ 162.02, 148.49, 141.92, 138.58, 138.01, 133.77, 129.51, 129.14, 129.00, 128.59, 128.50, 128.41, 127.46, 126.17, 125.98, 123.41, 59.00, 53.30, 52.80, 39.84, 33.78, 28.33, 25.70. TLC-MS (ESI+): calcd. *m*/*z* 468.22 for C₃₀H₃₂N₂OS. Found 491.2 [M+Na]⁺. HPLC *t*_{R} = 7.457 min.

*N-(3-(benzyl(ethyl)amino)propyl)-5-(3-fluorophenyl)thiophene-2-carboxamide (31).* Prepared from N-(3-(benzyl(ethyl)amino)propyl)-5-bromothiophene-2-carboxamide (31a, 0.29 mmol) and (3-fluorophenyl)boronic acid (0.32 mmol, 1.1 eq.) according to general procedure 2. Purification by flash chromatography with DCM/MeOH (1-4%). Yield: 29 mg (25%), brown semi-solid. ¹H NMR (400 MHz, CDCl₃) δ 7.98 (s, 1H), 7.39 - 7.25 (m, 8H), 7.24 - 7.21 (m, 1H), 7.18 (d, *J* = 3.9 Hz, 1H), 7.06 - 6.98 (m, 1H), 3.60 (s, 2H), 3.51 (q, *J* = 11.2, 4.1 Hz, 2H), 2.69 -2.57 (m, 4H), 1.78 (quint, *J* = 11.6, 5.8 Hz, 2H), 1.11 (t, *J* = 7.1 Hz, 3H). ¹³C NMR (101 MHz, CDCl₃) δ 164.45, 162.00, 161.65, 146.73, 146.70, 139.15, 135.97, 135.89, 130.75, 130.67, 129.62, 128.72, 128.59, 127.48, 124.05, 121.90, 121.87, 115.32, 115.11, 113.11, 112.89, 58.59, 52.72, 47.12, 40.58, 25.20, 11.15. TLC-MS (ESI+): calcd. *m*/*z* 396.17 for C₂₃H₂₅FN₂OS. Found 397.2 [M+H]⁺. HPLC *t*_{R} = 6.288 min.

*N-(3-(ethyl(4-fluorobenzyl)amino)propyl)-5-(3-fluorophenyl)thiophene-2-carboxamide (32).* Prepared from 5-bromo-N-(3-(ethyl(4-fluorobenzyl)amino)propyl)thiophene-2-carboxamide (32a, 0.33 mmol) and (3-fluorophenyl)boronic acid (0.36 mmol, 1.1 eq.) according to general procedure 2. Purification by flash chromatography with DCM/MeOH (1-4%). Yield: 53 mg (40%), brown semi-solid. ¹H NMR (400 MHz, CDCl₃) δ 7.83 (s, 1H), 7.40 - 7.33 (m, 2H), 7.30 - 7.23 (m, 4H), 7.20 (d, *J* = 3.9 Hz, 1H), 7.06 - 6.99 (m, *J* = 8.1, 5.7, 3.5 Hz, 1H), 6.99 - 6.92 (m, 2H), 3.56 (s, 2H), 3.51 (q, *J* = 11.5, 5.4 Hz, 2H), 2.67 -2.50 (m, 4H), 1.78 (quint, *J* = 11.7, 5.9 Hz, 2H), 1.10 (t, *J* = 7.1 Hz, 3H). ¹³C NMR (101 MHz, CDCl₃) δ 164.45, 163.42, 162.00, 161.63, 160.98, 146.82, 146.79, 138.96, 135.88, 135.80, 131.11, 131.03, 130.79, 130.71, 129.87, 128.75, 124.07, 121.91, 121.89, 115.50, 115.40, 115.29, 115.19, 113.11, 112.89, 57.74, 52.46, 47.02, 40.44, 25.29, 11.16. TLC-MS (ESI+): calcd. *m*/*z* 414.16 for C₂₃H₂₄F₂N₂OS. Found 415.2 [M+H]⁺. HPLC *t*_{R} = 6.443 min.

*tert-butyl (3-(5-phenylthiophene-2-carboxamido)propyl)carbamate (33).* Prepared from 5-phenylthiophene-2-carboxylic acid (2.01 mmol) and tert-butyl (3-aminopropyl)carbamate (2.01 mmol, 1 eq.) according to general procedure 1. Purification by suction filtration and wash with water. Yield: 637 mg (88%), brown solid. ¹H NMR (400 MHz, DMSO) δ 8.49 (t, 1H), 7.76 - 7.65 (m, *J* = 7.9, 5.9 Hz, 3H), 7.53 (d, *J* = 3.9 Hz, 1H), 7.44 (t, *J* = 7.6 Hz, 2H), 7.39 - 7.33 (m, *J* = 7.3 Hz, 1H), 6.80 (t, *J* = 5.0 Hz, 1H), 3.24 (q, *J* = 13.0, 6.7 Hz, 2H), 2.98 (q, *J* = 12.9, 6.6 Hz, 2H), 1.63 (quint, *J* = 6.9 Hz, 2H), 1.38 (s, 9H). ¹³C NMR (101 MHz, DMSO) δ 160.87, 155.58, 147.15, 139.02, 133.13, 129.20, 128.84, 128.41, 125.59, 124.19, 77.48, 39.52, 37.72, 36.88, 29.64, 28.23. TLC-MS (ESI+): calcd. *m*/*z* 360.15 for C₁₉H₂₄N₂O₃S. Found 382.8 [M+Na]⁺. HPLC *t*_{R} = 8.697 min.

*N-(3-(benzylamino)propyl)-5-phenylthiophene-2-carboxamide* (34). Prepared from benzaldehyde (0.51 mmol), *N*-(3-aminopropyl)-5-phenylthiophene-2-carboxamide hydrochloride (34a, 0.51 mmol, 1 eq.), and triethylamine according to general procedure 3. Purification by flash chromatography with DCM/MeOH (1-10%). Yield: 53 mg (30%), pale yellow solid. ¹H NMR (400 MHz, DMSO) δ 8.78 (t, *J* = 5.8 Hz, 1H), 8.21 (s, 1H), 7.79 (d, *J* = 3.9 Hz, 1H), 7.75 - 7.65 (m, 2H), 7.56 - 7.30 (m, 8H), 4.02 (s, 2H), 3.32 (q, *J* = 6.5 Hz, 2H), 2.85 (t, *J* = 7.4 Hz, 2H), 1.87 (quint, *J* = 6.8 Hz, 2H). ¹³C NMR (101 MHz, DMSO) δ 161.08, 147.28, 138.82, 134.55, 133.10, 129.41, 129.22, 129.15, 128.47, 128.44, 128.17, 125.61, 124.23, 50.80, 44.99, 36.61, 26.83. TLC-MS (ESI+): calcd. m/z 350.15 for C₂₁H₂₂N₂OS. Found 372.9 [M+Na]⁺. HPLC *t*_{R} = 5.935 min.

*N-(3-(3,4-dihydroisoquinolin-2(1H)-yl)propyl)-5-phenylthiophene-2-carboxamide* (35). Prepared from 5-phenylthiophene-2-carboxylic acid (0.53 mmol) and 3-(3,4-dihydroisoquinolin-2(1*H*)-yl)propan-1-amine dihydrochloride (35a, 0.53 mmol, 1 eq.) according to general procedure 1. Purification by flash chromatography with DCM/MeOH/1% NH₃ (1.5-2%). Yield: 17 mg (9%), yellow semi-solid. ¹H NMR (400 MHz, CDCl₃) δ 8.74 (s, 1H), 7.40 - 7.27 (m, 5H), 7.25 - 7.12 (m, 3H), 7.09 - 7.04 (m, *J* = 7.3 Hz, 1H), 7.02 (d, *J* = 3.9 Hz, 1H), 6.91 (d, *J* = 3.9 Hz, 1H), 3.75 (s, 2H), 3.62 (dd, *J* = 11.0, 5.1 Hz, 2H), 3.00 (t, *J* = 5.9 Hz, 2H), 2.85 (t, *J* = 6.0 Hz, 2H), 2.79 (t, 2H), 1.89 (quint, 2H). ¹³C NMR (101 MHz, CDCl₃) δ 161.83, 147.98, 138.07, 134.31, 134.18, 133.70, 129.15, 128.98, 128.82, 128.27, 126.92, 126.69, 126.11, 126.07, 123.25, 58.85, 56.23, 51.52, 41.42, 29.38, 24.24. TLC-MS (ESI+): calcd. *m*/*z* 376.16 for C₂₃H₂₄N₂OS. Found 399.3 [M+Na]⁺. HPLC *t*_{R} = 5.191 min.

*N-(3-((3-(4-fluorobenzamido)benzyl)(methyl)amino)propyl)-5-phenylthiophene-2-carboxamide (36).* Prepared from 5-phenylthiophene-2-carboxylic acid (0.34 mmol) and *N*-(3-(((3-aminopropyl)(methyl)amino)methyl)phenyl)-4-fluorobenzamide (36a, 0.34 mmol, 1 eq.) according to general procedure 1. Purification by flash chromatography with DCM/MeOH (5-8%). Yield: 96 mg (56%), pale yellow solid. ¹H NMR (400 MHz, DMSO) δ 10.35 (s, 1H), 8.63 (s, 1H), 8.09 - 8.00 (m, 2H), 7.92 (s, 1H), 7.75 - 7.62 (m, 4H), 7.52 (d, *J* = 3.9 Hz, 1H), 7.44 (dd, *J* = 10.3, 4.7 Hz, 2H), 7.37 (dd, *J* = 12.3, 5.2 Hz, 4H), 7.18 (s, 1H), 4.02 (s, 1H), 3.55 - 3.22 (m, 3H), 2.33 (s, 2H), 1.98 - 1.72 (m, 2H), 1.29 - 0.63 (m, 3H). ¹³C NMR (101 MHz, DMSO) δ 165.35, 164.48, 162.87, 161.10, 147.30, 139.26, 138.83, 133.11, 131.24, 130.45, 130.36, 129.24, 129.06, 128.80, 128.49, 125.62, 124.22, 115.46, 115.24, 54.91, 45.66, 36.96, 21.06, 8.60. TLC-MS (ESI+): calcd. *m*/*z* 501.19 for C₂₉H₂₈FN₃O₂S. Found 502.2 [M+H]⁺. HPLC *t*_{R} = 6.709 min.

*N-(tert-butyl)-1-(3-(5-phenylthiophene-2-carboxamido)propyl)piperidine-4-carboxamide (37).* Prepared from 5-phenylthiophene-2-carboxylic acid (0.48 mmol) and 1-(3-aminopropyl)-*N*-(*tert*-butyl)piperidine-4-carboxamide dihydrochloride (37a, 0.48 mmol, 1 eq.) according to general procedure 1. Purification by flash chromatography with DCM/MeOH (10-20%). Yield: 940 mg (46%), white solid. ¹H NMR (400 MHz, CDCl₃) δ 8.14 (t, 1H), 7.70 - 7.66 (m, 2H), 7.56 (d, *J* = 3.9 Hz, 1H), 7.43 - 7.37 (m, 2H), 7.33 - 7.30 (m, *J* = 3.3, 1.2 Hz, 1H), 7.30 (d, *J* = 3.9 Hz, 1H), 5.25 (s, 1H), 3.55 (q, *J* = 11.2, 5.4 Hz, 2H), 3.08 (d, *J* = 11.1 Hz, 2H), 2.53 (t, 2H), 2.04 - 1.82 (m, 7H), 1.77 (dt, *J* = 11.5, 5.9 Hz, 2H), 1.33 (s, 9H). ¹³C NMR (101 MHz, CDCl₃) δ 174.1, 162.0, 148.4, 137.9, 133.8, 129.5, 129.2, 128.4, 126.3, 123.6, 58.9, 53.7, 51.1, 41.0, 29.2, 29.0, 24.5. TLC-MS (ESI+): calcd. *m*/*z* 427.23 for C₂₄H₃₃N₃O₂S. Found 428.4 [M+H]⁺. HPLC *t*_{R} = 5.982 min.

*tert-butyl 3-((5-phenylthiophene-2-carboxamido)methyl)piperidine-1-carboxylate (38).* Prepared from 5-phenylthiophene-2-carboxylic acid (1.47 mmol) and *tert-*butyl 3-(aminomethyl)piperidine-1-carboxylate (1.47 mmol, 1 eq.) according to general procedure 1. Purification by flash chromatography with DCM/MeOH (0-1%). Yield: 588 mg (quant.), yellow oil. ¹H NMR (400 MHz, CDCl₃) δ 7.63 - 7.55 (m, 2H), 7.52 (d, *J* = 3.9 Hz, 1H), 7.41 - 7.33 (m, *J* = 7.4 Hz, 2H), 7.33 - 7.27 (m, *J* = 7.3 Hz, 1H), 7.22 (d, *J* = 3.9 Hz, 1H), 3.93 - 3.40 (m, *J* = 46.9 Hz, 3H), 3.35 - 2.98 (m, *J* = 39.8, 28.9 Hz, 2H), 2.97 - 2.74 (m, *J* = 47.8, 19.7 Hz, 2H), 1.95 - 1.76 (m, 2H), 1.70 - 1.56 (m, 1H), 1.44 (s, 9H), 1.41 - 1.18 (m, *J* = 21.7, 16.4, 7.7 Hz, 2H). ¹³C NMR (101 MHz, CDCl₃) δ 162.15, 155.28, 148.79, 137.94, 133.67, 129.07, 129.07, 128.87, 128.45, 128.45, 126.12, 123.51, 79.71, 47.06, 45.14, 41.58, 35.62, 28.51, 28.51, 28.51, 28.19, 23.66. TLC-MS (ESI+): calcd. *m*/*z* 400.18 for C₂₂H₂₈N₂O₃S. Found 423.2 [M+Na]⁺. HPLC *t*_{R} = 9.442 min.

*N-((1-benzylpiperidin-3-yl)methyl)-5-phenylthiophene-2-carboxamide (39).* Prepared from 5-phenylthiophene-2-carboxylic acid (0.5 mmol) and (1-benzylpiperidin-3-yl)methanamine hydrochloride (39a, 0.5 mmol, 1 eq.) according to general procedure 1. Purification by flash chromatography with DCM/MeOH (1-6%). Yield: 26 mg (14%), yellow oil. ¹H NMR (400 MHz, CDCl₃) δ 7.60 - 7.48 (m, 2H), 7.43 - 6.97 (m, 10H), 6.63 (s, 1H), 3.46 (dd, 2H), 3.37 - 3.19 (m, 2H), 2.82 - 2.42 (m, 2H), 2.27 - 2.00 (m, 2H), 1.96 - 1.80 (m, 1H), 1.73 - 1.59 (m, 2H), 1.58 - 1.44 (m, 1H), 1.16 - 1.03 (m, 1H). ¹³C NMR (101 MHz, CDCl₃) δ 162.09, 148.72, 137.94, 137.39, 133.71, 129.54, 129.15, 128.97, 128.54, 128.43, 127.44, 126.18, 123.49, 63.35, 57.43, 53.90, 43.81, 35.60, 28.26, 24.11. TLC-MS (ESI+): calcd. *m*/*z* 390.18 for C₂₄H₂₆N₂OS. Found 390.9 [M+H]⁺. HPLC *t*_{R} = 5.838 min.

*(4-benzyl-1,4-diazepan-1-yl)(5-phenylthiophen-2-yl)methanone (40).* Prepared from 2-phenylthiazole-4-carboxylic acid (0.5 mmol) and 1-benzyl-1,4-diazepane (0.5 mmol, 1 eq.) according to general procedure 1. Purification by flash chromatography with DCM/MeOH (1-3%). Yield: 152 mg (83%), white solid. ¹H NMR (400 MHz, DMSO) δ 7.70 (d, *J* = 7.5 Hz, 2H), 7.55 - 7.17 (m, 10H), 3.90 - 3.53 (m, *J* = 65.5 Hz, 6H), 2.79 - 2.57 (m, 4H), 1.88 (s, 2H). ¹³C NMR (101 MHz, DMSO) δ 162.66, 146.27, 138.99, 137.51, 132.94, 130.27, 129.25, 128.55, 128.45, 128.21, 126.91, 125.68, 123.66, 61.08, 54.42, 48.38, 45.81, 38.25, 28.27. TLC-MS (ESI+): calcd. *m*/*z* 376.16 for C₂₃H₂₄N₂OS. Found 377.2 [M+H]⁺. HPLC *t*_{R} = 5.182 min.

*4-fluoro-N-(3-((4-(5-phenylthiophene-2-carbonyl)-1,4-diazepan-1-yl)methyl)phenyl)benzamide (41).* Prepared from 5-phenylthiophene-2-carboxylic acid (0.27 mmol) and *N*-(3-((1,4-diazepan-1-yl)methyl)phenyl)-4-fluorobenzamide hydrochloride (41a, 0.27 mmol, 1 eq.) according to general procedure 1. Purification by flash chromatography with DCM/MeOH (1-3%). Yield: 80 mg (57%), pale yellow solid. ¹H NMR (400 MHz, CDCl₃) δ 8.07 (s, 1H), 7.85 (s, 2H), 7.70 - 7.53 (m, *J* = 6.9 Hz, 4H), 7.42 - 7.36 (m, *J* = 7.4 Hz, 2H), 7.35 - 7.26 (m, 3H), 7.22 (s, 1H), 7.15 - 7.05 (m, 3H), 3.79 (s, 4H), 3.65 (s, 2H), 2.84 - 2.74 (m, *J* = 13.7, 8.7 Hz, 2H), 2.70 (s, 2H), 1.96 (quint, 2H). ¹³C NMR (101 MHz, CDCl₃) δ 166.24, 164.94, 164.36, 163.73, 138.25, 137.07, 133.59, 131.23, 131.20, 130.19, 129.74, 129.66, 129.18, 129.15, 128.48, 126.19, 125.11, 122.80, 120.77, 119.46, 115.97, 115.75, 62.27, 54.93, 49.71, 49.05, 46.55, 29.82. TLC-MS (ESI+): calcd. *m*/*z* 513.19 for C₃₀H₂₈FN₃O₂S. Found 536.2 [M+Na]⁺. HPLC *t*_{R} = 6.447 min.

*N-(3-nitrobenzyl)-5-phenylthiophene-2-carboxamide (42)*. Prepared from 5-phenylthiophene-2-carboxylic acid (0.98 mmol) and (3-nitrophenyl)methanamine hydrochloride (42a, 0.98 mmol, 1 eq.) according to general procedure 1. Purification by flash chromatography with DCM/MeOH (0-1%). Yield: 230 mg (69%), white solid. ¹H NMR (400 MHz, CDCl₃) δ 8.19 (s, 1H), 8.11 (d, *J* = 8.2, 1.3 Hz, 1H), 7.72 (d, *J* = 7.8 Hz, 1H), 7.64 - 7.57 (m, 2H), 7.54 (d, *J* = 3.9 Hz, 1H), 7.50 (t, *J* = 7.9 Hz, 1H), 7.44 - 7.30 (m, 3H), 7.26 (d, *J* = 3.8 Hz, 1H), 6.73 (t, *J* = 5.6 Hz, 1H), 4.71 (d, *J* = 6.1 Hz, 2H). ¹³C NMR (101 MHz, CDCl₃) δ 162.24, 149.74, 148.58, 140.63, 136.67, 134.09, 133.45, 129.85, 129.70, 129.24, 128.85, 126.28, 123.69, 122.71, 122.60, 43.30. TLC-MS (ESI+): calcd. m/z 338.07 for C₁₈H₁₄N₂O₃S. Found 361.1 [M+Na]⁺. HPLC *t*_{R} = 8.347 min.

*N-(3-(4-fluorobenzamido)benzyl)-5-phenylthiophene-2-carboxamide (43)*. Prepared from 4-fluorobenzoic acid (0.5 mmol) and *N*-(3-aminobenzyl)-5-phenylthio-phene-2-carboxamide (43a, 0.5 mmol, 1 eq.) according to general procedure 1. Purification by suction filtration and washing with water. Yield: 187 mg (87%), white solid. ¹H NMR (400 MHz, DMSO) δ 10.29 (s, 1H), 9.12 (s, 1H), 8.36 - 6.84 (m, *J* = 183.8, 132.7, 90.8 Hz, 15H), 4.38 (d, *J* = 90.2 Hz, 2H). ¹³C NMR (101 MHz, DMSO) δ 165.28, 164.43, 162.80, 160.96, 147.51, 139.97, 139.19, 138.83, 133.12, 131.34, 130.43, 130.34, 129.23, 128.56, 128.49, 125.66, 124.32, 122.73, 119.16, 119.01, 115.37, 115.16, 42.55. TLC-MS (ESI+): calcd. *m*/*z* 430.12 for C₂₅H₁₉FN₂O₂S. Found 453.2 [M+Na]⁺. HPLC *t*_{R} = 8.786 min.

*N-(2-(1-methylpyrrolidin-2-yl)ethyl)-2-phenylthiazole-4-carboxamide (44).* Prepared from 2-phenylthiazole-4-carboxylic acid (0.39 mmol) and 2-(1-methylpyrrolidin-2-yl)ethan-1-amine (0.39 mmol, 1 eq.) according to general procedure 1. Purification by flash chromatography with DCM/MeOH (10%). Yield: 48 mg (39%), pale brown semi-solid. ¹H NMR (400 MHz, CDCl₃) δ 8.04 (s, 1H), 7.98 - 7.87 (m, 3H), 7.46 - 7.40 (m, 3H), 3.73 - 3.39 (m, 3H), 3.08 - 2.93 (m, 1H), 2.80 - 2.71 (m, *J* = 10.3 Hz, 1H), 2.69 (s, 3H), 2.39 - 2.02 (m, 4H), 1.99 - 1.84 (m, 2H). ¹³C NMR (101 MHz, CDCl₃) δ 168.42, 161.65, 150.51, 132.79, 130.78, 129.15, 126.73, 123.04, 66.48, 56.16, 39.35, 36.57, 31.07, 29.79, 21.73. TLC-MS (ESI+): calcd. *m*/*z* 315.14 for C₁₇H₂₁N₃OS. Found 316.2 [M+H]⁺. HPLC *t*_{R} = 3.921 min.

*tert-butyl 4-(2-phenylthiazole-4-carbonyl)-1,4-diazepane-1-carboxylate (45)*. Prepared from 2-phenylthiazole-4-carboxylic acid (2.44 mmol) and *tert*-butyl 1,4-di-azepane-1-carboxylate (2.44 mmol, 1 eq.) according to general procedure 1. Purification by flash chromatography with PE/EtOAc (10-30%). Yield: 784 mg (83%), yellow oil. ¹H NMR (400 MHz, DMSO) δ 8.27 - 8.09 (m, *J* = 26.9, 12.4 Hz, 1H), 8.04 - 7.91 (m, *J* = 3.4 Hz, 2H), 7.66 - 7.45 (m, *J* = 4.1 Hz, 3H), 3.95 - 3.36 (m, 8H), 1.99 - 1.72 (m, *J* = 32.8, 15.9 Hz, 2H), 1.47 - 1.20 (m, *J* = 35.3 Hz, 9H). ¹³C NMR (101 MHz, DMSO) δ 166.4, 163.1, 154.4, 151.0, 132.6, 130.7, 129.3, 126.3, 126.2, 78.7, 45.3, 34.9, 28.3, 28.0, 27.8, 26.0. TLC-MS (ESI+): calcd. *m*/*z* 387.16 for C₂₀H₂₅N₃O₃S. Found 410.2 [M+Na]⁺. HPLC *t*_{R} = 8.982 min.

*tert-butyl 4-(3-(2-phenylthiazole-4-carboxamido)propyl)piperazine-1-carboxylate (46).* Prepared from 2-phenylthiazole-4-carboxylic acid (0.43 mmol) and tert-butyl 4-(3-aminopropyl)piperazine-1-carboxylate (46a, 0.43 mmol, 1 eq.) according to general procedure 1. Purification by suction filtration and washing with water. Yield: 128 mg (69%), white solid. ¹H NMR (400 MHz, DMSO) δ 8.65 (t, 1H), 8.28 (s, 1H), 8.02 (s, 2H), 7.52 (s, 3H), 3.43 - 3.32 (m, *J* = 16.8 Hz, 6H), 2.47 - 2.20 (m, 6H), 1.71 (quint, 2H), 1.39 (d, *J* = 17.7 Hz, 9H). ¹³C NMR (101 MHz, DMSO) δ 167.2, 160.2, 153.8, 150.9, 132.5, 130.7, 129.2, 126.4, 123.9, 78.7, 56.2, 52.6, 37.9, 28.0, 25.9. TLC-MS (ESI+): calcd. *m*/*z* 430.20 for C₂₂H₃₀N₄O₃S. Found 431.2 [M+H]⁺. HPLC *t*_{R} = 5.651 min.

*tert-butyl 4-(3-(((2-phenylthiazol-4-yl)methyl)amino)propyl)piperazine-1-carboxylate (47).* Prepared from 2-phenylthiazole-4-carbaldehyde (0.47 mmol, 1.1 eq.) and tert-butyl 4-(3-aminopropyl)piperazine-1-carboxylate (46a, 0.43 mmol) according to general procedure 3. Purification by flash chromatography with DCM/MeOH (5-8%). Yield: 13 mg (7%), yellow oil. ¹H NMR (400 MHz, CDCl₃) δ 7.94 - 7.81 (m, *J* = 6.3, 2.7 Hz, 2H), 7.54 (s, 1H), 7.48 - 7.40 (m, *J* = 6.7, 3.5 Hz, 3H), 4.26 (s, 2H), 3.41 (s, 4H), 3.20 (s, 2H), 2.49 (d, *J* = 33.3 Hz, 6H), 2.00 (quint, 2H), 1.42 (s, 9H). ¹³C NMR (101 MHz, CDCl₃) δ 169.9, 154.6, 133.0, 130.8, 129.2, 126.7, 119.7, 80.0, 58.1, 48.8, 46.8, 29.8, 28.5, 22.1, 14.2. TLC-MS (ESI+): calcd. *m*/*z* 416.22 for C₂₂H₃₂N₄O₂S. Found 417.2 [M+Na]⁺. HPLC *t*_{R} = 3.448 min.

*N-benzyl-1-((2-phenylthiazol-4-yl)methyl)piperidine-4-carboxamide (48).* Prepared from 2-phenylthiazole-4-carbaldehyde (0.39 mmol, 1 eq.) and *N*-benzylpiperidine-4-carboxamide hydrochloride (48a, 0.48 mmol) according to general procedure 3. Purification by flash chromatography with DCM/MeOH (2-7%). Yield: 99 mg (71%), white solid. ¹H NMR (400 MHz, CDCl₃) δ 8.00 - 7.89 (m, 2H), 7.45 - 7.37 (m, 3H), 7.35 - 7.22 (m, 5H), 7.13 (s, 1H), 5.76 (t, 1H), 4.44 (d, *J* = 5.6 Hz, 2H), 3.73 (d, *J* = 0.6 Hz, 2H), 3.07 (dt, *J* = 8.9, 3.0 Hz, 2H), 2.21 - 2.04 (m, 3H), 1.92 - 1.75 (m, 4H). ¹³C NMR (101 MHz, CDCl₃) δ 174.9, 168.0, 155.2, 138.5, 133.9, 130.0, 129.0, 128.9, 127.9, 127.7, 126.7, 115.9, 58.7, 53.4, 43.6, 43.5, 29.2. TLC-MS (ESI+): calcd. *m*/*z* 391.17 for C₂₃H₂₅N₃OS. Found 392.2 [M+H]⁺. HPLC *t*_{R} = 5.446 min.

*tert-butyl 4-(2-(benzo[b]thiophene-2-carboxamido)ethyl)piperazine-1-carboxylate (49).* Prepared from benzo[b]thiophene-2-carboxylic acid (0.39 mmol) and *tert-*butyl 4-(2-aminoethyl)piperazine-1-carboxylate (0.39 mmol, 1 eq.) according to general procedure 1. Purification by flash chromatography with DCM/MeOH (0-3%). Yield: 7 mg (46%), yellow oil. ¹H NMR (400 MHz, CDCl₃) δ 7.88 - 7.80 (m, *J* = 10.3, 4.2 Hz, 2H), 7.77 (s, 1H), 7.49 - 7.33 (m, 2H), 6.95 (t, 1H), 3.57 (q, *J* = 11.3, 5.6 Hz, 2H), 3.52 - 3.41 (m, 4H), 2.63 (t, *J* = 6.0 Hz, 2H), 2.55 -2.39 (m, 4H), 1.46 (s, 9H). ¹³C NMR (101 MHz, CDCl₃) δ 162.4, 154.8, 140.9, 139.2, 138.6, 126.4, 125.3, 125.1, 125.0, 122.8, 80.0, 56.5, 52.7, 36.4, 28.5. TLC-MS (ESI+): calcd. *m*/*z* 389.18 for C₂₀H₂₇N₃O₃S. Found 412.2 [M+Na]⁺. HPLC *t*_{R} = 5.225 min.

### Assays

ACKR3 *β-Arrestin* Recruitment Assay. The potency and efficacy of final compounds 1 to 49 were evaluated by ligand-induced recruitment of *β-Arrestin* to ACKR3 using a NanoLuc complementation assay (NanoBiT, Promega Corporation, Madison, WI, USA) in HEK293T cells (Szpakowska, M.; Nevins, A. M.; Meyrath, M.; Rhainds, D.; D'huys, T.; Guité-Vinet, F.; Dupuis, N.; Gauthier, P.-A.; Counson, M.; Kleist, A.; St-Onge, G.; Hanson, J.; Schols, D.; Volkman, B. F.; Heveker, N.; Chevigné, A. Different contributions of chemokine n-terminal features attest to a different ligand binding mode and a bias towards activation of ACKR3/CXCR7 compared with CXCR4 and cxcr3. Br. J. Pharmacol. 2018, 175, 1419-1438; Dixon, A. S.; Schwinn, M. K.; Hall, M. P.; Zimmerman, K.; Otto, P.; Lubben, T. H.; Butler, B. L.; Binkowski, B. F.; Machleidt, T.; Kirkland, T. A.; Wood, M. G.; Eggers, C. T.; Encell, L. P.; Wood, K. V. Nanoluc complementation reporter optimized for accurate measurement of protein interactions in cells. ACS Chem. Biol. 2016, 11, 400-408; Meyrath, M.; Palmer, C. B.; Reynders, N.; Vanderplasschen, A.; Ollert, M.; Bouvier, M.; Szpakowska, M.; Chevigné, A. Proadrenomedullin n-terminal 20 peptides (pamps) are agonists of the chemokine scavenger receptor ACKR3/CXCR7. ACS Pharmacology & Translational Science 2021, 4, 813-823; and Szpakowska, M.; Meyrath, M.; Reynders, N.; Counson, M.; Hanson, J.; Steyaert, J.; Chevigné, A. Mutational analysis of the extracellular disulphide bridges of the atypical chemokine receptor ACKR3/CXCR7 uncovers multiple binding and activation modes for its chemokine and endogenous non-chemokine agonists. Biochem. Pharmacol. 2018, 153, 299-309). In short: 5 × 10E6 HEK293T cells were seeded in 10-cm culture dishes and 24 h later co-transfected with pNBe vectors encoding human ACKR3 or CXCR4 receptors C-terminally fused to SmBiT and *β-Arrestin*-1 N-terminally fused to LgBiT. After 24 h, transfection cells were harvested, incubated for 15 minutes at 37 °C with 200-fold diluted Nano-Glo Live Cell substrate, and distributed into white 96-well plates (5 × 104 cells per well). The prepared cells were then treated with compounds at concentrations ranging from 0.3 nM to 100 µM. Induction of *β-Arrestin* recruitment to receptors was evaluated by measuring bioluminescence with a Mithras LB940 luminometer (Berthold Technologies). For determination of a compound's efficacy and concentration-response curves, the signal recorded was compared to values for CXCL12 at 300 nM considered as full agonist reference ligand (Eₘₐₓ = 100 %).

All curves were fitted to data points generated from the mean of at least three independent experiments. Results with values of p < 0.05 were considered statistically significant.

Flow Cytometry Assay. The inhibition of platelet degranulation was determined via flow cytometry experiments. Platelets were treated with collagen-related peptide (CRP-XL, CambCol Laboratories, Cambridge, UK) and the platelets' activation state was determined (Geue, S.; Aurbach, K.; Manke, M.-C.; Manukjan, G.; Münzer, P.; Stegner, D.; Brähler, C.; Walker-Allgaier, B.; Märklin, M.; Borst, C. E.; Quintanilla-Fend, L.; Rath, D.; Geisler, T.; Salih, H. R.; Seizer, P.; Lang, F.; Nieswandt, B.; Gawaz, M.; Schulze, H.; Pleines, I.; Borst, O. Pivotal role of pdk1 in megakaryocyte cytoskeletal dynamics and polarization during platelet biogenesis. Blood 2019, 134, 1847-1858; and Malehmir, M.; Pfister, D.; Gallage, S.; Szydlowska, M.; Inverso, D.; Kotsiliti, E.; Leone, V.; Peiseler, M.; Surewaard, B. G. J.; Rath, D.; Ali, A.; Wolf, M. J.; Drescher, H.; Healy, M. E.; Dauch, D.; Kroy, D.; Krenkel, O.; Kohlhepp, M.; Engleitner, T.; Olkus, A.; Sijmonsma, T.; Volz, J.; Deppermann, C.; Stegner, D.; Helbling, P.; Nombela-Arrieta, C.; Rafiei, A.; Hinterleitner, M.; Rail, M.; Baku, F.; Borst, O.; Wilson, C. L.; Leslie, J.; O'Connor, T.; Wes-ton, C. J.; Chauhan, A.; Adams, D. H.; Sheriff, L.; Teijeiro, A.; Prinz, M.; Bogeska, R.; An-stee, N.; Bongers, M. N.; Notohamiprodjo, M.; Geisler, T.; Withers, D. J.; Ware, J.; Mann, D. A.; Augustin, H. G.; Vegiopoulos, A.; Milsom, M. D.; Rose, A. J.; Lalor, P. F.; Llovet, J. M.; Pinyol, R.; Tacke, F.; Rad, R.; Matter, M.; Djouder, N.; Kubes, P.; Knolle, P. A.; Unger, K.; Zender, L.; Nieswandt, B.; Gawaz, M.; Weber, A.; Heikenwalder, M. Platelet gpibα is a mediator and potential interventional target for nash and subsequent liver cancer. Nat. Med. 2019, 25, 641-655). Briefly, PRP was isolated from peripheral human blood collected in citrate-phosphate-dextrose solution with adenine (CPDA) and diluted with phosphate-buffered saline (PBS) (Sigma Aldrich Co., Ltd., St. Luis, Missouri, USA) supplemented with CaCl₂ and MgCl₂. PRP (106 platelets /sample) was preincubated with the respective compound (100 *µ*M) for 15 min. Samples were treated with 1 *µ*g/ml CRP-XL (CambCol Laboratories, Cambridge, UK) for 30 min at room temperature in the presence of the respective fluorochrome-conjugated antibody against P-Selectin (CD62P-FITC; Beckman Coulter, Brea, CA, USA). Subsequently, samples were fixed in 0.5% paraformaldehyde and analyzed on a FACS-Calibur flow cytometer (FACS-Caliber flow cytometer, Becton-Dickinson, East Rutherford, NJ, USA). Mean fluorescence intensity (MFI) was used as a quantitative measurement of platelet surface expression.

Cell Viability Assay. RealTime-Glo^{™} MT Cell Viability Assay (Promega Corporation, Madison, WI, USA) was performed using THP1 cells and HEK293 cells as indicated in the manufacturer's manual to measure a compound's cytotoxicity on cells. In brief, in a white solid-bottom 96-well plate cells (5x103/well) were seeded and incubated with the respective compounds diluted in DMEM (Gibco, Carlsbad, CA, USA). After cell seeding viability reagent is added. Subsequently, cells were incubated with the compound and viability reagent containing medium at 37 °C, 5% CO2 for 72 h. The luminescence signal was determined after 1, 24, 48, and 72 hours on a GloMax^{®}-Multi Detection System plate reader (Promega Corporation, Madison, Wl, USA).

Metabolic Stability Assay. Pooled liver microsomes from mice (male) and humans (male) were purchased from *Sekisui XenoTech, LLC,* Kansas City, KS, USA. Metabolic stability assays were performed in the presence of an NADPH-regenerating system consisting of 5 mM glucose-6-phosphate, 5 U/mL glucose-6-phosphate dehydrogenase, and 1 mM NADP⁺. Liver microsomes (20 mg/mL), NADPH-regenerating system, and 4 mM MgCl₂·6 H₂O in 0.1 M TRIS-HCl-buffer (pH 7.4) were preincubated for 5 min at 37 °C and 750 rpm on a shaker. The reaction was started by adding the preheated compound at 10 mM resulting in a final concentration of 0.1 mM. The reaction was quenched at selected time points (0, 10, 20, 30, 60, and 120 min) by pipetting 100 *µ*L of internal standard (ketoprofen) in acetonitrile at concentrations of 600 *µ*M (compound 26), 800 *µ*M (compound 27), and 450 *µ*M (compound 35), respectively. The samples were vortexed for 30 s and centrifuged (21910 relative centrifugal force, 4 °C, 20 min). The supernatant was used directly for LC-MS analysis.

All compound incubations were conducted at least in triplicates. Additionally, a negative control containing BSA (20 mg/mL) instead of liver microsomes and a positive control using Verapamil instead of the compound was performed. A limit of 1% organic solvent during incubation was not exceeded. Sample separation and detection were performed on an *Alliance 2695 Separations Module* HPLC system (*Waters Corporation*, Milford, MA, USA) equipped with a *Phenomenex Kinetex* 2.6 *µ*m XB-C18 100 Å 50 × 3 mm column (*Phenomenex Inc.*, Torrance, CA, USA) coupled to an *Alliance* 2996 *Photodiode Array Detector* and a *MICROMASS QUATTRO micro API* mass spectrometer (both *Waters Corporation*, Milford, MA, USA) using electrospray ionization in positive mode. Mobile phase A: 90% water, 10% acetonitrile, and additionally 0.1% formic acid (v/v), mobile phase B: 100% acetonitrile with additional 0.1% formic acid (v/v). The gradient was set to 0-2.5 min 0% B, 2.5-10 min from 0 to 40% B, 10-12 min 40% B, 12-12.01 min from 40 to 0% B, 12.01-17 min 0% B at a flow rate of 0.7 mL/min. Samples were maintained at 10 °C, the column temperature was set to 20 °C with an injection volume of 5 *µ*L. Spray, cone, extractor, and RF lens voltages were at 4 kV, 30 V, 8 V, and 2 V, respectively. The source and desolvation temperatures were set to 120 °C and 350 °C, respectively, and the desolvation gas flow was set to 750 Uh. Data analysis was conducted using *Mass-Lynx 4.1* software (*Waters Corporation*, Milford, MA, USA).

Solubility Assay. A 100 mL flask with phosphate-buffered saline was prepared from NaCl, KCI, Na₂HPO₄, KH₂PO₄, and distilled water. The buffer was adjusted to pH 7.4 with HCl. Afterwards, 10mM stock solutions of compounds in DMSO were prepared. 10 µL compound stock solution was added to 990 µL PBS in Eppendorf tubes. As a reference, 10 µL compound stock solution was added to 990 µL HPLC-grade MeOH in Eppendorf tubes. All tubes were centrifuged (16000 relative centrifugal force, 4 °C, 20 min). Subsequently, 600 µL supernatant was extracted carefully and filled into HPLC vials. Compound area under the curve (AUC) was determined by RP-HPLC using an Agilent 1100 Series LC with a Phenomenex Luna C8 analytical column (150 × 4.6 mm, 5 µm) and detected by a UV DAD detector at 254 nm and 230 nm wavelength. Compounds solubility was calculated by AUC of compound in PBS divided by AUC of reference solution multiplied by concentration of the compound in solution.

Aggregation experiments. For the light transmission aggregation experiments, citrate anticoagulated blood from healthy donors was used. To obtain platelet-rich plasma, whole blood was centrifuged at 210 × g for 20 min at room temperature. 1 × 10^8 platelets were preincubated with 100 µM ACKR3 agonist for 15 min at 37°C. Subsequently, platelets were activated with 2.5 µM ADP and aggregation was analyzed for 5 min at 1,000 rpm and 37°C using a light transmission aggregometer (Aggregometer 490-X; Chrono-Log Corp., Havertown, Pennsylvania, United States). Maximum platelet aggregation and the area under the curve were quantified using Aggrolink8 software (Chrono-Log Corp., Havertown, Pennsylvania, United States).

### Synthesis of intermediates

*N*¹-benzyl-*N*¹-methylpropane-1,3-diamine dihydrochloride (1a). 0.33 g (1.2 mmol) 1b was dissolved in 6 mL (23.7 mmol) 4M HCl in 1,4-dioxane. The mixture was stirred at room temperature for 3 h. The formed HCl-salt of the product was filtered off by suction filtration and dried under reduced pressure obtaining a yellow solid in 72 % yield (0.215 g, 0.86 mmol). The product was used without further purification. ¹H NMR (400 MHz, DMSO) δ 11.24 (s, 1H), 8.31 (s, 3H), 7.69 - 7.60 (m, 2H), 7.48 - 7.41 (m, 3H), 4.41 - 4.18 (m, *J* = 18.8, 13.0, 5.2 Hz, 2H), 3.24 - 3.03 (m, 2H), 2.94 - 2.79 (m, 2H), 2.61 (d, *J* = 4.9 Hz, 3H), 2.10 (quint, 2H). ¹³C NMR (101 MHz, DMSO) δ 131.50, 129.87, 129.46, 128.76, 58.15, 51.69, 38.50, 36.24, 21.61. TLC-MS (ESI+): calcd. *m*/*z* 178.15 for C₁₁H₁₈N₂. Found 179.2 [M+H]⁺.

*N*¹-(4-methoxybenzyl)- *N*¹-methylpropane-1,3-diamine dihydrochloride (18a). 0.315 g (1.02 mmol) 18b was dissolved in 5.1 mL (20.4 mmol) 4M HCl in 1,4-dioxane. The mixture was stirred at room temperature for 3 h. The formed product was filtered off by suction filtration and dried under reduced pressure obtaining a white solid in 90 % yield (0.258 g, 0.92 mmol). The product was used without further purification. ¹H NMR (400 MHz, DMSO) δ 11.14 (s, 1H), 8.34 (s, 3H), 7.56 (d, *J* = 8.7 Hz, 2H), 6.98 (d, *J* = 8.7 Hz, 2H), 4.34 - 4.10 (m, *J* = 18.2, 13.0, 4.7 Hz, 2H), 3.77 (s, 3H), 3.24 - 2.96 (m, 2H), 2.95 - 2.77 (m, *J* = 12.1, 6.4 Hz, 2H), 2.58 (d, *J* = 4.4 Hz, 3H), 2.10 (quint, 2H). ¹³C NMR (101 MHz, DMSO) δ 159.99, 133.02, 121.56, 114.06, 57.64, 55.23, 51.32, 38.21, 36.22, 21.61. TLC-MS (ESI+): calcd. *m*/*z* 208.16 for C₁₂H₂₀N₂O. Found 209.1 [M+H]⁺.

*N*¹-(4-bromobenzyl)-*N*¹-methylpropane-1,3-diamine dihydrochloride (19a). 0.354 g (0.99 mmol) 19b was dissolved in 5 mL (19.8 mmol) 4M HCl in 1,4-dioxane. The mixture was stirred at room temperature for 3 h. The formed product was filtered off by suction filtration and dried under reduced pressure obtaining a white solid in 98 % yield (0.32 g, 0.97 mmol). The product was used without further purification. ¹H NMR (400 MHz, DMSO) δ 11.38 (s, 1H), 8.30 (s, 3H), 7.71 - 7.58 (m, 4H), 4.42 - 4.18 (m, *J* = 18.7, 13.1, 5.1 Hz, 2H), 3.27 - 2.98 (m, 2H), 2.94 - 2.77 (m, 2H), 2.61 (d, *J* = 4.8 Hz, 3H), 2.10 (quint, 2H). ¹³C NMR (101 MHz, DMSO) δ 133.73, 131.68, 129.26, 123.10, 57.30, 51.61, 38.47, 36.19, 21.57. TLC-MS (ESI+): calcd. m/z 256.06 for C₁₁H₁₇BrN₂. Found 257.0/259.0 [M+H]⁺.

*N*¹-(4-fluorobenzyl)-*N*¹-methylpropane-1,3-diamine dihydrochloride (20a). 0.313 g (1.06 mmol) 20b was dissolved in 5.3 mL (21.1 mmol) 4M HCl in 1,4-dioxane. The mixture was stirred at room temperature for 3 h. The formed HCl-salt of the product was filtered off by suction filtration and dried under reduced pressure obtaining a white solid. The crude product was used without further purification.

*N*¹-(2-fluorobenzyl)- *N*¹-methylpropane-1,3-diamine dihydrochloride (21a). 0.43 g (1.45 mmol) 21b was dissolved in 7.25 mL (29 mmol) 4M HCl in 1,4-dioxane. The mixture was stirred at room temperature for 3 h. The formed product was filtered off by suction filtration and dried under reduced pressure obtaining a white semi-solid in 86 % yield (0.336 g, 1.25 mmol). The product was used without further purification. ¹H NMR (400 MHz, DMSO) δ 11.32 (s, 1H), 8.34 (s, 3H), 7.83 (td, *J* = 7.6, 1.4 Hz, 1H), 7.65 - 7.44 (m, *J* = 7.3, 1.6 Hz, 1H), 7.39 - 7.19 (m, 2H), 4.45 - 4.20 (m, *J* = 18.9, 13.4, 4.6 Hz, 2H), 3.33 - 3.06 (m, 2H), 2.98 - 2.77 (m, 2H), 2.65 (d, *J* = 4.6 Hz, 3H), 2.23 - 2.03 (m, 2H). ¹³C NMR (101 MHz, DMSO) δ 161.09, 134.10, 132.29, 124.85, 116.85, 115.85, 51.84, 51.22, 38.68, 36.17, 21.75. TLC-MS (ESI+): calcd. *m*/*z* 196.14 for C₁₁H₁₇FN₂. Found 197.1 [M+H]⁺.

*N*¹-(3-fluorobenzyl)-*N*¹-methylpropane-1,3-diamine dihydrochloride (22a). 0.35 g (1.18 mmol) 22b was dissolved in 5.9 mL (23.6 mmol) 4M HCl in 1,4-dioxane. The mixture was stirred at room temperature for 3 h. The formed product was filtered off by suction filtration and dried under reduced pressure obtaining a white solid in 97 % yield (0.307 g, 1.14 mmol). The product was used without further purification. ¹H NMR (400 MHz, DMSO) δ 11.40 (s, 1H), 8.29 (s, 3H), 7.61 (d, *J* = 8.4 Hz, 1H), 7.55 - 7.43 (m, 2H), 7.39 - 7.23 (m, *J* = 9.0, 5.9, 3.7 Hz, 1H), 4.51 - 4.18 (m, 2H), 3.29 - 2.99 (m, 2H), 2.98 - 2.76 (m, 2H), 2.62 (s, 3H), 2.11 (quint, 2H). ¹³C NMR (101 MHz, DMSO) δ 161.90, 132.50, 130.79, 127.68, 118.27, 116.42, 57.44, 51.80, 38.57, 36.21, 21.60. TLC-MS (ESI+): calcd. *m*/*z* 196.14 for C₁₁H₁₇FN₂. Found 197.1 [M+H]⁺.

*N*¹-benzyl-*N*¹-ethylpropane-1,3-diamine dihydrochloride (23a). 0.28 g (0.96 mmol) 23b was dissolved in 4.8 mL (19.2 mmol) 4M HCl in 1,4-dioxane. The mixture was stirred at room temperature for 3 h. The formed HCl-salt of the product was filtered off by suction filtration and dried under reduced pressure obtaining a white solid. The crude product was used without further purification.

*N*¹-benzyl-*N*¹-isopropylpropane-1,3-diamine dihydrochloride (24a). 0.205 g (0.67 mmol) 24b was dissolved in 3.34 mL (13.4 mmol) 4M HCl in 1,4-dioxane. The mixture was stirred at room temperature for 1 h. The formed product was filtered off by suction filtration and dried under reduced pressure. The product was used without further purification.

*N*¹-benzyl-*N*¹-cyclopropylpropane-1,3-diamine dihydrochloride (25a). 0.357 g (1.17 mmol) 25b was dissolved in 5.85 mL (23.45 mmol) 4M HCl in 1,4-dioxane. The mixture was stirred at room temperature for 1 h. The formed product was filtered off by suction filtration and dried under reduced pressure. The product was used without further purification. TLC-MS (ESI+): calcd. *m*/*z* 204.16 for C₁₃H₂₀N₂. Found 205.2 [M+H]⁺.

*N*¹-benzyl-*N*¹-propylpropane-1,3-diamine dihydrochloride (26a). 0.37 g (1.21 mmol) 26b was dissolved in 6.04 mL (24.15 mmol) 4M HCl in 1,4-dioxane. The mixture was stirred at room temperature for 1 h. The formed product was filtered off by suction filtration and dried under reduced pressure obtaining a colorless oil. The product was used without further purification. TLC-MS (ESI+): calcd. *m*/*z* 206.18 for C₁₃H₂₂N₂. Found 207.2 [M+H]⁺.

*N*¹-benzyl-*N*¹-butylpropane-1,3-diamine dihydrochloride (27a). 0.375 g (1.17 mmol) 27b was dissolved in 5.85 mL (23.4 mmol) 4M HCl in 1,4-dioxane. The mixture was stirred at room temperature for 1 h. The formed product was filtered off by suction filtration and dried under reduced pressure. The product was used without further purification. TLC-MS (ESI+): calcd. *m*/*z* 220.19 for C₁₄H₂₄N₂. Found 221.2 [M+H]⁺.

3-((3-aminopropyl)(benzyl)amino)propan-1-ol dihydrochloride (28a). 0.388 g (1.2 mmol) 28b was dissolved in 6 mL (24 mmol) 4M HCl in 1,4-dioxane. The mixture was stirred at room temperature overnight. The formed product was dried under reduced pressure obtaining a yellow oil in 99 % yield (0.353 g, 1.2 mmol). The product was used without further purification. ¹H NMR (400 MHz, DMSO) δ 11.09 (s, 1H), 8.24 (s, 3H), 7.75 - 7.61 (m, 2H), 7.49 - 7.39 (m, 3H), 4.38 - 4.24 (m, 2H), 3.49 - 3.36 (m, 2H), 3.22 - 2.79 (m, 7H), 2.20 - 1.97 (m, 2H), 2.00 - 1.81 (m, 2H). ¹³C NMR (101 MHz, DMSO) δ 131.39, 129.83, 129.41, 128.75, 57.90, 55.81, 49.25, 48.85, 36.25, 25.76, 21.16. TLC-MS (ESI+): calcd. *m*/*z* 222.17 for C₁₃H₂₂N₂O. Found 223.1 [M+H]⁺.

*N*¹-benzyl-*N*¹-phenethylpropane-1,3-diamine dihydrochloride (29a). 0.178 g (0.48 mmol) 29b was dissolved in 2.4 mL (9.66 mmol) 4M HCl in 1,4-dioxane. The mixture was stirred at room temperature overnight. The formed product was filtered off by suction filtration and dried under reduced pressure obtaining a pale-brown solid in 99 % yield (0.164 g, 0.48 mmol). The product was used without further purification. ¹³C NMR (101 MHz, CDCl₃) δ 136.27, 131.57, 130.01, 129.39, 129.05, 128.92, 128.84, 127.14, 56.98, 52.71, 51.21, 37.89, 29.72, 22.35. TLC-MS (ESI+): calcd. *m*/*z* 268.19 for C₁₈H₂₄N₂. Found 269.1 [M+H]⁺. HPLC *t*_{R} = 1.907 min.

*N*¹-benzyl-*N*¹-(3-phenylpropyl)propane-1,3-diamine dihydrochloride (30a). 0.184 g (0.48 mmol) 30b was dissolved in 2.4 mL (9.6 mmol) 4M HCl in 1,4-dioxane. The mixture was stirred at room temperature overnight. The formed product was filtered off by suction filtration and dried under reduced pressure obtaining a pale-brown solid in 99 % yield (0.168 g, 0.47 mmol). The product was used without further purification. ¹H NMR (400 MHz, DMSO) δ 8.29 (s, 3H), 7.65 (dd, *J* = 7.3, 2.2 Hz, 2H), 7.48 - 7.36 (m, 3H), 7.35 - 7.10 (m, 6H), 4.39 - 4.22 (m, 2H), 3.24 - 3.07 (m, 2H), 3.03 - 2.90 (m, 2H), 2.90 - 2.79 (m, 2H), 2.64 - 2.54 (m, 2H), 2.26 - 1.95 (m, 4H). ¹³C NMR (101 MHz, DMSO) δ 140.36, 131.38, 129.74, 129.34, 128.70, 128.32, 128.28, 126.06, 55.65, 50.66, 48.95, 36.23, 31.98, 24.01, 21.22. TLC-MS (ESI+): calcd. *m*/*z* 282.21 for C₁₉H₂₆N₂. Found 283.3 [M+H]⁺. HPLC *t*_{R} = 2.399 min.

*N*-(3-(benzyl(ethyl)amino)propyl)-5-bromothiophene-2-carboxamide (31a). 0.112 g (0.54 mmol) 5-bromothiophene-2-carboxylic acid was dissolved in 2.2 mL dry DMF and 0.227 g (0.6 mmol) HATU was slowly added. After 15 min of continuous stirring, 0.144 g (0.54 mmol) 23a and 0.43 mL (2.44 mmol) DIPEA was added to the mixture. The mixture was allowed to stir at room temperature overnight. After complete reaction, the mixture was diluted with DCM, washed with water (3x 10 mL) and brine (3x 10 mL). The combined organic phased were dried over Na₂SO₄, filtered, and evaporated to dryness. The mixture was purified via flash chromatography (SiO₂; DCM/MeOH 1 - 4 %). The crude product was used without further purification. TLC-MS (ESI+): calcd. *m*/*z* 380.06 for C₁₇H₂₁BrN₂OS. Found 381.1 [M+H]⁺.

5-bromo-*N*-(3-(ethyl(4-fluorobenzyl)amino)propyl)thiophene-2-carboxamide (32a). 0.088 g (0.42 mmol) 5-bromothiophene-2-carboxylic acid was dissolved in 1.7 mL dry DMF and 0.177 g (0.47 mmol) HATU was slowly added. After 15 min of continuous stirring, 0.12 g (0.42 mmol) 32b and 0.33 mL (1.91 mmol) DIPEA was added to the mixture. The mixture was allowed to stir at room temperature overnight. After complete reaction, the mixture was diluted with DCM, washed with water (3x 10 mL) and brine (3x 10 mL). The combined organic phased were dried over Na₂SO₄, filtered, and evaporated to dryness. The mixture was purified via flash chromatography (SiO₂; DCM/MeOH 1 - 4 %) obtaining a white solid. The crude product was used without further purification. TLC-MS (ESI+): calcd. *m*/*z* 398.05 for C₁₇H₂₀BrFN₂OS. Found 399.1 [M+H]⁺.

*N*-(3-aminopropyl)-5-phenylthiophene-2-carboxamide hydrochloride (34a). 0.6 g (1.66 mmol) 33 was dissolved in 8.3 mL (33.3 mmol) 4M HCl in 1,4-dioxane. The mixture was stirred at room temperature overnight. The formed product was filtered off by suction filtration and dried under reduced pressure obtaining a pale-yellow solid in 99 % yield (0.486 g, 1.64 mmol). The product was used without further purification. ¹H NMR (400 MHz, DMSO) δ 8.92 (t, *J* = 5.6 Hz, 1H), 8.10 (s, 3H), 7.88 (d, *J* = 3.9 Hz, 1H), 7.74 - 7.64 (m, 2H), 7.54 (d, *J* = 3.9 Hz, 1H), 7.44 (dd, *J* = 10.3, 4.7 Hz, 2H), 7.39 - 7.31 (m, 1H), 3.32 (q, *J* = 6.4 Hz, 2H), 2.89 - 2.77 (m, 2H), 1.84 (quint, 2H). ¹³C NMR (101 MHz, DMSO) δ 161.19, 147.33, 138.79, 133.12, 129.36, 129.24, 128.48, 125.64, 124.29, 39.52, 36.68, 36.10, 27.26. TLC-MS (ESI+): calcd. *m*/*z* 260.1 for C₁₄H₁₆N₂OS. Found 261.1 [M+H]⁺. HPLC *t*_{R} = 3.973 min.

3-(3,4-dihydroisoquinolin-2(1*H*)-yl)propan-1-amine dihydrochloride (35a). 0.61 g (2.1 mmol) 35b was dissolved in 10.5 mL (42 mmol) 4M HCl in 1,4-dioxane. The mixture was stirred at room temperature for 1 h. The formed product was filtered off by suction filtration and dried under reduced pressure. The product was used without further purification. TLC-MS (ESI+): calcd. *m*/*z* 190.15 for C₁₂H₁₈N₂. Found 191.2 [M+H]⁺.

*N*-(3-(((3-aminopropyl)(methyl)amino)methyl)phenyl)-4-fluorobenzamide (36a). 0.506 g (1.22 mmol) 36b was dissolved in 6.1 mL (24.4 mmol) 4M HCl in 1,4-dioxane. The mixture was stirred at room temperature for 3 h. After complete reaction, the mixture was diluted with DCM, washed with NH₄Cl (3x 20 mL), NaHCO₃ (3x 20 mL) and brine (3x 20 mL). The combined organic phased were dried over Na₂SO₄, filtered, and evaporated to dryness obtaining a yellow oil. The crude product was used without further purification. TLC-MS (ESI+): calcd. *m*/*z* 315.17 for C₁₈H₂₂FN₃O. Found 316.2 [M+H]⁺.

1-(3-aminopropyl)-*N*-(*tert*-butyl)piperidine-4-carboxamide dihydrochloride (37a). 0.36 g (1.27 mmol) 37b was dissolved in 6.3 mL (25.3 mmol) 4M HCl in 1,4-dioxane. The mixture was stirred at room temperature for 3 h. The formed HCl-salt of the product was filtered off by suction filtration and dried under reduced pressure obtaining a white solid in 77 % yield (0.306 g, 0.98 mmol). The product was used without further purification. ¹H NMR (400 MHz, DMSO) δ 3.45 (d, *J* = 12.4 Hz, 2H), 3.16 (dd, *J* = 12.8, 7.7 Hz, 3H), 2.89 - 2.77 (m, 4H), 2.39 - 2.29 (m, 1H), 2.11 - 1.78 (m, 8H), 1.25 (s, 2H), 1.22 (d, *J* = 4.8 Hz, 9H). ¹³C NMR (101 MHz, DMSO) δ 172.3, 52.9, 51.1, 49.9, 48.7, 36.3, 28.5, 25.7, 23.5, 21.4.

(1-benzylpiperidin-3-yl)methanamine hydrochloride (39a). 0.165 g (0.54 mmol) 39b was dissolved in 2.71 mL (10.8 mmol) 4M HCl in 1,4-dioxane. The mixture was stirred at room temperature overnight. The formed product was filtered off by suction filtration and dried under reduced pressure obtaining a white solid in 99 % yield (0.163 g, 0.53 mmol). The product was used without further purification. ¹H NMR (400 MHz, DMSO) δ 8.55 - 8.01 (m, 3H), 7.76 - 7.59 (m, 2H), 7.53 - 7.17 (m, 3H), 4.45 - 4.09 (m, 2H), 3.49 - 3.23 (m, 2H), 3.11 - 2.57 (m, 4H), 2.46 - 2.28 (m, 1H), 2.02 - 1.69 (m, 3H), 1.19 - 1.00 (m, 1H). ¹³C NMR (101 MHz, DMSO) δ 131.72, 129.37, 129.32, 128.62, 59.39, 53.13, 51.15, 41.12, 31.82, 25.56, 21.24. TLC-MS (ESI+): calcd. *m*/*z* 204.16 for C₁₃H₂₀N₂. Found 205.0 [M+H]⁺. HPLC *t*_{R} = 1.102 min.

*N*-(3-((1,4-diazepan-1-yl)methyl)phenyl)-4-fluorobenzamide hydrochloride (41a). 0.5 g (1.17 mmol) 41b was dissolved in 5.9 mL (23.4 mmol) 4M HCl in 1,4-dioxane. The mixture was stirred at room temperature for 3 h. The formed HCl-salt of the product was filtered off by suction filtration and dried under reduced pressure obtaining a white solid in 92 % yield (0.392 g, 1.08 mmol). The product was used without further purification. ¹H NMR (400 MHz, DMSO) δ 10.53 (s, 1H), 9.91 (s, *J* = 37.5 Hz, 1H), 9.62 (s, 1H), 8.12 - 8.06 (m, 2H), 8.03 (s, 1H), 7.81 - 7.73 (m, *J* = 8.1 Hz, 1H), 7.52 - 7.34 (m, 4H), 3.75 - 3.56 (m, *J* = 25.4, 14.7 Hz, 4H), 3.48 - 3.10 (m, 4H), 2.54 (s, 2H), 2.30 - 2.09 (m, 2H). ¹³C NMR (101 MHz, DMSO) δ 165.38, 164.51, 162.91, 139.39, 131.06, 130.46, 130.23, 129.16, 126.66, 123.38, 121.64, 115.47, 66.35, 59.14, 52.39, 48.83, 43.72, 40.43, 20.34. TLC-MS (ESI+): calcd. *m*/*z* 327.17 for C₁₉H₂₂FN₃O. Found 328.2 [M+H]⁺.

(3-nitrophenyl)methanamine dihydrochloride (42a). 1.13 g (4 mmol) 42b was dissolved in 20 mL EtOH and 0.83 mL (20 mmol) hydrazine hydrate (80% solution in water) was slowly added. The mixture was heated to 60 °C and stirred overnight. After complete consumption of the starting material, the formed precipitate was filtered off. The filtrate was concentrated and redissolved in DCM. To the suspension HCl in 1,4-dioxane was added and the formed solid was filtered off and washed multiple times with EtOAc. The product was dried under reduced pressure obtaining a brown solid. ¹H NMR (400 MHz, DMSO) δ 8.76 (s, 3H), 8.46 (t, *J* = 1.8 Hz, 1H), 8.23 (d, *J* = 8.2, 1.5 Hz, 1H), 8.01 (d, *J* = 7.7 Hz, 1H), 7.71 (t, *J* = 8.0 Hz, 1H), 4.18 (q, *J* = 5.8 Hz, 2H). ¹³C NMR (101 MHz, DMSO) δ 147.65, 136.33, 135.99, 130.05, 124.01, 123.25, 41.26.

*N*-(3-aminobenzyl)-5-phenylthiophene-2-carboxamide (43a). 0.224 g (0.66 mmol) 42 was dissolved in 4 mL EtOH and the solution was cooled to 0 °C. 0.04 g 10% Pd/C was added under a nitrogen atmosphere to the solution. The flask was repeatedly evacuated and flushed with H2 gas and the H2 gas was allowed to bubble in the suspension for 15 min. The mixture was stirred overnight at room temperature. After complete reaction, the flask was purged with N2, and the crude reaction mixture was filtered through a short plug of celite. The reaction vessel and celite were rinsed with EtOH. The combined filtrates were concentrated under reduced pressure and the residue was dried under high vac overnight obtaining a brown solid. The crude product was used without further purification. TLC-MS (ESI+): calcd. *m*/*z* 308.10 for C₁₈H₁₆N₂OS. Found 309.1 [M+H]⁺.

*tert*-butyl 4-(3-aminopropyl)piperazine-1-carboxylate (46a). 4.5 g (12.2 mmol) 46b was dissolved in 61 mL EtOH and 2.52 mL (61 mmol) hydrazine hydrate (80% solution in water) was slowly added. The mixture was heated to 60 °C and stirred overnight. After complete consumption of the starting material, the formed precipitate was filtered off. The filtrate was concentrated and redissolved in DCM. The solution was washed with water (3x 30 mL) and brine (3x 30 mL). The combined organic phased were dried over Na₂SO₄, filtered, and evaporated to dryness obtaining a yellow oil in 77 % yield (2.286 g, 9.4 mmol). The product was used without further purification. ¹H NMR (400 MHz, CDCl₃) δ 3.45 - 3.37 (m, 4H), 2.73 (t, *J* = 6.8 Hz, 2H), 2.45 - 2.30 (m, 6H), 1.62 (quint, 2H), 1.44 (s, 9H), 1.38 (s, *J* = 13.9 Hz, 2H). ¹³C NMR (101 MHz, CDCl₃) δ 154.9, 79.7, 56.6, 53.2, 40.8, 30.6, 28.5.

*N*-benzylpiperidine-4-carboxamide hydrochloride (48a). 0.64 g (2 mmol) 48b was dissolved in 10 mL (40 mmol) 4M HCl in 1,4-dioxane. The mixture was stirred at room temperature for 3 h. The formed HCl-salt of the product was filtered off by suction filtration and dried under reduced pressure obtaining a white solid in 52 % yield (0.266 g, 1.04 mmol). The product was used without further purification. ¹H NMR (400 MHz, DMSO) δ 8.89 (d, *J* = 7.3 Hz, 1H), 8.55 (t, *J* = 5.9 Hz, 1H), 7.37 - 7.17 (m, 5H), 4.25 (d, *J* = 5.9 Hz, 2H), 3.21 (t, *J* = 19.1 Hz, 2H), 2.93 - 2.74 (m, 2H), 2.50 - 2.44 (m, *J* = 7.5, 3.4 Hz, 1H), 1.97 - 1.67 (m, 4H). ¹³C NMR (101 MHz, DMSO) δ 173.1, 139.6, 128.3, 127.1, 126.8, 42.3, 41.9, 25.2.

*tert*-butyl (3-(benzyl(methyl)amino)propyl)carbamate (1b). 0.97 mL (7.56 mmol) *N*-methyl-1-phenylmethanamine, 1.8 g (7.56 mmol) *tert*-butyl (3-bromopropyl)carbamate, 4.93 g (15.12 mmol) cesium carbonate, and 0.627 g (3.78 mmol) potassium iodide were added in a flask with 30.2 mL acetone. The mixture stirred at room temperature for 72 h. After complete reaction, the formed precipitate was filtered off, the filtrate was concentrated and redissolved in DCM. The solution was washed with water (3x 30 mL) and brine (3x 30 mL). The combined organic phased were dried over Na₂SO₄, filtered, and evaporated to dryness. The crude product was purified via flash chromatography (SiO₂; DCM/MeOH 0 - 5 %) obtaining a yellow oil in 53 % yield (1.12 g, 4.08 mmol). TLC-MS (ESI+): calcd. *m*/*z* 278.20 for C₁₆H₂₆N₂O₂. Found 301.2 [M+Na]⁺.

*tert*-butyl (3-((4-methoxybenzyl)(methyl)amino)propyl)carbamate (18b). 0.31 mL (2.1 mmol) 1-(4-methoxyphenyl)-N-methylmethanamine, 0.5 g (2.1 mmol) *tert-*butyl (3-bromopropyl)carbamate, 1.37 g (4.2 mmol) cesium carbonate, and 0.174 g (1.05 mmol) potassium iodide were added in a flask with 8.4 mL acetone. The mixture stirred at room temperature for 72 h. After complete reaction, the formed precipitate was filtered off, the filtrate was concentrated and redissolved in DCM. The solution was washed with water (3x 10 mL) and brine (3x 10 mL). The combined organic phased were dried over Na₂SO₄, filtered, and evaporated to dryness. The crude product was purified via flash chromatography (SiO₂; DCM/MeOH 1 - 3 %) obtaining a yellow oil in 49 % yield (0.315 g, 1.03 mmol). ¹H NMR (400 MHz, CDCl₃) δ 7.25 - 7.15 (m, 2H), 6.90 - 6.79 (m, 2H), 5.58 (s, 1H), 3.79 (s, 3H), 3.41 (s, 2H), 3.18 (q, *J* = 11.6, 5.7 Hz, 2H), 2.43 (t, *J* = 6.3 Hz, 2H), 2.15 (s, 3H), 1.67 (quint, *J* = 6.3 Hz, 2H), 1.44 (s, 9H). ¹³C NMR (101 MHz, CDCl₃) δ 158.82, 156.25, 130.90, 130.29, 130.29, 113.77, 113.77, 78.85, 62.05, 55.97, 55.35, 42.03, 40.18, 28.61, 28.61, 28.61, 26.57. TLC-MS (ESI+): calcd. *m*/*z* 308.21 for C₁₇H₂₈N₂O₃. Found 331.2 [M+Na]⁺.

*tert*-butyl (3-((4-bromobenzyl)(methyl)amino)propyl)carbamate (19b). 0.42 mL (2.1 mmol) 1-(4-bromophenyl)-*N*-methylmethanamine, 0.5 g (2.1 mmol) tert-butyl (3-bromopropyl)carbamate, 1.37 g (4.2 mmol) cesium carbonate, and 0.174 g (1.05 mmol) potassium iodide were added in a flask with 8.4 mL acetone. The mixture stirred at room temperature for 72 h. After complete reaction, the formed precipitate was filtered off, the filtrate was concentrated and redissolved in DCM. The solution was washed with water (3x 10 mL) and brine (3x 10 mL). The combined organic phased were dried over Na₂SO₄, filtered, and evaporated to dryness. The crude product was purified via flash chromatography (SiO₂; PE/EtOAc 10 - 40 %) obtaining a yellow oil in 47 % yield (0.354 g, 0.99 mmol). ¹H NMR (400 MHz, CDCl₃) δ 7.46 - 7.38 (m, 2H), 7.22 - 7.16 (m, *J* = 8.4 Hz, 2H), 5.43 (s, 1H), 3.42 (s, 2H), 3.18 (q, *J* = 11.6, 5.7 Hz, 2H), 2.43 (t, *J* = 6.4 Hz, 2H), 2.15 (s, 3H), 1.67 (quint, *J* = 6.4 Hz, 2H), 1.44 (s, 9H). ¹³C NMR (101 MHz, CDCl₃) δ 156.19, 138.11, 131.52, 131.52, 130.74, 130.74, 120.96, 78.97, 62.05, 56.07, 42.12, 40.00, 28.61, 28.61, 28.61, 26.73. TLC-MS (ESI+): calcd. *m*/*z* 356.11/358.11 for C₁₆H₂₅BrN₂O₂. Found 379.1/381.1 [M+Na]⁺.

*tert*-butyl (3-((4-fluorobenzyl)(methyl)amino)propyl)carbamate (20b). 0.37 g (2.65 mmol) 20c, 0.63 g (2.65 mmol) *tert*-butyl (3-bromopropyl)carbamate, 1.72 g (5.3 mmol) cesium carbonate, and 0.22 g (1.32 mmol) potassium iodide were added in a flask with 10.6 mL acetone. The mixture stirred at room temperature for 72 h. After complete reaction, the formed precipitate was filtered off, the filtrate was concentrated and redissolved in DCM. The solution was washed with water (3x 10 mL) and brine (3x 10 mL). The combined organic phased were dried over Na₂SO₄, filtered, and evaporated to dryness obtaining a yellow oil. The crude product was used without further purification.

*tert-butyl* (3-((2-fluorobenzyl)(methyl)amino)propyl)carbamate (21b). 0.28 mL (2.1 mmol) 1-(2-fluorophenyl)-*N*-methylmethanamine, 0.5 g (2.1 mmol) tert-butyl (3-bromopropyl)carbamate, 1.37 g (4.2 mmol) cesium carbonate, and 0.174 g (1.05 mmol) potassium iodide were added in a flask with 8.4 mL acetone. The mixture stirred at room temperature for 72 h. After complete reaction, the formed precipitate was filtered off, the filtrate was concentrated and redissolved in DCM. The solution was washed with water (3x 10 mL) and brine (3x 10 mL). The combined organic phased were dried over Na₂SO₄, filtered, and evaporated to dryness. The crude product was purified via flash chromatography (SiO₂; PE/EtOAc 10 - 40 %) obtaining a yellow oil in 69 % yield (0.429 g, 1.45 mmol). ¹H NMR (400 MHz, CDCl₃) δ 7.41 - 7.32 (m, *J* = 7.5, 1.5 Hz, 1H), 7.25 - 7.18 (m, *J* = 7.3, 4.7, 2.0 Hz, 1H), 7.14 - 7.07 (m, *J* = 7.5, 1.0 Hz, 1H), 7.05 - 6.98 (m, 1H), 5.39 (s, 1H), 3.55 (s, 2H), 3.18 (q, *J* = 11.8, 5.8 Hz, 2H), 2.47 (t, *J* = 6.4 Hz, 2H), 2.20 (s, 3H), 1.67 (quint, 2H), 1.44 (s, 9H). ¹³C NMR (101 MHz, CDCl₃) δ 161.56, 156.25, 131.51, 128.91, 125.48, 124.05, 115.41, 78.88, 55.96, 54.91, 42.02, 39.98, 28.59, 26.81. TLC-MS (ESI+): calcd. *m*/*z* 296.19 for C₁₆H₂₅FN₂O₂. Found 319.2 [M+Na]⁺.

*tert*-butyl (3-((3-fluorobenzyl)(methyl)amino)propyl)carbamate (22b). 0.29 mL (2.1 mmol) 1-(3-fluorophenyl)-*N*-methylmethanamine, 0.5 g (2.1 mmol) *tert*-butyl (3-bromopropyl)carbamate, 1.37 g (4.2 mmol) cesium carbonate, and 0.174 g (1.05 mmol) potassium iodide were added in a flask with 8.4 mL acetone. The mixture stirred at room temperature for 72 h. After complete reaction, the formed precipitate was filtered off, the filtrate was concentrated and redissolved in DCM. The solution was washed with water (3x 10 mL) and brine (3x 10 mL). The combined organic phased were dried over Na₂SO₄, filtered, and evaporated to dryness. The crude product was purified via flash chromatography (SiO₂; PE/EtOAc 10 - 40 %) obtaining a yellow oil in 56 % yield (0.336 g, 1.18 mmol). ¹H NMR (400 MHz, CDCl₃) δ 7.30 - 7.22 (m, 1H), 7.08 (d, *J* = 7.6 Hz, 1H), 7.06 - 7.01 (m, 1H), 6.97 - 6.89 (m, *J* = 8.3, 2.0 Hz, 1H), 5.45 (s, 1H), 3.46 (s, 2H), 3.20 (q, *J* = 11.8, 5.8 Hz, 2H), 2.45 (t, *J* = 6.3 Hz, 2H), 2.17 (s, 3H), 1.67 (quint, 2H), 1.44 (s, 9H). ¹³C NMR (101 MHz, CDCl₃) δ 163.01, 156.23, 141.80, 129.84, 124.54, 115.76, 114.08, 79.01, 62.23, 56.19, 42.16, 40.04, 28.57, 26.74. TLC-MS (ESI+): calcd. *m*/*z* 296.19 for C₁₆H₂₅FN₂O₂. Found 319.2 [M+Na]⁺.

*tert*-butyl (3-(benzyl(ethyl)amino)propyl)carbamate (23b). 0.31 mL (2.1 mmol) *N*-benzylethanamine, 0.5 g (2.1 mmol) *tert*-butyl (3-bromopropyl)carbamate, 1.37 g (4.2 mmol) cesium carbonate, and 0.174 g (1.05 mmol) potassium iodide were added in a flask with 8.4 mL acetone. The mixture stirred at room temperature for 72 h. After complete reaction, the formed precipitate was filtered off, the filtrate was concentrated and redissolved in DCM. The solution was washed with water (3x 10 mL) and brine (3x 10 mL). The combined organic phased were dried over Na₂SO₄, filtered, and evaporated to dryness. The crude product was purified via flash chromatography (SiO₂; DCM/MeOH 2 - 7 %) obtaining a yellow oil in 46 % yield (0.282 g, 0.97 mmol). TLC-MS (ESI+): calcd. *m*/*z* 292.22 for C₁₇H₂₈N₂O₂. Found 315.2 [M+Na]⁺.

*tert*-butyl (3-(benzyl(isopropyl)amino)propyl)carbamate (24b). 0.35 mL (2.1 mmol) *N*-benzylpropan-2-amine, 0.5 g (2.1 mmol) *tert*-butyl (3-bromopropyl)carbamate, 1.37 g (4.2 mmol) cesium carbonate, and 0.35 g (2.1 mmol) potassium iodide were added in a flask with 8.4 mL acetone. The mixture was stirred at rt for 72 h. After complete reaction, the mixture was diluted with EtOAc, washed with water (3x 10 mL) and brine (3x 10 mL). The combined organic phased were dried over Na₂SO₄, filtered, and evaporated to dryness. The mixture was purified via flash chromatography (SiO₂; PE/EtOAc 0 - 30 %). The crude product was used without further purification. TLC-MS (ESI+): calcd. m/z 306.23 for C₁₈H₃₀N₂O₂. Found 329.2 [M+Na]⁺.

*tert*-butyl (3-(benzyl(cyclopropyl)amino)propyl)carbamate (25b). 0.31 mL (2.1 mmol) *N*-benzylcyclopropanamine, 0.5 g (2.1 mmol) tert-butyl (3-bromopropyl)carbamate, 1.37 g (4.2 mmol) cesium carbonate, and 0.35 g (2.1 mmol) potassium iodide were added in a flask with 8.4 mL acetone. The mixture was stirred at rt for 72 h. After complete reaction, the mixture was diluted with EtOAc, washed with water (3x 10 mL) and brine (3x 10 mL). The combined organic phased were dried over Na₂SO₄, filtered, and evaporated to dryness. The mixture was purified via flash chromatography (SiO₂; DCM/MeOH 0 - 3 %). The crude product was used without further purification. TLC-MS (ESI+): calcd. m/z 304.22 for C₁₈H₂₈N₂O₂. Found 327.2 [M+Na]⁺.

*tert*-butyl (3-(benzyl(propyl)amino)propyl)carbamate (26b). 0.35 mL (2.1 mmol) *N*-benzylpropan-1-amine, 0.5 g (2.1 mmol) *tert*-butyl (3-bromopropyl)carbamate, 1.37 g (2.1 mmol) cesium carbonate, and 0.174 g (1.05 mmol) potassium iodide were added in a flask with 8.4 mL acetone. The mixture was stirred at rt for 72 h. After complete reaction, the mixture was diluted with EtOAc, washed with water (3x 10 mL) and brine (3x 10 mL). The combined organic phased were dried over Na₂SO₄, filtered, and evaporated to dryness obtaining a yellow oil. The crude product was used without further purification. TLC-MS (ESI+): calcd. *m*/*z* 306.23 for C₁₈H₃₀N₂O₂. Found 307.2 [M+H]⁺.

*tert*-butyl (3-(benzyl(butyl)amino)propyl)carbamate (27b). 0.38 mL (2.1 mmol) *N*-benzylbutan-1-amine, 0.5 g (2.1 mmol) tert-butyl (3-bromopropyl)carbamate, 1.37 g (4.2 mmol) cesium carbonate, and 0.35 g (2.1 mmol) potassium iodide were added in a flask with 8.4 mL acetone. The mixture was stirred at rt for 72h. After complete reaction, the mixture was diluted with EtOAc, washed with water (3x 10 mL) and brine (3x 10 mL). The combined organic phased were dried over Na₂SO₄, filtered, and evaporated to dryness. The mixture was purified via flash chromatography (SiO₂; DCM/MeOH 0 - 3 %). The crude product was used without further purification. TLC-MS (ESI+): calcd. m/z 320.25 for C₁₉H₃₂N₂O₂. Found 343.2 [M+Na]⁺.

*tert*-butyl (3-(benzyl(3-hydroxypropyl)amino)propyl)carbamate (28b). 0.32 mL (2 mmol) 3-(benzylamino)propan-1-ol, 0.478 mg (2 mmol) *tert*-butyl (3-bromopropyl)carbamate, 1.3 g (4 mmol) cesium carbonate, and 0.333 g (2 mmol) potassium iodide were added in a flask with 8 mL acetone. The mixture was stirred at rt for 16 h. After complete reaction, the mixture was diluted with EtOAc, washed with water (3x 10 mL) and brine (3x 10 mL). The combined organic phased were dried over Na₂SO₄, filtered, and evaporated to dryness. The crude product was purified via flash chromatography (SiO₂; DCM/MeOH + 1 % NH₃ 0 - 5 %) obtaining a colorless oil in 60 % yield (0.388 g, 1.2 mmol). ¹H NMR (400 MHz, CDCl₃) δ 7.36 - 7.22 (m, 5H), 3.72 (t, 2H), 3.54 (s, 2H), 3.09 (q, *J* = 6.6 Hz, 2H), 2.63 (t, *J* = 5.2 Hz, 2H), 2.46 (t, *J* = 7.0 Hz, 2H), 1.77 - 1.64 (m, 4H), 1.46-1.40 (m, 11H). ¹³C NMR (101 MHz, CDCl₃) δ 156.12, 138.44, 129.29, 128.61, 127.44, 63.58, 59.03, 53.60, 51.45, 29.40, 28.55, 28.50, 28.33, 27.16. TLC-MS (ESI+): calcd. *m*/*z* 322.23 for C₁₈H₃₀N₂O₃. Found 345.0 [M+Na]⁺. HPLC *t*_{R} = 3.595 min.

*tert*-butyl (3-(benzyl(phenethyl)amino)propyl)carbamate (29b). 0.2 mg (0.94 mmol) 29c, 0.225 mg (0.94 mmol) *tert*-butyl (3-bromopropyl)carbamate, 0.616 g (1.89 mmol) cesium carbonate, and 0.157 g (0.94 mmol) potassium iodide were added in a flask with 4 mL acetone. The mixture was stirred at rt for 16 h. After complete reaction, the mixture was diluted with EtOAc, washed with water (3x 10 mL) and brine (3x 10 mL). The combined organic phased were dried over Na₂SO₄, filtered, and evaporated to dryness. The crude product was purified via flash chromatography (SiO₂; DCM/MeOH 0 - 4 %) obtaining a yellow oil in 51 % yield (0.178 g, 0.48 mmol). ¹H NMR (400 MHz, CDCl₃) δ 7.34 - 7.00 (m, 11H), 3.56 (s, 2H), 2.81 - 2.56 (m, 4H), 2.49 (t, *J* = 6.4 Hz, 2H), 1.65 - 1.51 (m, 2H), 1.46 - 1.36 (m, 11H). ¹³C NMR (101 MHz, CDCl₃) δ 155.99, 140.44, 139.35, 128.88, 128.70, 128.31, 128.27, 126.98, 125.93, 58.81, 55.49, 52.14, 39.60, 33.39, 28.49, 28.44, 26.69. TLC-MS (ESI+): calcd. m/z 368.25 for C₂₃H₃₂N₂O₂. Found 369.1 [M+H]⁺. HPLC *t*_{R} = 5.924 min.

*tert*-butyl (3-(benzyl(3-phenylpropyl)amino)propyl)carbamate (30b). 0.224 mg (1 mmol) 30c, 0.237 mg (1 mmol) tert-butyl (3-bromopropyl)carbamate, 0.649 g (2 mmol) cesium carbonate, and 0.165 g (1 mmol) potassium iodide were added in a flask with 4 mL acetone. The mixture was stirred at rt for 16 h. After complete reaction, the mixture was diluted with EtOAc, washed with water (3x 10 mL) and brine (3x 10 mL). The combined organic phased were dried over Na₂SO₄, filtered, and evaporated to dryness. The crude product was purified via flash chromatography (SiO₂; DCM/MeOH 0 - 4 %) obtaining a yellow oil in 48 % yield (0.184 g, 0.48 mmol). ¹H NMR (400 MHz, CDCl₃) δ 7.36 - 7.30 (m, 4H), 7.28 - 7.12 (m, 7H), 3.54 (s, 2H), 3.16 (q, *J* = 6.1 Hz, 2H), 2.60 (t, *J* = 6.8 Hz, 2H), 2.53 - 2.40 (m, 4H), 1.86 - 1.78 (m, 2H), 1.63 (quint, *J* = 6.3 Hz, 2H), 1.43 (s, 9H). ¹³C NMR (101 MHz, CDCl₃) δ 156.15, 142.41, 139.58, 129.13, 128.72, 128.49, 128.43, 127.11, 125.84, 78.82, 59.02, 53.55, 52.69, 40.03, 33.77, 28.94, 28.61, 28.56, 26.63. TLC-MS (ESI+): calcd. *m*/*z* 382.26 for C₂₄H₃₄N₂O₂. Found 383.1 [M+H]⁺. HPLC *t*_{R} = 6.308 min.

*N*¹-ethyl-*N*¹-(4-fluorobenzyl)propane-1,3-diamine dihydrochloride (32b). 0.731 g (2.35 mmol) 32c was dissolved in 11.8 mL (47.1 mmol) 4M HCl in 1,4-dioxane. The mixture was stirred at room temperature for 1 h. The formed product was filtered off by suction filtration and dried under reduced pressure obtaining a white semi-solid in 55 % yield (0.368 g, 1.3 mmol). The product was used without further purification. TLC-MS (ESI+): calcd. *m*/*z* 210.15 for C₁₂H₁₉FN₂. Found 211.2 [M+H]⁺.

*tert*-butyl (3-(3,4-dihydroisoquinolin-2(1*H*)-yl)propyl)carbamate (35b). 0.26 mL (2.1 mmol) 1,2,3,4-tetrahydroisoquinoline, 0.5 g (2.1 mmol) *tert*-butyl (3-bromopropyl)carbamate, 1.37 g (4.2 mmol) cesium carbonate, and 0.35 g (2.1 mmol) potassium iodide were added in a flask with 8.4 mL acetone. The mixture was stirred at rt for 72 h. After complete reaction, the mixture was diluted with EtOAc, washed with water (3x 10 mL) and brine (3x 10 mL). The combined organic phased were dried over Na₂SO₄, filtered, and evaporated to dryness. The mixture was purified via flash chromatography (SiO₂; DCM/MeOH 0 - 3 %). The crude product was used without further purification. TLC-MS (ESI+): calcd. *m*/*z* 290.2 for C₁₇H₂₆N₂O₂. Found 313.2 [M+Na]⁺.

*tert*-butyl (3-((3-(4-fluorobenzamido)benzyl)(methyl)amino)propyl)carbamate (36b). 0.4 g (1.65 mmol) 36c was dissolved in 15 mL THF and one drop of acetic acid was added. After 15 min of continuous stirring, 0.3 mL (1.5 mmol) *tert*-butyl (3-(me-thylamino)propyl)carbamate was added and the mixture was stirred for 1 h. The mixture was cooled to 0 °C and 0.476 g (2.25 mmol) sodium triacetoxyborohydride was added. After complete reaction, the mixture was diluted with EtOAc, washed with water (3x 10 mL) and brine (3x 10 mL). The combined organic phased were dried over Na₂SO₄, filtered, and evaporated to dryness. The crude product was used without further purification. TLC-MS (ESI+): calcd. *m*/*z* 415.23 for C₂₃H₃₀FN₃O₃. Found 438.2 [M+Na]⁺.

*tert*-butyl (3-(4-(*tert*-butylcarbamoyl)piperidin-1-yl)propyl)carbamate (37b). 0.3 g (1.36 mmol) 37c, 0.324 g (1.36 mmol) *tert*-butyl (3-bromopropyl)carbamate, 1.328 g (4.08 mmol) cesium carbonate, and 0.113 g (0.68 mmol) potassium iodide were added in a flask with 5.44 mL acetone. The mixture was stirred for 16 h at rt. After complete reaction, the mixture was diluted with EtOAc, washed with water (3x 10 mL) and brine (3x 10 mL). The combined organic phased were dried over Na₂SO₄, filtered, and evaporated to dryness obtaining a yellow semi-solid in 80 % yield (0.371 g, 1.09 mmol). ¹H NMR (400 MHz, CDCl₃) δ 5.40 (s, 1H), 5.24 (s, 1H), 3.22 - 3.11 (m, 2H), 3.02 - 2.88 (m, *J* = 11.7 Hz, 2H), 2.37 (t, *J* = 6.7 Hz, 2H), 1.98 - 1.77 (m, 5H), 1.73 - 1.57 (m, 4H), 1.43 (s, 9H), 1.32 (s, 9H). ¹³C NMR (101 MHz, CDCl₃) δ 174.3, 156.2, 57.1, 53.4, 51.1, 44.3, 40.1, 29.3, 29.0, 28.6, 28.5, 26.7. TLC-MS (ESI+): calcd. *m*/*z* 341.27 for C₁₈H₃₅N₃O₃. Found 342.3 [M+H]⁺.

*tert*-butyl ((1-benzylpiperidin-3-yl)methyl)carbamate (39b). 0.16 mL (1.54 mmol) benzaldehyde was dissolved in 14 mL THF, and one drop of acetic acid was added. After 15 min of continuous stirring, 0.3 g (1.4 mmol) *tert*-butyl (piperidin-3-ylme-thyl)carbamate was added, and the mixture was stirred for 1 h. The mixture was cooled to 0 °C and 0.445 g (2.1 mmol) sodium triacetoxyborohydride was added. After complete reaction, the mixture was diluted with EtOAc, washed with water (3x 10 mL) and brine (3x 10 mL). The combined organic phased were dried over Na₂SO₄, filtered, and evaporated to dryness. The crude product was purified via flash chromatography (SiO₂; DCM/MeOH 0 - 7 %) obtaining a yellow oil in 39 % yield (0.165 g, 0.54 mmol). ¹H NMR (400 MHz, CDCl₃) δ 7.54 - 7.27 (m, 5H), 4.66 (s, 1H), 3.65 (s, 2H), 3.33 - 2.74 (m, 5H), 2.24 - 1.58 (m, 6H), 1.42 (s, 9H). TLC-MS (ESI+): calcd. *m*/*z* 304.22 for C₁₈H₂₈N₂O₂. Found 327.1 [M+Na]⁺.

*tert*-butyl 4-(3-(4-fluorobenzamido)benzyl)-1,4-diazepane-1-carboxylate (41b). 0.37 g (1.52 mmol) 36c was dissolved in 14 mL THF and one drop of acetic acid was added. After 15 min of continuous stirring, 0.27 mL (1.38 mmol) *tert*-butyl 1,4-diaze-pane-1-carboxylate was added, and the mixture was stirred for 1 h. The mixture was cooled to 0 °C and 0.44 g (2.07 mmol) sodium triacetoxyborohydride was added. After complete reaction, the mixture was diluted with EtOAc, washed with water (3x 10 mL) and brine (3x 10 mL). The combined organic phased were dried over Na₂SO₄, filtered, and evaporated to dryness. The crude product was purified via flash chromatography (SiO₂; DCM/MeOH 1 - 3 %) obtaining a yellow oil in 92 % yield (0.544 g, 1.27 mmol). ¹H NMR (400 MHz, CDCl₃) δ 8.30 (d, *J* = 61.0 Hz, 1H), 8.01 - 7.88 (m, 2H), 7.85 - 7.65 (m, 2H), 7.33 (t, *J* = 7.8 Hz, 1H), 7.14 (t, *J* = 8.5 Hz, 3H), 3.80 (d, *J* = 9.5 Hz, 2H), 3.67 - 3.52 (m, *J* = 20.1 Hz, 2H), 3.53 - 3.41 (m, 2H), 2.92 - 2.71 (m, *J* = 16.0 Hz, 4H), 2.02 - 1.93 (m, *J* = 10.7, 5.5 Hz, 2H), 1.45 (s, 9H). ¹³C NMR (101 MHz, CDCl₃) δ 166.32, 165.01, 163.81, 155.81, 155.46, 138.68, 131.08, 129.78, 129.46, 125.68, 121.36, 120.26, 115.99, 115.77, 80.00, 61.91, 56.13, 54.85, 54.13, 45.58, 44.87, 28.60. TLC-MS (ESI+): calcd. *m*/*z* 427.23 for C₂₂H₃₂N₄O₂S. Found 450.2 [M+Na]⁺. HPLC *t*_{R} = 5.309 min.

2-(3-nitrobenzyl)isoindoline-1,3-dione (42b). 1 g (4.63 mmol) 1-(bromomethyl)-3-nitrobenzene was dissolved in 14 mL DMF, and 0.943 g (5.09 mmol) phthalimide potassium salt was added in small portions. The reaction mixture was stirred for 2h at room temperature. Water was added to the mixture and the resulting suspension was filtered. The solid was washed with water multiple times and dried at 50°C under reduced pressure obtaining a brown solid in 92 % yield (1.202 g, 4.26 mmol). ¹H NMR (400 MHz, DMSO) δ 8.22 - 8.17 (m, 1H), 8.16 - 8.10 (m, 1H), 7.96 - 7.83 (m, 4H), 7.79 (d, *J* = 7.7 Hz, 1H), 7.63 (t, *J* = 7.9 Hz, 1H), 4.92 (s, 2H). ¹³C NMR (101 MHz, DMSO) δ 167.70, 147.88, 138.83, 134.60, 134.19, 131.61, 130.17, 123.31, 122.45, 122.34, 40.23. TLC-MS (ESI+): calcd. *m*/*z* 282.06 for C₁₅H₁₀N₂O₄. Found 305.1 [M+Na]⁺. HPLC *t*_{R} = 7.573 min.

*tert*-butyl 4-(3-(1,3-dioxoisoindolin-2-yl)propyl)piperazine-1-carboxylate (46b). 3 g (11.2 mmol) *N*-(3-Bromopropyl)phthalimide and 2.08 g (11.2 mmol) 1-Boc-piperazine were dissolved in 45 mL DMF. 2.3 mL (13.4 mmol) DIPEA was added, and the mixture was heated to 100 °C for 72 h. After complete reaction, the mixture was diluted with EtOAc, washed with water (3x 30 mL) and brine (3x 30 mL). The combined organic phased were dried over Na₂SO₄, filtered, and evaporated to dryness obtaining a brown solid. The crude product was used without further purification. TLC-MS (ESI+): calcd. m/z 373.20 for C₂₀H₂₇N₃O₄. Found 374.2 [M+H]⁺.

*tert*-butyl 4-(benzylcarbamoyl)piperidine-1-carboxylate (48b). 0.314 g (1.37 mmol) 37e was dissolved in 4 mL DCM and 0.573 g (1.51 mmol) HATU was slowly added. After 15 min of continuous stirring, 0.45 mL (4.11 mmol) phenylmethanamine and 0.21 mL (1.51 mmol) triethylamine was added to the mixture. The mixture was allowed to stir at room temperature overnight. After complete reaction, the mixture was diluted with DCM, washed with water (3x 10 mL) and brine (3x 10 mL). The combined organic phased were dried over Na₂SO₄, filtered, and evaporated to dryness obtaining a brown solid. TLC-MS (ESI+): calcd. *m*/*z* 318.19 for C₁₈H₂₆N₂O₃. Found 341.2 [M+Na]⁺.

1-(4-fluorophenyl)-*N*-methylmethanamine (20c). 1.32 mL (2.65 mmol) 2M methanamine in THF, 0.33 mL (2.65 mmol) 1-(bromomethyl)-4-fluorobenzene, 1.72 g (5.29 mmol) cesium carbonate, and 0.22 g (1.32 mmol) potassium iodide were added in a flask with 11 mL acetone. The mixture stirred at room temperature for 72 h. After complete reaction, the formed precipitate was filtered off, the filtrate was concentrated and redissolved in DCM. The solution was washed with water (3x 10 mL) and brine (3x 10 mL). The combined organic phased were dried over Na₂SO₄, filtered, and evaporated to dryness obtaining a yellow oil. The crude product was used without further purification.

*N*-benzyl-2-phenylethan-1-amine (29c). 0.25 mL (2.48 mmol) benzaldehyde was dissolved in 12 mL EtOH. After 15 min of continuous stirring, 0.31 mL (2.48 mmol) 2-phenylethan-1-amine was added, and the mixture was stirred for 1 h. The mixture was cooled to 0 °C and 0.311 g (4.95 mmol) sodium cyanoborohydride was added. After complete reaction, the solvent was removed under reduced pressure, and the mixture was diluted with water. The mixture was extracted with DCM (3x 10 mL), washed with water (3x 10 mL), and brine (3x 10 mL). The combined organic phased were dried over Na₂SO₄, filtered, and evaporated to dryness. The crude product was purified via flash chromatography (SiO₂; DCM/MeOH + 1% NH₃ 0 - 4 %) obtaining a yellow oil in 38 % yield (0.2 g, 0.94 mmol). ¹H NMR (400 MHz, CDCl₃) δ 7.33 - 7.13 (m, 11H), 3.76 (s, 2H), 2.91 - 2.83 (m, 2H), 2.83 - 2.75 (m, 2H). ¹³C NMR (101 MHz, CDCl₃) δ 140.40, 140.16, 128.85, 128.58, 128.51, 128.20, 127.03, 126.27, 53.99, 50.67, 36.49. TLC-MS (ESI+): calcd. *m*/*z* 211.14 for C₁₅H₁₇N. Found 212.0 [M+H]⁺. HPLC *t*_{R} = 3.352 min.

*N*-benzyl-3-phenylpropan-1-amine (30c). 0.23 mL (2.22 mmol) benzaldehyde was dissolved in 11 mL EtOH. After 15 min of continuous stirring, 0.32 mL (2.22 mmol) 3-phenylpropan-1-amine was added, and the mixture was stirred for 1 h. The mixture was cooled to 0 °C and 0.279 g (4.44 mmol) sodium cyanoborohydride was added. After complete reaction, the solvent was removed under reduced pressure, and the mixture was diluted with water. The mixture was extracted with DCM (3x 10 mL), washed with water (3x 10 mL), and brine (3x 10 mL). The combined organic phased were dried over Na₂SO₄, filtered, and evaporated to dryness. The crude product was purified via flash chromatography (SiO₂; DCM/MeOH + 1% NH₃ 0 - 5 %) obtaining a yellow oil in 33 % yield (0.166 g, 0.73 mmol). ¹H NMR (400 MHz, CDCl₃) δ 7.32 - 7.04 (m, 10H), 3.72 (s, 2H), 2.71 - 2.56 (m, 4H), 1.86 - 1.66 (m, 2H). ¹³C NMR (101 MHz, CDCl₃) δ 142.31, 140.61, 128.52, 128.45, 128.26, 127.03, 125.88, 54.15, 49.06, 33.77, 31.86. TLC-MS (ESI+): calcd. *m*/*z* 225.15 for C₁₆H₁₉N. Found 225.9 [M+H]⁺. HPLC *t*_{R} = 4.126 min.

*tert*-butyl (3-(ethyl(4-fluorobenzyl)amino)propyl)carbamate (32c). 0.48 mL (3.15 mmol) *N*-(4-fluorobenzyl)ethanamine, 0.75 g (3.15 mmol) *tert*-butyl (3-bromopropyl)carbamate, 2.05 g (6.3 mmol) cesium carbonate, and 0.26 g (1.57 mmol) potassium iodide were added in a flask with 12.6 mL acetone. The mixture was stirred at rt for 72 h. After complete reaction, the mixture was diluted with EtOAc, washed with water (3x 10 mL) and brine (3x 10 mL). The combined organic phased were dried over Na₂SO₄, filtered, and evaporated to dryness. The crude product was purified via flash chromatography (SiO₂; PE/EtOAc 10 - 30%) obtaining a yellow oil in 39 % yield (0.377 g, 1.21 mmol). ¹H NMR (400 MHz, CDCl₃) δ 7.32 - 7.25 (m, *J* = 8.5, 5.7 Hz, 2H), 7.03 - 6.95 (m, 2H), 5.47 (s, 1H), 3.49 (s, *J* = 14.4 Hz, 2H), 3.15 (q, *J* = 11.6, 5.7 Hz, 2H), 2.51 - 2.39 (m, 4H), 1.61 (quint, *J* = 12.6, 6.4 Hz, 2H), 1.43 (s, 9H), 1.03 (t, 3H). TLC-MS (ESI+): calcd. *m*/*z* 310.41 for C₁₇H₂₇FN₂O₂. Found 311.2 [M+H]⁺.

4-fluoro-*N*-(3-formylphenyl)benzamide (36c). 10% Hydrochloric acid (16 mL) was added dropwise to a stirring solution of 1.4 g (4.91 mmol) 36d in 27 mL acetone. The mixture was stirred for 30 minutes at room temperature. The solution was cooled to 4°C and stirred overnight. After complete reaction, the mixture was treated with NaHCO₃ solution and acetone was evaporated under reduced pressure. The remaining solid was filtered by suction filtration and washed multiple times with water. The solid product was dried under reduced pressure obtaining a white solid in 68 % yield (0.812 g, 3.34 mmol). ¹H NMR (400 MHz, CDCl₃) δ 9.98 (s, 1H), 8.29 (s, 1H), 8.12 (d, *J* = 1.6 Hz, 1H), 8.03 (td, *J* = 8.2, 2.3 Hz, 1H), 7.96 - 7.88 (m, 2H), 7.64 (t, *J* = 8.8 Hz, 1H), 7.56 - 7.48 (m, 1H), 7.19 -7.11 (m, 2H). ¹³C NMR (101 MHz, CDCl₃) δ 192.2, 166.5, 165.1, 164.0, 139.0, 137.2, 130.0, 129.8, 129.7, 126.3, 126.2, 120.9, 116.2, 116.0. TLC-MS (ESI+): calcd. *m*/*z* 243.07 for C₁₄H₁₀FNO₂. Found 266.2 [M+Na]⁺.

*N*-(*tert*-butyl)piperidine-4-carboxamide hydrochloride (37c). 1.47 g (5.17 mmol) 37d was dissolved in 26 mL (104 mmol) 4M HCl in 1,4-dioxane. The mixture was stirred at room temperature for 3 h. The formed product was filtered off by suction filtration and dried under reduced pressure obtaining a white solid in 89 % yield (1.015 g, 4.6 mmol). The product was used without further purification.

*N*-(3-(1,3-dioxolan-2-yl)phenyl)-4-fluorobenzamide (36d). 0.9 g (6.42 mmol) 4-fluorobenzoic acid was dissolved in 26 mL dry DMF and 2.69 g (7.07 mmol) HATU was slowly added. After 15 min of continuous stirring, 1.06 g (6.42 mmol) 3-(1,3-dioxolan-2-yl)aniline and 2.8 mL (16.06 mmol) DIPEA was added to the mixture. The mixture was allowed to stir at room temperature overnight. After complete reaction, the mixture was diluted with DCM, washed with water (3x 20 mL) and brine (3x 20 mL). The combined organic phased were dried over Na₂SO₄, filtered, and evaporated to dryness. The crude product was purified via flash chromatography (SiO₂; PE/EtOAc 0 - 30 %) obtaining a white solid in 76 % yield (1.4 g, 4.88 mmol). ¹H NMR (400 MHz, DMSO) δ 10.32 (s, 1H), 8.08 - 8.02 (m, 2H), 7.87 (t, *J* = 1.7 Hz, 1H), 7.81 - 7.77 (m, *J* = 12.2, 4.0, 3.0 Hz, 1H), 7.39 - 7.34 (m, *J* = 12.4, 5.2 Hz, 3H), 7.17 (d, *J* = 7.6 Hz, 1H), 5.73 (s, 1H), 4.09 - 3.91 (m, 4H). ¹³C NMR (101 MHz, DMSO) δ 164.5, 139.1, 138.7, 130.5, 130.4, 128.5, 122.0, 121.0, 118.5, 115.5, 115.3, 102.7, 64.8. TLC-MS (ESI+): calcd. *m*/*z* 287.10 for C₁₆H₁₄FNO₃. Found 310.1 [M+Na]⁺.

*tert*-butyl 4-(tert-butylcarbamoyl)piperidine-1-carboxylate (37d). 1.23 g (5.36 mmol) 37e was dissolved in 16 mL DCM and 2.24 g (5.9 mmol) HATU was slowly added. After 15 min of continuous stirring, 1.7 mL (16.1 mmol) tert-Butylamine and 0.82 mL (5.9 mmol) triethylamine was added to the mixture. The mixture was allowed to stir at room temperature overnight. After complete reaction, the mixture was diluted with DCM, washed with water (3x 20 mL) and brine (3x 20 mL). The combined organic phased were dried over Na₂SO₄, filtered, and evaporated to dryness obtaining a white solid in 97 % yield (1.48 g, 5.2 mmol). ¹H NMR (400 MHz, DMSO) δ 7.35 (s, 1H), 3.93 (d, *J* = 12.1 Hz, 2H), 2.77 - 2.61 (m, *J* = 9.2 Hz, 2H), 2.23 (tt, *J* = 11.5, 3.6 Hz, 1H), 1.62 - 1.53 (m, *J* = 12.8, 2.2 Hz, 2H), 1.39 (s, 9H), 1.37 - 1.30 (m, 2H), 1.22 (s, 9H). ¹³C NMR (101 MHz, DMSO) δ 173.6, 153.8, 78.5, 49.7, 42.1, 38.2, 28.5, 28.3, 28.1. TLC-MS (ESI+): calcd. *m*/*z* 284.21 for C₁₅H₂₈N₂O₃. Found 307.2 [M+Na]⁺.

1-(*tert*-butoxycarbonyl)piperidine-4-carboxylic acid (37e). 1 g (3.9 mmol) 1-(*tert*-butyl) 4-ethyl piperidine-1,4-dicarboxylate was stirred with 29 mL (29.15 mmol) 1M aqueous NaOH solution at room temperature for 16 h. After complete reaction, the mixture was acidified, and the precipitate was collected by suction filtration obtaining a white solid in 82 % yield (0.731 g, 3.19 mmol). ¹H NMR (400 MHz, DMSO) δ 12.25 (s, 1H), 3.83 (d, *J* = 13.1 Hz, 2H), 2.81 (s, 2H), 2.39 (tt, *J* = 11.0, 3.8 Hz, 1H), 1.77 (dd, *J* = 13.3, 3.2 Hz, 2H), 1.44 - 1.31 (m, 11H). ¹³C NMR (101 MHz, DMSO) δ 175.7, 153.9, 78.6, 42.7, 40.1, 28.1, 27.8. TLC-MS (ESI-): calcd. *m*/*z* 229.13 for C₁₁H₁₉NO₄. Found 228.1 [M-H]⁻.

## Claims

1. A compound of any of the general formulas (Ia) to (Ig): 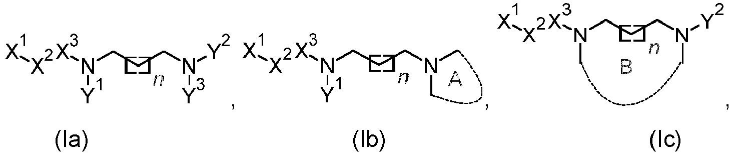 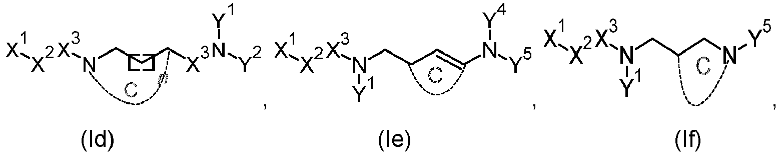 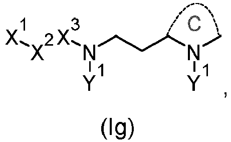 or a stereoisomer, enantiomer, tautomer, pro-drug and/or a pharmaceutically acceptable salt thereof,
wherein
X¹ is selected from substituted or unsubstituted cyclyl, substituted or unsubstituted heterocyclyl, substituted or unsubstituted fused cyclyl, and substituted or unsubstituted fused heterocyclyl,
X² is selected from -SCH₂-, -CH₂CH₂-, and a bond,
X³ is selected from carbonyl, thiocarbonyl, and methylene,
Y¹ is selected from H and methyl,
Y² is selected from substituted or unsubstituted phenyl, substituted or unsubstituted benzyl, *tert*-butyl and *tert*-butyloxycarbonyl,
Y³ is selected from H, substituted or unsubstituted C₁-C₅ alkyl, and C₃-C₅ cycloalkyl,
Y⁴ and Y⁵ are O, or Y⁴ is selected from H, and methyl, Y⁵ is selected from substituted or unsubstituted benzyl, substituted or unsubstituted benzoyl, and linear or branched C₁-C₅ alkylcarboxylate,
A and B are independently selected from substituted or unsubstituted heterocyclyl, and substituted or unsubstituted fused heterocyclyl,
C is selected from substituted or unsubstituted cyclyl, and
n is 0-2.

2. The compound of claim 1, wherein the compound has the formula (Ia) or (Ib), wherein
X¹ is substituted or unsubstituted heterocyclyl, preferably substituted or unsubstituted thiophen-2-yl, more preferably, (substituted or unsubstituted phenyl)thiophen-2-yl,
X² is a bond,
X³ is carbonyl,
Y¹ is H
Y² is unsubstituted benzyl,
Y³ is selected from linear or branched C₁-C₄ alkyl, C₂-C₃ alkyl phenyl and C₃-C₄ cycloalkyl, preferably methyl, and
A is selected from substituted or unsubstituted 1-piperidinyl, substituted or unsubstituted 1-(2,3,4-triyhydro)chinoline, and substituted or unsubstituted 1-(2,3-dihydro)indole, and
n is 1.

3. The compound according to any of the preceding claims, wherein the compound has any of the general formulas (IIa) to (IIc): 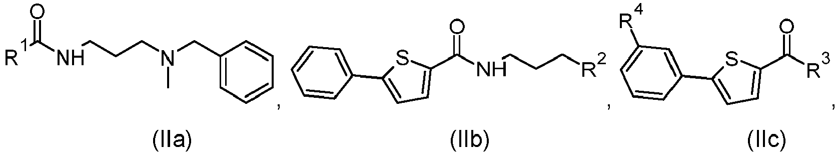 wherein
R¹ is 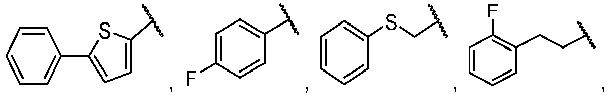 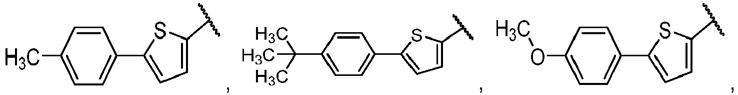 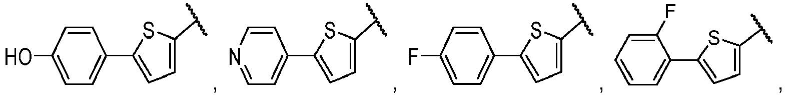 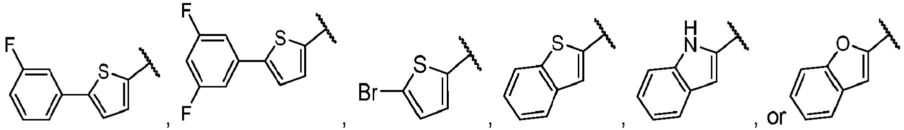
R² is 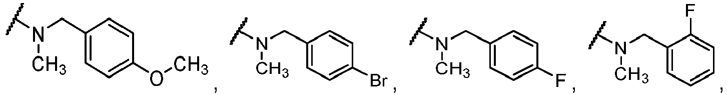 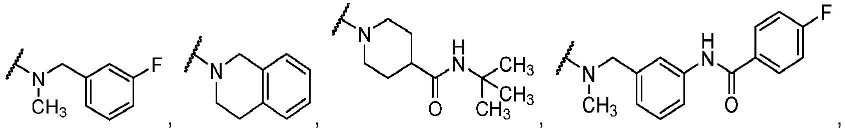 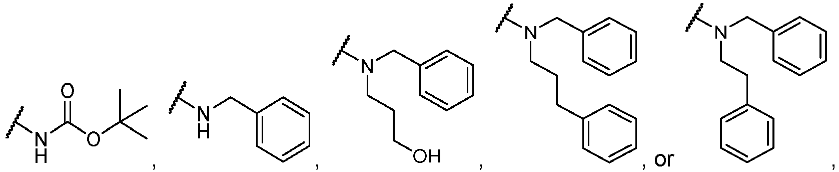
R³ is 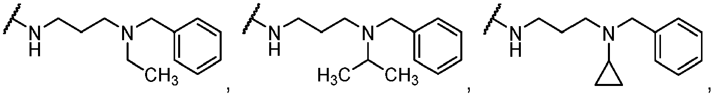 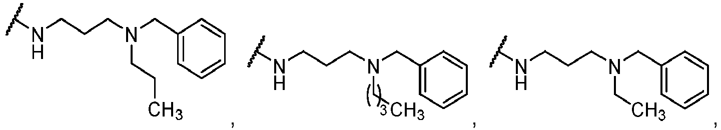 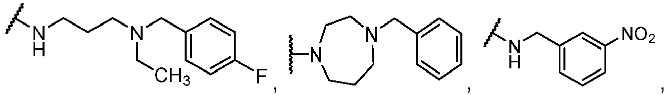 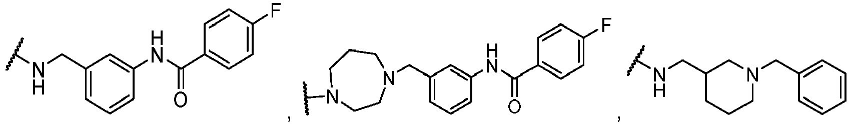 or 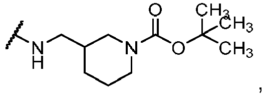 and
R⁴ is halogen, preferably F.

4. The compound according to any of the preceding claims, wherein the compound has the general formula (Ild) or (Ile): 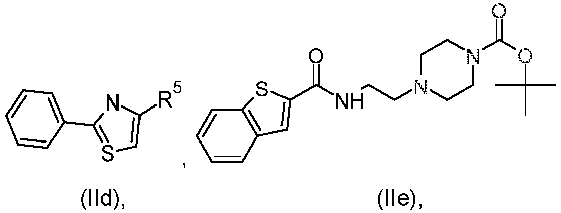 wherein
R⁵ is

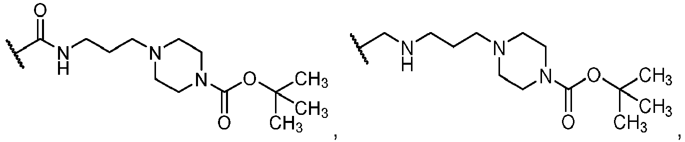

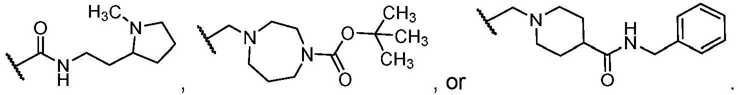

5. The compound according to any of the preceding claims, wherein the compound has a β-Arrestin recruitment EC₅₀ of 0.5 µM or less, preferably 0.1 µM or less, and/or a P-Selectin expression reduction of 71% or more, preferably 90% or more.

6. A pharmaceutical composition comprising the compound of any one of claims 1 to 5 or a pharmaceutically acceptable salt, pro-drug, biologically active metabolite, solvate or stereoisomer thereof.

7. The pharmaceutical composition according to claim 6, further comprising an inert carrier and/or one or more other therapeutic agents.

8. A compound of any one of claims 1 to 5 or a pharmaceutically acceptable salt, pro-drug, biologically active metabolite, solvate or stereoisomer thereof, or the composition of claim 6 or 7, for use in the treatment of a disease.

9. The compound or the composition of claim 8, wherein the disease is mediated by ACKR3.

10. The compound or the composition of claim 8 or 9, wherein the disease is a cardiovascular disease or a platelet disorder.

11. The compound or the composition of claim 10, wherein the cardiovascular disease is selected from coronary artery diseases, such as angina pectoris and heart attack, stroke, heart failure, hypertensive heart disease, rheumatic heart disease, cardiomyopathy, abnormal heart rhythms, congenital heart disease, valvular heart disease, carditis, aortic aneurysms, peripheral artery disease, thromboembolic disease, venous thrombosis, and atherosclerosis and wherein the platelet disorder is selected from thrombocytosis, thrombocythemia, and thrombocytopenia, such as immune thrombocytopenia and thrombotic thrombocytopenic purpura, Bernard Soulier disease, Glanzmann's thrombasthenia, Hermansky Pudlak syndrome, Jacobsen syndrome, Lowe syndrome, platelet release and storage pool defects, thrombocytopenia with absent radius (TAR) syndrome and thrombotic thrombocytopenic purpura (TTP).

12. A method for treating a disease in which mediation, such as activation or inhibition, regulation and modulation, of ACKR3 is beneficial in a human or a warm-blooded or mammal animal in need of such treatment, which comprises administering to said human or warm-blooded or mammal animal a therapeutically effective amount of the compound of any one of claims 1 to 5, or a pharmaceutically acceptable salt, pro-drug, biologically active metabolite, solvate or stereoisomer thereof, or the composition of claim 6 or 7.

13. The method of claim 12, wherein the disease is a cardiovascular disease or a platelet disorder.

14. The method of claim 13, wherein the cardiovascular disease is selected from coronary artery diseases, such as angina pectoris and heart attack, stroke, heart failure, hypertensive heart disease, rheumatic heart disease, cardiomyopathy, abnormal heart rhythms, congenital heart disease, valvular heart disease, carditis, aortic aneurysms, peripheral artery disease, thromboembolic disease, venous thrombosis, and atherosclerosis and wherein the platelet disorder is selected from thrombocytosis, thrombocythemia, and thrombocytopenia, such as immune thrombocytopenia and thrombotic thrombocytopenic purpura, Bernard Soulier disease, Glanzmann's thrombasthenia, Hermansky Pudlak syndrome, Jacobsen syndrome, Lowe syndrome, platelet release and storage pool defects, thrombocytopenia with absent radius (TAR) syndrome and thrombotic thrombocytopenic purpura (TTP).

15. The method of claim 12, wherein the disease is selected from the group consisting of: distress dysfunction diseases or conditions, chronic or acute pain, cancers (incl. breast, brain, lymphoma), fibrosis (incl. cardiac fibrosis), inflammatory or autoimmune diseases and conditions, conditions of excessive or abnormal vascularization (incl. wound healing), stem cell differentiation and mobilization disorders, brain and neuronal dysfunctions (incl. Alzheimer's disease, multiple sclerosis and demyelinating diseases), kidney dysfunction, renal dysfunction, preeclampsia and obesity.
